# EUROPEAN PATENT SPECIFICATION

(11) **EP 2 241 640 B1**
(45) Date of publication and mention of the grant of the patent: **22.06.2016**
(21) Application number: 09704882.1
(22) Date of filing: 22.01.2009
(51) Int. Cl.: C12N 15/10, C12Q 1/68

(54) **NUCLEIC ACID-BINDING PROTEIN ASSAY METHOD AND KIT**
NUKLEINSÄUREBINDUNGSPROTEIN-TESTVERFAHREN UND -KIT
PROCÉDÉ ET KIT DE DOSAGE DE PROTÉINE DE LIAISON À L'ACIDE NUCLÉIQUE

(30) Priority: 22.01.2008 JP 2008011750
(43) Date of publication of application: 20.10.2010
(73) Proprietor: Hiroshima University, Hiroshima 739-8528 (JP)
(72) Inventor: TAHARA, Hidetoshi, Hiroshima-shi Hiroshima 734-8553 (JP); MIYAGI, Toru, Akitakata-shi Hiroshima 739-1195 (JP)
(74) Representative: ABG Patentes, S.L.
(86) International application number: PCT/JP2009/051006
(87) International publication number: WO 2009/093667

(56) References cited:
- WO-A1-85/00813
- WO-A1-97/23646
- WO-A1-2006/048025
- WO-A1-2007/124758
- WO-A2-01/69200
- JP-T- 2003 527 119
- JP-T- 2005 519 618
- KIM W.J.: 'Polycation graft copolymers accelerating DNA strand exchange: involvement of ionic interaction.' NUCLEIC ACIDS RES. SUPPL. no. 1, 2001, pages 151 - 152, XP008139422

## Description

### Technical Field

The present invention relates to a method and a kit of detecting a nucleic acid binding protein. The present invention also relates to a method of screening for a binding inhibitor or promoter for a nucleic acid binding protein.

### Background Art

Nucleic acid binding proteins are molecules responsible for very important intracellular functions, such as transcription factors, chromosome structural proteins, DNA repair enzymes, enzymes involved in RNA processing, and polymerases. Behavior analysis on the nucleic acid binding proteins is thought to have profound implications for understanding life activities. Moreover, the nucleic acid binding proteins have received increasing attention as targets of development of pharmaceutical agents, functional foods, or the like, because of their important functions.

With technical progress in recent years, various methods of detecting nucleic acid binding proteins have been developed so far.

In general, gel shift or footprinting assay is used in the detection of nucleic acid binding proteins. However, these methods involve electrophoresis and therefore have the problems of time-consuming and complicated procedures.

A homogeneous method of detecting a nucleic acid binding protein using two types of DNA probes that bind only to nucleic acids bound to the proteins (U.S. Patent No. 6,544,746; and Heyduk, T., et al. (2002) Nat. Biotechnol. 20 (2) 171-176) has the problems of complicated design of DNA probes and its limited applicability to a DNA binding protein capable of binding to sequences at two distant locations (Wang, J., et al. (2005) Nucleic Acids Res. 33 (2) e23; and Jantz, D., et al. (2002) Nat. Biotechnol. 20 (2) 126-127).

A method of detecting a nucleic acid binding protein using exonuclease III (Non-Patent Document 2) has the difficulty in detecting a weakly bound nucleic acid binding protein and requires taking into consideration the influence of a drug on exonuclease III activity when used in the screening of drugs such as pharmaceutical agents. This method further has the problems of inability to screen for drugs susceptible to the activity of proteins related to exonuclease III.

A method of detecting a nucleic acid binding protein using fluorescence correlation spectroscopy (Kobayashi, T., et al. (2004) Anal. Biocheln. 332 (1) 58-66) detects a difference in size between a nucleic acid alone and a complex of the nucleic acid bound to the nucleic acid binding protein. This method has the problem that a sufficient signal/noise ratio is not obtained from small nucleic acid binding proteins. Furthermore, this method requires specialized equipment and therefore has the problems associated with general versatility.

Thus, a method of rapidly and conveniently handling a plurality of samples has not yet been reported, though various detection methods have been developed. There is a strong demand for the development of a method applicable to the screening of pharmaceutical agents, functional foods, or the like targeting a nucleic acid binding protein.

### Summary of the Invention

In general, a nucleic acid duplex consisting of two fully complementary nucleic acid strands is very stable. Such nucleic acid duplexes neither interact with each other nor are structurally changed. However, nucleic acid duplexes having a single-stranded moiety at their termini are known to bind to each other via these single-stranded moieties to cause structural change. The present inventors have found that such structural change between nucleic acid duplexes is inhibited by the binding of a nucleic acid binding protein. The present invention is based on this finding.

An object of the present invention is to provide a method of detecting binding between a nucleic acid and a nucleic acid binding protein and a method of screening for a binding inhibitor or promoter for a nucleic acid binding protein.

According to the present invention, there is provided a method of detecting binding between a nucleic acid and a nucleic acid binding protein, comprising determining the degree of structural change in a nucleic acid complex having at least two nucleic acid duplex moieties (hereinafter, referred to as a "detection method according to the present invention").

According to the present invention, there is also provided a method of screening for a binding inhibitor or promoter for a nucleic acid binding protein, comprising performing the detection method according to the present invention in the presence of and in the absence of a test substance (hereinafter, referred to as a "screening method according to the present invention").

According to the present invention, there is further provided a kit comprising a nucleic acid duplex A and a nucleic acid duplex B capable of binding to each other at their terminal sequences or a kit comprising an integrated nucleic acid complex E or an integrated nucleic acid complex F (hereinafter, referred to as a "kit according to the present invention").

The present invention is advantageous in that it eliminates the need for electrophoresis, achieves homogeneous assay using general-purpose equipment, eliminates the need for enzymes such as exonuclease III, and eliminates the need for fluorescence introduction or unnatural structures (e.g., nick) in protein binding sites. The present invention is also advantageous in that it is widely applicable to general DNA binding proteins including DNA binding proteins having a DNA binding motif, for example, loop, leucine zipper, or Zn finger. The present invention is further advantageous in that it can screen for an inhibitor or promoter targeting nucleic acid binding proteins that are difficult to evaluate by reporter assay (e.g., structural proteins such as telomere binding proteins). Thus, the present invention is useful because of its applicability to search for or identification of novel pharmaceutical agents (e.g., inhibitors), health foods, or the like.

### Brief Description of the Drawings

Figure 1A is a diagram specifically showing one aspect of the structures of a nucleic acid A1, a nucleic acid A2, a nucleic acid B1, and a nucleic acid B2.
Figure 1B is a diagram specifically showing one aspect (integrated nucleic acid complex E) of the structures of a nucleic acid G1, a nucleic acid G2, a nucleic acid H1, and a nucleic acid H2.
Figure 1C is a diagram specifically showing one aspect (integrated nucleic acid complex F) of the structures of the nucleic acid G1, the nucleic acid G2, the nucleic acid H1, and the nucleic acid H2.
Figure 2 is a diagram showing the relationship between the concentration of recombinant human NF_{κ}B p50 and a fluorescence value.
Figure 3 is a diagram showing a fluorescence value derived from the inhibition of the binding of recombinant human NF_{κ}B p50 using decoy oligonucleotides.
Figure 4 is a diagram showing a fluorescence value derived from the binding of recombinant human AP1 (c-jun) proteins.
Figure 5 is a diagram showing a fluorescence value derived from the binding of recombinant human SP1 proteins.
Figure 6 is a diagram showing results of detecting the binding of recombinant human TRF2 proteins (N-terminal deletion variants) to nucleic acids.
Figure 7 is a diagram showing the relationship between mismatch introduction and detection sensitivity. The numeric values above the bars represent a relative fluorescence value with the fluorescence value of each protein-unsupplemented nucleic acid set defined as 100.
Figure 8 is a diagram showing the relationship between mismatch introduction and detection sensitivity. The numeric values above the bars represent a relative fluorescence value with the fluorescence value of each protein-unsupplemented nucleic acid set defined as 100.
Figure 9 is a diagram showing the fluorescence intensities of NF-_{κ}B/DNA and SP1/DNA in terms of a relative fluorescence value to that in the absence of aurothioglucose.
Figure 10 is a diagram showing the detection of NF-_{κ}B (p50) using an integrated complex. This diagram shows the fluorescence intensities of NF-_{κ}B/DNA and SP1/DNA in terms of a relative fluorescence value to that in the absence of the proteins.
Figure 11 is a diagram showing results of detecting the bindings of NF-_{κ}B and SP1 in a multiplexed system. This diagram shows the fluorescence intensities of NF-_{κ}B/DNA and SP1/DNA in terms of a relative fluorescence value to that in the absence of the proteins.
Figure 12 is a diagram specifically showing one aspect of detection using an integrated nucleic acid complex. The asterisks represent a fluorescent label. The filled circles represent a quenching label. The open circles and the open squares represent a variation. The gray solid lines represent a sequence to which a nucleic acid binding protein binds.
Figure 13 is a diagram specifically showing one aspect of the introduction of a variation for preventing proteins from binding to the protein binding sequence.
Figure 14 is a diagram specifically showing one aspect of the introduction of a variation for preventing proteins from binding to the protein binding sequence.

### Detailed Description of the Invention

### Definition

In the present specification, examples of a "nucleic acid" include not only DNA and RNA but modified nucleic acids and nucleic acid analogs (e.g., peptide nucleic acid (PNA) and locked nucleic acid (LNA)). The nucleic acid is preferably DNA.

In the present specification, a "nucleic acid duplex" means two nucleic acid molecules bound to each other as complementary strands.

In the present specification, a "single-stranded nucleic acid" means a nucleic acid molecule that is not bound as a complementary stand to another nucleic acid molecule. For example, a nucleic acid molecule having a double-stranded moiety as a result of hybridization of a portion of the nucleic acid molecule to within the nucleic acid molecule is also included in the "single-stranded nucleic acid". Moreover, the single-stranded nucleic acid may be bound in series with another single-stranded nucleic acid either directly or via an additional sequence (e.g., a linker sequence) or the like.

In the present specification, the term "complementary" means that a hydrogen bond is formed between bases constituting sequences and specifically means that adenine is paired with thymine; thymine is paired with adenine; guanine is paired with cytosine; and cytosine is paired with guanine. In the present specification, the term "complementarity" means the ratio of complementary base pairs between sequences to be compared. All the numeric values of "complementarity" shown in the present specification need only to be numeric values calculated as homology between the complementary strands of one nucleotide sequence and another nucleotide sequence using a homology search program generally known by those skilled in the art. These numeric values of complementarity can be calculated easily using default (initial setting) parameters in, for example, FASTA or BLAST.

In the present specification, the term "hybridizing" means hybridizing to a target nucleic acid under stringent conditions and not hybridizing to nucleic acids other than the target. Examples of a nucleic acid capable of hybridizing to the nucleotide sequence of the target nucleic acid under stringent conditions include nucleic acids consisting of a nucleotide sequence having 70% or higher, preferably 80% or higher, more preferably 90% or higher, most preferably 95% or higher complementarity to the nucleotide sequence of the target nucleic acid. The stringent conditions can be determined appropriately by those skilled in the art (Molecular Cloning 2nd edition, Cold Spring Harbor Laboratory (1989)) and refer to, for example, conditions involving a salt concentration of 0.1 mM to 3 M and a temperature of 10 to 80°C, preferably conditions involving a salt concentration of 1 mM to 1 M and a temperature of 20 to 70°C.

In the present specification, the number of "contiguous" nucleotides is not particularly limited as long as each nucleotide sequence can bind (preferably, hybridize) to a nucleotide sequence corresponding thereto. The ratio of a nucleotide sequence consisting of the contiguous nucleotides to each nucleotide sequence is preferably 15% or higher, more preferably 20% or higher, further preferably 25% or higher, particularly preferably 30% or higher.

In the present specification, a "Tm value" means a temperature at which 50% of two single-stranded nucleic acids to be compared form a double strand under reaction conditions where the method of the present invention is performed (these conditions are specified based on e.g., nucleic acid concentration, salt concentration, and pH). The Tm value can be calculated using, for example, change in ultraviolet absorption caused by temperature change, double-stranded DNA detecting reagents such as SYBR Green I, or change in fluorescence value in fluorescence energy transfer. More conveniently, it can be calculated using algorithm known in the art (e.g., Markham NR and Zuker M (2005) Nucleic Acids Research 33, W577-W581).

In the present specification, a "mismatch" means a base pair other than adenine/thymine and guanine/cytosine base pairs or a state free from corresponding bases (bulge).

### Nucleic acid complex

According to the present invention, there is provided a method of detecting binding between a nucleic acid and a nucleic acid binding protein, comprising determining the degree of structural change in a nucleic acid complex having at least two nucleic acid duplex moieties.

In this context, the "structural change" is preferably nucleotide strand exchange between the nucleic acid duplexes in the nucleic acid complex. The nucleotide strand exchange between the nucleic acid duplexes means replacement of a strand complementary to each nucleotide strand forming one nucleic acid duplex with a nucleotide strand forming the other nucleic acid duplex. The nucleotide strand exchange between the nucleic acid duplexes may be irreversible or reversible and is based on the structure of the nucleic acid complex used.

Examples of the nucleic acid complex having at least two nucleic acid duplex moieties include a complex comprising the two nucleic acid duplexes bound to each other at their terminal sequences (nucleic acid duplex complex; first aspect) and a complex comprising the two nucleic acid duplex moieties to cause strand exchange reversibly (integrated nucleic acid complex; second aspect). The nucleic acid complex of the first aspect and the nucleic acid complex of the second aspect will be described below.

### Nucleic acid complex of first aspect

According to the first aspect of the present invention, there is provided the method comprising determining the degree of structural change in the complex comprising the two nucleic acid duplexes (nucleic acid duplex A and nucleic acid duplex B) bound to each other at their terminal sequences (nucleic acid duplex complex).

The nucleic acid duplex A is a nucleic acid duplex that consists of two single-stranded nucleic acids, i.e., a nucleic acid A1 and a nucleic acid A2 and is capable of binding at its terminal sequence to the nucleic acid duplex B.

The nucleic acid duplex B is a nucleic acid duplex that consists of two single-stranded nucleic acids, i.e., a nucleic acid B1 and a nucleic acid B2 and is capable of binding at its terminal sequence to the nucleic acid duplex A.

The nucleic acid A1, the nucleic acid A2, the nucleic acid B1, and the nucleic acid B2 can respectively be designed as follows:
- nucleic acid A1: a single-stranded nucleic acid having a first nucleotide sequence and a second nucleotide sequence (terminal sequence);
- nucleic acid A2: a single-stranded nucleic acid having a sequence corresponding to the first nucleotide sequence and a third nucleotide sequence (terminal sequence);
- nucleic acid B1: a single-stranded nucleic acid having a sequence corresponding to the second nucleotide sequence (terminal sequence) and a fourth nucleotide sequence; and
- nucleic acid B2: a single-stranded nucleic acid having a sequence corresponding to the third nucleotide sequence (terminal sequence) and a sequence corresponding to the fourth nucleotide sequence.

For example, the nucleic acid A1 is first designed based on the nucleic acid binding protein of interest. Then, the nucleic acid A2, the nucleic acid B1, and the nucleic acid B2 can be designed in order based on the nucleic acid A1.

The nucleic acid A1, the nucleic acid A2, the nucleic acid B1, and the nucleic acid B2 can be designed in such a way that nucleotide strand exchange between the nucleic acid duplexes in the nucleic acid duplex complex occurs irreversibly.

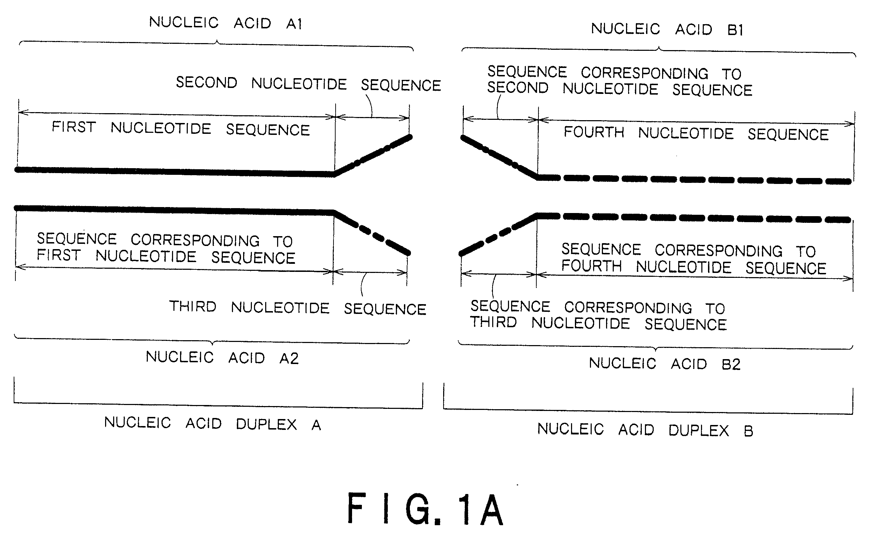

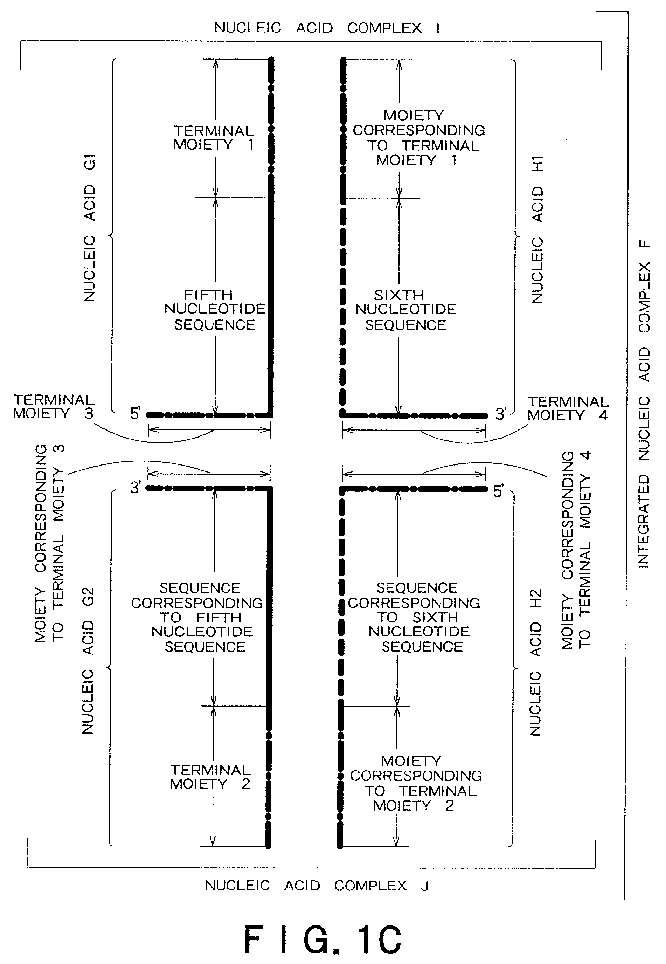

One aspect of the specific structures of the nucleic acid A1, the nucleic acid A2, the nucleic acid B1, and the nucleic acid B2 is shown in Figure 1A.

The first to fourth nucleotide sequences (polynucleotides) and the sequences corresponding thereto (polynucleotides) are not particularly limited as long as the nucleic acid duplex A and the nucleic acid duplex B can respectively be formed. These sequences can be selected from optional sequences. For example, the first nucleotide sequence in the nucleic acid A1 can be selected as a sequence that binds (preferably, hybridizes) to the sequence corresponding to the first nucleotide sequence in the nucleic acid A2. Moreover, the fourth nucleotide sequence in the nucleic acid B1 can be selected as a sequence that binds (preferably, hybridizes) to the sequence corresponding to the fourth nucleotide sequence in the nucleic acid B2.

The first nucleotide sequence can be selected in such a way that it has 70% or higher, preferably 80% or higher, more preferably 90% or higher, particularly preferably 95% or higher complementarity to the sequence corresponding to the first nucleotide sequence.

The fourth nucleotide sequence can be selected in such a way that it has 70% or higher, more preferably 80% or higher, particularly preferably 90% or higher, most preferably 95% or higher complementarity to the sequence corresponding to the fourth nucleotide sequence.

The sequence having 70% or higher complementarity to each nucleotide sequence is preferably a sequence complementary to the nucleotide sequence. As described later, a mismatch may be introduced therein for the purpose of improving detection sensitivity.

The first nucleotide sequence can be selected in such a way that it comprises at least 5, preferably at least 10, more preferably at least 15, further preferably at least 20, particularly preferably at least 25 contiguous nucleotides of a sequence complementary to the sequence corresponding to the first nucleotide sequence. Moreover, the sequence corresponding to the first nucleotide sequence can be selected in such a way that it comprises at least 5, preferably at least 10, more preferably at least 15, further preferably at least 20, particularly preferably at least 25 contiguous nucleotides of a sequence complementary to the first nucleotide sequence.

The fourth nucleotide sequence can be selected in such a way that it comprises at least 5, preferably at least 10, more preferably at least 15, further preferably at least 20, particularly preferably at least 25 contiguous nucleotides of a sequence complementary to the sequence corresponding to the fourth nucleotide sequence. Moreover, the sequence corresponding to the fourth nucleotide sequence can be selected in such a way that it comprises at least 5, preferably at least 10, more preferably at least 15, further preferably at least 20, particularly preferably at least 25 contiguous nucleotides of a sequence complementary to the fourth nucleotide sequence.

The first nucleotide sequence can be selected in such a way that the Tm value between the first nucleotide sequence and the sequence corresponding to the first nucleotide sequence is 5°C or more, preferably 10°C or more, more preferably 15°C or more, further preferably 20°C or more, higher than the reaction temperature.

The fourth nucleotide sequence can be selected in such a way that the Tm value between the fourth nucleotide sequence and the sequence corresponding to the fourth nucleotide sequence is 5°C or more, preferably 10°C or more, more preferably 15°C or more, further preferably 20°C or more, higher than the reaction temperature.

The first to fourth nucleotide sequences and the sequences corresponding thereto are not particularly limited as long as the nucleic acid duplex A and the nucleic acid duplex B can form a nucleic acid duplex complex. These sequences can be selected from optional sequences. For example, the second nucleotide sequence in the nucleic acid duplex A can be selected as a sequence that binds (preferably, hybridizes) to the sequence corresponding to the second nucleotide sequence in the nucleic acid duplex B but does not bind (preferably, hybridize) to the third nucleotide sequence in the nucleic acid duplex A, the sequence corresponding to the third nucleotide sequence in the nucleic acid duplex B, or second nucleotide sequences in other nucleic acid duplexes A. Moreover, the third nucleotide sequence in the nucleic acid duplex A can be selected as a sequence that binds (preferably, hybridizes) to the sequence corresponding to the third nucleotide sequence in the nucleic acid duplex B but does not bind (preferably, hybridize) to the second nucleotide sequence in the nucleic acid duplex A, the sequence corresponding to the second nucleotide sequence in the nucleic acid duplex B, or third nucleotide sequences in other nucleic acid duplexes A. Furthermore, the second nucleotide sequence and the third nucleotide sequence in the nucleic acid duplex A as well as the sequence corresponding to the second nucleotide sequence and the sequence corresponding to the third nucleotide sequence in the nucleic acid duplex B can respectively be selected as a sequence that does not bind (preferably, hybridize) to within the nucleic acid molecule.

The second nucleotide sequence can be selected in such a way that it has 70% or higher, preferably 80% or higher, more preferably 90% or higher, particularly preferably 95% or higher complementarity to the sequence corresponding to the second nucleotide sequence. On the other hand, the second nucleotide sequence can be selected in such a way that it has complementarity lower than 50%, more preferably lower than 30%, to each of the third nucleotide sequence and the sequence corresponding to the third nucleotide sequence. Moreover, the sequence corresponding to the second nucleotide sequence can be selected in such a way that it has complementarity lower than 50%, more preferably lower than 30%, to each of the third nucleotide sequence and the sequence corresponding to the third nucleotide sequence.

The third nucleotide sequence can be selected in such a way that it has 70% or higher, more preferably 80% or higher, particularly preferably 90% or higher, most preferably 95% or higher complementarity to the sequence corresponding to the third nucleotide sequence. On the other hand, the third nucleotide sequence can be selected in such a way that it has complementarity lower than 50%, more preferably lower than 30%, to each of the second nucleotide sequence and the sequence corresponding to the second nucleotide sequence. Moreover, the sequence corresponding to the third nucleotide sequence can be selected in such a way that it has complementarity lower than 50%, more preferably lower than 30%, to each of the second nucleotide sequence and the sequence corresponding to the second nucleotide sequence.

The sequence having 70% or higher complementarity to each nucleotide sequence is preferably a sequence complementary to the nucleotide sequence. As described later, a mismatch may be introduced therein for the purpose of improving detection sensitivity.

The second nucleotide sequence can be selected in such a way that it comprises at least 5, preferably at least 10, more preferably at least 15, further preferably at least 20 contiguous nucleotides of a sequence complementary to the sequence corresponding to the second nucleotide sequence. Moreover, the sequence corresponding to the second nucleotide sequence can be selected in such a way that it comprises at least 5, preferably at least 10, more preferably at least 15, further preferably at least 20 contiguous nucleotides of a sequence complementary to the second nucleotide sequence. On the other hand, the second nucleotide sequence may contain contiguous nucleotides of a sequence complementary to the third nucleotide sequence and/or the sequence corresponding to the third nucleotide sequence. Moreover, the sequence corresponding to the second nucleotide sequence may contain contiguous nucleotides of a sequence complementary to the third nucleotide sequence and/or the sequence corresponding to the third nucleotide sequence. However, the number of the nucleotides is 4 at the maximum, preferably 3 or less. Moreover, the third nucleotide sequence may contain contiguous nucleotides of a sequence complementary to the second nucleotide sequence and/or the sequence corresponding to the second nucleotide sequence. Moreover, the sequence corresponding to the third nucleotide sequence may contain contiguous nucleotides of a sequence complementary to the second nucleotide sequence and/or the sequence corresponding to the second nucleotide sequence. However, the number of the nucleotides is 4 at the maximum, preferably 3 or less.

The third nucleotide sequence can be selected in such a way that it comprises at least 5, preferably at least 10, more preferably at least 15, further preferably at least 20 contiguous nucleotides of a sequence complementary to the sequence corresponding to the third nucleotide sequence. Moreover, the sequence corresponding to the third nucleotide sequence can be selected in such a way that it comprises at least 5, preferably at least 10, more preferably at least 15, further preferably at least 20 contiguous nucleotides of a sequence complementary to the third nucleotide sequence. On the other hand, the third nucleotide sequence may contain contiguous nucleotides of a sequence complementary to the second nucleotide sequence and/or the sequence corresponding to the second nucleotide sequence. Moreover, the sequence corresponding to the third nucleotide sequence may contain contiguous nucleotides of a sequence complementary to the second nucleotide sequence and/or the sequence corresponding to the second nucleotide sequence. However, the number of the nucleotides is 4 at the maximum, preferably 3 or less. Moreover, the second nucleotide sequence may contain contiguous nucleotides of a sequence complementary to the third nucleotide sequence and/or the sequence corresponding to the third nucleotide sequence. Moreover, the sequence corresponding to the second nucleotide sequence may contain contiguous nucleotides of a sequence complementary to the third nucleotide sequence and/or the sequence corresponding to the third nucleotide sequence. However, the number of the nucleotides is 4 at the maximum, preferably 3 or less.

The second nucleotide sequence can be selected in such a way that the Tm value between the second nucleotide sequence and the sequence corresponding to the second nucleotide sequence is 5°C or more, preferably 10°C or more, more preferably 15°C or more, further preferably 20°C or more, higher than the reaction temperature. On the other hand, the second nucleotide sequence can be selected in such a way that the Tm value between the second nucleotide sequence and each of the third nucleotide sequence and the sequence corresponding to the third nucleotide sequence is 5°C or more, preferably 10°C or more, more preferably 15°C or more, further preferably 20°C or more, lower than the reaction temperature. Moreover, the sequence corresponding to the second nucleotide sequence can be selected in such a way that the Tm value between the sequence corresponding to the second nucleotide sequence and each of the third nucleotide sequence and the sequence corresponding to the third nucleotide sequence is 5°C or more, preferably 10°C or more, more preferably 15°C or more, further preferably 20°C or more, lower than the reaction temperature.

The third nucleotide sequence can be selected in such a way that the Tm value between the third nucleotide sequence and the sequence corresponding to the third nucleotide sequence is 5°C or more, preferably 10°C or more, more preferably 15°C or more, further preferably 20°C or more, higher than the reaction temperature. On the other hand, the third nucleotide sequence can be selected in such a way that the Tm value between the third nucleotide sequence and each of the second nucleotide sequence and the sequence corresponding to the second nucleotide sequence is 5°C or more, preferably 10°C or more, more preferably 15°C or more, further preferably 20°C or more, lower than the reaction temperature. Moreover, the sequence corresponding to the third nucleotide sequence can be selected in such a way that the Tm value between the sequence corresponding to the third nucleotide sequence and each of the second nucleotide sequence and the sequence corresponding to the second nucleotide sequence is 5°C or more, preferably 10°C or more, more preferably 15°C or more, further preferably 20°C or more, lower than the reaction temperature.

The first to fourth nucleotide sequences and the sequences corresponding thereto are not particularly limited as long as structural change occurs to form a nucleic acid duplex C and a nucleic acid duplex D from the nucleic acid duplex A and the nucleic acid duplex B. These sequences can be selected from optional sequences. For example, the first nucleotide sequence in the nucleic acid A1 can be selected as a sequence that binds (preferably, hybridizes) to the fourth nucleotide sequence in the nucleic acid B1. Moreover, the sequence corresponding to the first nucleotide sequence in the nucleic acid A2 can be selected as a sequence that binds (preferably, hybridizes) to the sequence corresponding to the fourth nucleotide sequence in the nucleic acid B2.

The first nucleotide sequence can be selected in such a way that it has 70% or higher, preferably 80% or higher, more preferably 90% or higher, particularly preferably 95% or higher complementarity to the fourth nucleotide sequence.

The sequence corresponding to the first nucleotide sequence can be selected in such a way that it has 70% or higher, more preferably 80% or higher, particularly preferably 90% or higher, most preferably 95% or higher complementarity to the sequence corresponding to the fourth nucleotide sequence.

The sequence having 70% or higher complementarity to each nucleotide sequence is preferably a sequence complementary to the nucleotide sequence. As described later, a mismatch may be introduced therein for the purpose of improving detection sensitivity.

The first nucleotide sequence can be selected in such a way that it comprises at least 10, preferably at least 15, more preferably at least 20, further preferably at least 25 contiguous nucleotides of a sequence complementary to the fourth nucleotide sequence. Moreover, the fourth nucleotide sequence can be selected in such a way that it comprises at least 10, preferably at least 15, more preferably at least 20, further preferably at least 25 contiguous nucleotides of a sequence complementary to the first nucleotide sequence.

The sequence corresponding to the first nucleotide sequence can be selected in such a way that it comprises at least 10, preferably at least 15, more preferably at least 20, further preferably at least 25 contiguous nucleotides of a sequence complementary to the sequence corresponding to the fourth nucleotide sequence. Moreover, the sequence corresponding to the fourth nucleotide sequence can be selected in such a way that it comprises at least 10, preferably at least 15, more preferably at least 20, further preferably at least 25 contiguous nucleotides of a sequence complementary to the sequence corresponding to the first nucleotide sequence.

The first nucleotide sequence can be selected in such a way that the Tm value between the first nucleotide sequence and the fourth nucleotide sequence is 5°C or more, preferably 10°C or more, more preferably 15°C or more, further preferably 20°C or more, higher than the reaction temperature.

The sequence corresponding to the first nucleotide sequence can be selected in such a way that the Tm value between the sequence corresponding to the first nucleotide sequence and the sequence corresponding to the fourth nucleotide sequence is 5°C or more, preferably 10°C or more, more preferably 15°C or more, further preferably 20°C or more, higher than the reaction temperature.

The second nucleotide sequence and the third nucleotide sequence as the terminal sequences of the nucleic acid duplex A may both be positioned at one end of the nucleic acid duplex A or may respectively be positioned at both ends thereof. The sequence corresponding to the second nucleotide sequence and the sequence corresponding to the third nucleotide sequence as the terminal sequences of the nucleic acid duplex B may both be positioned at one end of the nucleic acid duplex B or may respectively be positioned at both ends thereof. Preferably, the second nucleotide sequence and the third nucleotide sequence as the terminal sequences of the nucleic acid duplex A may both be positioned at one end of the nucleic acid duplex A; and the sequence corresponding to the second nucleotide sequence and the sequence corresponding to the third nucleotide sequence as the terminal sequences of the nucleic acid duplex B may both be positioned at one end of the nucleic acid duplex B.

The base pair length of the duplex moiety (moiety in which the first nucleotide sequence is paired with the sequence corresponding to the first nucleotide sequence) in the nucleic acid duplex A is not particularly limited unless structural change to form a nucleic acid duplex C and a nucleic acid duplex D is inhibited. The base pair length can be set to, for example, 5 base pairs to 1000 base pairs and is preferably 10 base pairs to 100 base pairs, more preferably 15 base pairs to 50 base pairs.

The base pair length of the duplex moiety (moiety in which the fourth nucleotide sequence is paired with the sequence corresponding to the fourth nucleotide sequence) in the nucleic acid duplex B is not particularly limited unless structural change to form a nucleic acid duplex C and a nucleic acid duplex D is inhibited. The base pair length can be set to, for example, 5 base pairs to 1000 base pairs and is preferably 10 base pairs to 100 base pairs, more preferably 15 base pairs to 50 base pairs.

The base length of each single-stranded moiety (the second nucleotide sequence and the third nucleotide sequence) in the nucleic acid duplex A is not particularly limited as long as the nucleic acid duplex complex of the present invention can be formed. The base length can be set to, for example, 3 bases to 1000 bases and is preferably 5 bases to 100 bases, more preferably 10 bases to 30 bases.

The base length of each single-stranded moiety (the sequence corresponding to the second nucleotide sequence and the sequence corresponding to the third nucleotide sequence) in the nucleic acid duplex B is not particularly limited as long as the nucleic acid duplex complex of the present invention can be formed. The base length can be set to, for example, 3 bases to 1000 bases and is preferably 5 bases to 100 bases, more preferably 10 bases to 30 bases.

According to the present invention, at least one of the nucleic acid duplex A and the nucleic acid duplex B has a site to which the nucleic acid binding protein binds.

The site to which the nucleic acid binding protein binds is at least one type of protein binding site existing at at least one location selected from the duplex moieties and the single-stranded moieties of the nucleic acid duplex A and/or the nucleic acid duplex B and the branch moieties of the nucleic acid duplex complex and is preferably located in the duplex moiety. When proteins that recognize and bind a particular nucleotide sequence, such as transcription factors, are used, the protein binding site comprises the sequence recognized thereby and its surrounding sequence.

The binding pattern of the nucleic acid duplex complex is not particularly limited as long as the nucleic acid duplex A and the nucleic acid duplex B are bound to each other at their terminal sequences and structural change such as nucleotide strand exchange occurs. Preferably, the binding can be performed by hybridization.

For causing nucleotide strand exchange between the nucleic acid duplex A and the nucleic acid duplex B in the nucleic acid duplex complex, the nucleic acid duplex A and the nucleic acid duplex B are preferably prepared in such a way that the 3'-terminal sequence (second nucleotide sequence) of the nucleic acid duplex A binds to the 5'-terminal sequence (sequence corresponding to the second nucleotide sequence) of the nucleic acid duplex B and/or the 5'-terminal sequence (third nucleotide sequence) of the nucleic acid duplex A binds to the 3'-terminal sequence (sequence corresponding to the third nucleotide sequence) of the nucleic acid duplex B.

In a preferable aspect of the present invention, the nucleic acid duplex A and the nucleic acid duplex B are designed in such a way that the 3'-terminal sequence of the nucleic acid duplex A hybridizes to the 5'-terminal sequence of the nucleic acid duplex B and/or the 5'-terminal sequence of the nucleic acid duplex A hybridizes to the 3'-terminal sequence of the nucleic acid duplex B.

For allowing the formed nucleic acid duplex C and nucleic acid duplex D to exist stably, the nucleic acid duplex A and the nucleic acid duplex B are preferably prepared in such a way that the nucleic acid duplex C and the nucleic acid duplex D do not bind to each other.

In a preferable aspect of the present invention, the nucleic acid duplex A and the nucleic acid duplex B are designed in such a way that a single-stranded sequence derived from the nucleic acid A1 and the nucleic acid B1 bound with each other does not hybridize to a single-stranded sequence derived from the nucleic acid A2 and the nucleic acid B2 bound with each other.

According to a preferable aspect of the present invention, there is provided the detection method according to the present invention, wherein the complementarity between the first nucleotide sequence and the fourth nucleotide sequence is 70% or higher and/or the complementarity between the sequence corresponding to the first nucleotide sequence and the sequence corresponding to the fourth nucleotide sequence is 70% or higher; and/or the complementarity between the second nucleotide sequence and the third nucleotide sequence, the complementarity between the second nucleotide sequence and the sequence corresponding to the third nucleotide sequence, the complementarity between the sequence corresponding to the second nucleotide sequence and the third nucleotide sequence, and the complementarity between the sequence corresponding to the second nucleotide sequence and the sequence corresponding to the third nucleotide sequence are respectively lower than 50%.

According to a preferable aspect of the present invention, there is also provided the detection method according to the present invention, wherein the first nucleotide sequence comprises at least 10 contiguous nucleotides of a sequence complementary to the fourth nucleotide sequence or the fourth nucleotide sequence comprises at least 10 contiguous nucleotides of a sequence complementary to the first nucleotide sequence, and/or the sequence corresponding to the first nucleotide sequence comprises at least 10 contiguous nucleotides of a sequence complementary to the sequence corresponding to the fourth nucleotide sequence or the sequence corresponding to the fourth nucleotide sequence comprises at least 10 contiguous nucleotides of a sequence complementary to the sequence corresponding to the first nucleotide sequence; and/or when the second nucleotide sequence contains contiguous nucleotides of a sequence complementary to the third nucleotide sequence, the number of the nucleotides is 4 at the maximum; when the second nucleotide sequence contains contiguous nucleotides of a sequence complementary to the sequence corresponding to the third nucleotide sequence, the number of the nucleotides is 4 at the maximum; when the sequence corresponding to the second nucleotide sequence contains contiguous nucleotides of a sequence complementary to the third nucleotide sequence, the number of the nucleotides is 4 at the maximum; when the sequence corresponding to the second nucleotide sequence contains contiguous nucleotides of a sequence complementary to the sequence corresponding to the third nucleotide sequence, the number of the nucleotides is 4 at the maximum; when the third nucleotide sequence contains contiguous nucleotides of a sequence complementary to the second nucleotide sequence, the number of the nucleotides is 4 at the maximum; when the third nucleotide sequence contains contiguous nucleotides of a sequence complementary to the sequence corresponding to the second nucleotide sequence, the number of the nucleotides is 4 at the maximum; when the sequence corresponding to the third nucleotide sequence contains contiguous nucleotides of a sequence complementary to the second nucleotide sequence, the number of the nucleotides is 4 at the maximum; and when the sequence corresponding to the third nucleotide sequence contains contiguous nucleotides of a sequence complementary to the sequence corresponding to the second nucleotide sequence, the number of the nucleotides is 4 at the maximum.

According to a preferable aspect of the present invention, there is further provided the detection method according to the present invention, wherein the Tm value between the first nucleotide sequence and the fourth nucleotide sequence and/or the Tm value between the sequence corresponding to the first nucleotide sequence and the sequence corresponding to the fourth nucleotide sequence are 5°C or more higher than the reaction temperature; and/or the Tm value between the second nucleotide sequence and the third nucleotide sequence, the Tm value between the second nucleotide sequence and the sequence corresponding to the third nucleotide sequence, the Tm value between the sequence corresponding to the second nucleotide sequence and the third nucleotide sequence, and the Tm value between the sequence corresponding to the second nucleotide sequence and the sequence corresponding to the third nucleotide sequence are respectively 5°C or more lower than the reaction temperature.

### Nucleic acid complex of second aspect

According to the second aspect of the present invention, there is provided the method comprising determining the degree of structural change in the complex comprising the two nucleic acid duplex moieties to cause strand exchange reversibly (integrated nucleic acid complex E or integrated nucleic acid complex F).

The integrated nucleic acid complex E comprises a nucleic acid complex G having a nucleic acid duplex moiety and a nucleic acid complex H having a nucleic acid duplex moiety.

The nucleic acid complex G is a nucleic acid complex that comprises two single-stranded nucleic acid-containing nucleic acids, i.e., a nucleic acid G1 and a nucleic acid G2 and is capable of binding at its terminal sequence to the nucleic acid complex H.

The nucleic acid complex H is a nucleic acid complex that comprises two single-stranded nucleic acid-containing nucleic acids, i.e., a nucleic acid H1 and a nucleic acid H2 and is capable of binding at its terminal sequence to the nucleic acid complex G.

The integrated nucleic acid complex F comprises a nucleic acid complex I having a nucleic acid duplex moiety and a nucleic acid complex J having a nucleic acid duplex moiety.

The nucleic acid complex I is a nucleic acid complex that comprises two single-stranded nucleic acid-containing nucleic acids, i.e., the nucleic acid G1 and the nucleic acid H1 and is capable of binding at its terminal sequence to the nucleic acid complex J.

The nucleic acid complex J is a nucleic acid complex that comprises two single-stranded nucleic acid-containing nucleic acids, i.e., the nucleic acid G2 and the nucleic acid H2 and is capable of binding at its terminal sequence to the nucleic acid complex I.

The integrated nucleic acid complex E is converted through strand exchange reaction between the nucleic acid complex G and the nucleic acid complex H to a new integrated nucleic acid complex F comprising the nucleic acid complex I and the nucleic acid complex J. Moreover, the integrated nucleic acid complex F is also converted through strand exchange reaction between the nucleic acid complex I and the nucleic acid complex J to a new integrated nucleic acid complex E comprising the nucleic acid complex G and the nucleic acid complex H. Such structural change between the integrated nucleic acid complex E and the integrated nucleic acid complex F is reversibly repeated.

The nucleic acid G1, the nucleic acid G2, the nucleic acid H1, and the nucleic acid H2 can respectively be designed as follows:
- nucleic acid G1: a single-stranded nucleic acid-containing nucleic acid consisting of a terminal moiety 1, a fifth nucleotide sequence, and a terminal moiety 3, the fifth nucleotide sequence being linked at the 3' terminus to the terminal moiety 1 and at the 5' terminus to the terminal moiety 3;
- nucleic acid G2: a single-stranded nucleic acid-containing nucleic acid consisting of a terminal moiety 2, a sequence corresponding to the fifth nucleotide sequence, and a moiety corresponding to the terminal moiety 3, the sequence corresponding to the fifth nucleotide sequence being linked at the 5' terminus to the terminal moiety 2 and at the 3' terminus to the moiety corresponding to the terminal moiety 3;
- nucleic acid H1: a single-stranded nucleic acid-containing nucleic acid consisting of a moiety corresponding to the terminal moiety 1, a sixth nucleotide sequence, and a terminal moiety 4, the sixth nucleotide sequence being linked at the 5' terminus to the moiety corresponding to the terminal moiety 1 and at the 3' terminus to the terminal moiety 4; and
- nucleic acid H2: a single-stranded nucleic acid-containing nucleic acid consisting of a moiety corresponding to the terminal moiety 4, a sequence corresponding to the sixth nucleotide sequence, and a moiety corresponding to the terminal moiety 2, the sequence corresponding to the sixth nucleotide sequence being linked at the 5' terminus to the moiety corresponding to the terminal moiety 4 and at the 3' terminus to the moiety corresponding to the terminal moiety 2.

For example, the nucleic acid G1 is first designed based on the nucleic acid binding protein of interest. Then, the nucleic acid G2, the nucleic acid H2, and the nucleic acid H1 can be designed in order based on the nucleic acid G1.

The nucleic acid G1, the nucleic acid G2, the nucleic acid H1, and the nucleic acid H2 are designed in such a way that integrated nucleic acid complexes consisting of the nucleic acid G1, the nucleic acid G2, the nucleic acid H1, and the nucleic acid H2 repeat reversible structural change through strand exchange reaction.

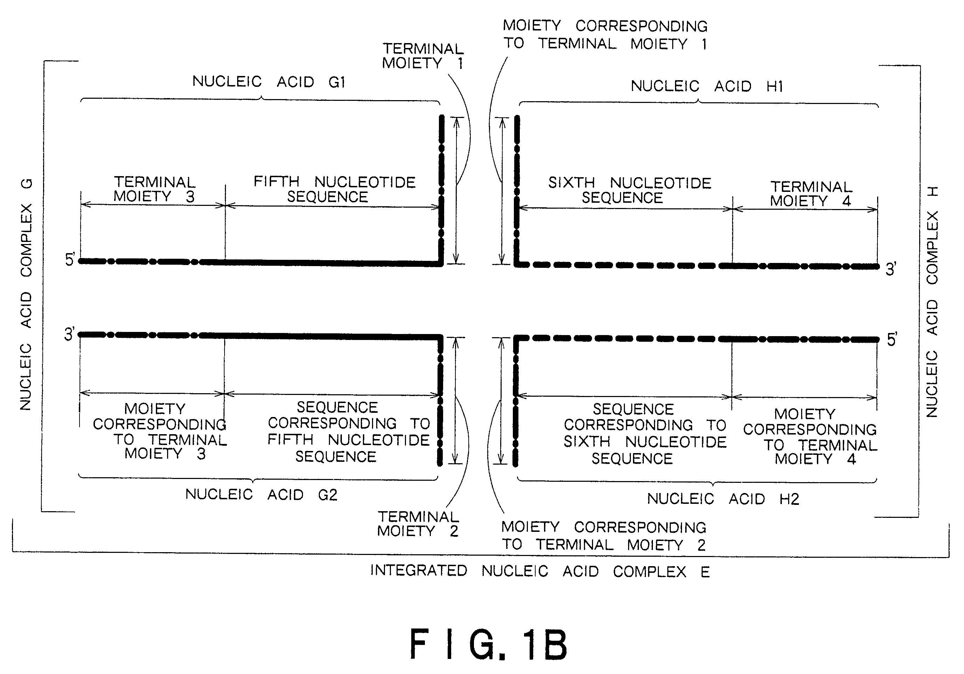

One aspect of the specific structures of the nucleic acid G1, the nucleic acid G2, the nucleic acid H1, and the nucleic acid H2 is shown in Figs. 1B (one aspect of the specific structure of the integrated nucleic acid complex E) and 1C (one aspect of the specific structure of the integrated nucleic acid complex F).

In order that reversible structural change between the nucleic acid complexes through strand exchange reaction is repeated, for example, terminal moieties for stopping the progress of strand exchange and reversing its direction can respectively be added to both ends of each sequence to undergo strand exchange (the fifth nucleotide sequence, the sequence corresponding to the fifth nucleotide sequence, the sixth nucleotide sequence, and the sequence corresponding to the sixth nucleotide sequence) in the nucleic acid G1, the nucleic acid G2, the nucleic acid H1, and the nucleic acid H2.

In this context, the "terminal moieties" are not particularly limited as long as they can stop the progress of strand exchange between two duplex moieties and reverse its direction. The terminal moieties may be polynucleotides. Alternatively, they may be linkers that link the termini of two nucleotide strands to undergo strand exchange (e.g., alkyl chains bound via phosphodiester bonds to the termini of two nucleotide strands to undergo strand exchange) or may be polynucleotides comprising such a linker introduced at its very end. The terminal moieties are preferably polynucleotides.

The case in which the terminal moieties are polynucleotides will be described below.

When the terminal moieties are polynucleotides, the nucleic acid G1, the nucleic acid G2, the nucleic acid H1, and the nucleic acid G2 can respectively be provided as a single-stranded nucleic acid.

The reversible structural change between the integrated nucleic acid complex E and the integrated nucleic acid complex F occurs as follows: first, (1) strand exchange to form a nucleic acid duplex consisting of the fifth nucleotide sequence and the sequence corresponding to the fifth nucleotide sequence is performed in the 5'→3' direction of the fifth nucleotide sequence and stopped at the 3' terminus of the fifth nucleotide sequence and/or strand exchange to form a nucleic acid duplex consisting of the sixth nucleotide sequence and the sequence corresponding to the sixth nucleotide sequence is performed in the 3'→5' direction of the sixth nucleotide sequence and stopped at the 5' terminus of the sixth nucleotide sequence (i.e., the integrated nucleic acid complex E is formed); and then, the directions of these strand exchanges are changed. Subsequently, (2) strand exchange to form a nucleic acid duplex consisting of the fifth nucleotide sequence and the sixth nucleotide sequence proceeds in the 3'→5' direction of the fifth nucleotide sequence and/or strand exchange to form a nucleic acid duplex consisting of the sequence corresponding to the fifth nucleotide sequence and the sequence corresponding to the sixth nucleotide sequence proceeds in the 5'→3' direction of the sequence corresponding to the fifth nucleotide sequence. Subsequently, (3) the strand exchange to form a nucleic acid duplex consisting of the fifth nucleotide sequence and the sixth nucleotide sequence is performed in the 3'→5' direction of the fifth nucleotide sequence and stopped at the 5' terminus of the fifth nucleotide sequence and/or the strand exchange to form a nucleic acid duplex consisting of the sequence corresponding to the fifth nucleotide sequence and the sequence corresponding to the sixth nucleotide sequence is performed in the 5'→3' direction of the sequence corresponding to the fifth nucleotide sequence and stopped at the 3' terminus of the sequence corresponding to the fifth nucleotide sequence (i.e., the integrated nucleic acid complex F is formed); and then, the directions of these strand exchanges are changed. Subsequently, (4) the strand exchange to form a nucleic acid duplex consisting of the fifth nucleotide sequence and the sequence corresponding to the fifth nucleotide sequence proceeds in the 5'→3' direction of the fifth nucleotide sequence and/or the strand exchange to form a nucleic acid duplex consisting of the sixth nucleotide sequence and the sequence corresponding to the sixth nucleotide sequence proceeds in the 3'→5' direction of the sixth nucleotide sequence. Again, the processes (1) to (4) are repeated.

For the process in which strand exchange to form a nucleic acid duplex consisting of the fifth nucleotide sequence and the sequence corresponding to the fifth nucleotide sequence is performed in the 5'→3' direction of the fifth nucleotide sequence and stopped at the 3' terminus of the fifth nucleotide sequence and/or strand exchange to form a nucleic acid duplex consisting of the sixth nucleotide sequence and the sequence corresponding to the sixth nucleotide sequence is performed in the 3'→5' direction of the sixth nucleotide sequence and stopped at the 5' terminus of the sixth nucleotide sequence; and then, the directions of these strand exchanges are changed, the terminal moieties can be designed in such a way that:
a highly stable base pair group is formed neither between the 5'-terminal nucleotide sequence of the terminal moiety 1 and the 3'-terminal nucleotide sequence of the terminal moiety 2 nor between the 3'-terminal nucleotide sequence of the moiety corresponding to the terminal moiety 1 and the 5'-terminal nucleotide sequence of the moiety corresponding to the terminal moiety 2; and/or
a highly stable base pair group is formed between the 5'-terminal nucleotide sequence of the terminal moiety 1 and the 3'-terminal nucleotide sequence of the moiety corresponding to the terminal moiety 1 and between the 3'-terminal nucleotide sequence of the terminal moiety 2 and the 5'-terminal nucleotide sequence of the moiety corresponding to the terminal moiety 2.

The terminal moieties can be designed in such a way that the highly stable base pair group formed between the 5'-terminal nucleotide sequence of the terminal moiety 1 and the 3'-terminal nucleotide sequence of the moiety corresponding to the terminal moiety 1 and between the 3'-terminal nucleotide sequence of the terminal moiety 2 and the 5'-terminal nucleotide sequence of the moiety corresponding to the terminal moiety 2 is more thermodynamically stable than a low stable base pair group between the 5'-terminal nucleotide sequence of the terminal moiety 1 and the 3'-terminal nucleotide sequence of the terminal moiety 2 and between the 3'-terminal nucleotide sequence of the moiety corresponding to the terminal moiety 1 and the 5'-terminal nucleotide sequence of the moiety corresponding to the terminal moiety 2.

Moreover, for the process in which the strand exchange to form a nucleic acid duplex consisting of the fifth nucleotide sequence and the sixth nucleotide sequence is performed in the 3'→5' direction of the fifth nucleotide sequence and stopped at the 5' terminus of the fifth nucleotide sequence and/or the strand exchange to form a nucleic acid duplex consisting of the sequence corresponding to the fifth nucleotide sequence and the sequence corresponding to the sixth nucleotide sequence is performed in the 5'→3' direction of the sequence corresponding to the fifth nucleotide sequence and stopped at the 3' terminus of the sequence corresponding to the fifth nucleotide sequence; and then, the directions of these strand exchanges are changed,
the terminal moieties can be designed in such a way that:
a highly stable base pair group is formed neither between the 3'-terminal nucleotide sequence of the terminal moiety 3 and the 5'-terminal nucleotide sequence of the terminal moiety 4 nor between the 5'-terminal nucleotide sequence of the moiety corresponding to the terminal moiety 3 and the 3'-terminal nucleotide sequence of the moiety corresponding to the terminal moiety 4; and/or
a highly stable base pair group is formed between the 3'-terminal nucleotide sequence of the terminal moiety 3 and the 5'-terminal nucleotide sequence of the moiety corresponding to the terminal moiety 3 and between the 5'-terminal nucleotide sequence of the terminal moiety 4 and the 3'-terminal nucleotide sequence of the moiety corresponding to the terminal moiety 4.

The terminal moieties can be designed in such a way that the highly stable base pair group formed between the 3'-terminal nucleotide sequence of the terminal moiety 3 and the 5'-terminal nucleotide sequence of the moiety corresponding to the terminal moiety 3 and between the 5'-terminal nucleotide sequence of the terminal moiety 4 and the 3'-terminal nucleotide sequence of the moiety corresponding to the terminal moiety 4 is more thermodynamically stable than a low stable base pair group between the 3'-terminal nucleotide sequence of the terminal moiety 3 and the 5'-terminal nucleotide sequence of the terminal moiety 4 and between the 5'-terminal nucleotide sequence of the moiety corresponding to the terminal moiety 3 and the 3'-terminal nucleotide sequence of the moiety corresponding to the terminal moiety 4.

The thermodynamic stability can be determined appropriately by those skilled in the art from the base pairs used.

The "low stable base pair group" means, in a duplex, corresponding moieties to be paired that do not form complementary strands because they contain a base pair unable to pair up due to thermodynamic instability.

The "highly stable base pair group" (base pair group H) is not particularly limited as long as it is more thermodynamically stable than the "low stable base pair group" (base pair group L). For example, the base pair group H can be set to a base pair group having a larger ratio of highly stable GC base pairs and AT base pairs (preferably, a larger ratio of GC base pairs) than that of the base pair group L. Moreover, the base pair group L is not particularly limited as long as it is more thermodynamically unstable than the base pair group H. For example, the base pair group L can be set to a base pair group having a larger ratio of low stable GG base pairs, GT base pairs, GA base pairs, TT base pairs, AA base pairs, TC base pairs, AC base pairs, CC base pairs, bulge bases (bases free from bases corresponding thereto) than that of the base pair group H.

The base pair group L is not particularly limited as long as the progress of strand exchange between two duplex moieties can be stopped. The base pair group L can be set to a base pair group consisting of at least 1, preferably 2 to 3, more preferably 4 or more (e.g., 4 to 10 or 4 to the base length of each terminal moiety) bases. More preferably, the base pair group L is a base pair group comprising at least 2 to 4 contiguous base pairs with low stability.

The base pair group H is not particularly limited as long as the progress of strand exchange between two duplex moieties can be stopped and its direction can be changed. The base pair group H can be set to a base pair group consisting of at least 2 or more, preferably 3 or more (e.g., 3 to 10 or 3 to the base length of each terminal moiety) bases. More preferably, the base pair group H is a base pair group comprising at least 2 to 4 contiguous base pairs with high stability.

The nucleotide sequences of the terminal moieties 1 to 4 and the sequences corresponding thereto are not particularly limited as long as the reversible structural change as described above is achieved between the integrated nucleic acid complex E and the integrated nucleic acid complex F. These sequences can be selected from optional sequences.

For example, the nucleotide sequence of the terminal moiety 1 can be selected as a sequence that binds (preferably, hybridizes) to the nucleotide sequence of the moiety corresponding to the terminal moiety 1. The nucleotide sequence of the terminal moiety 2 can be selected as a sequence that binds (preferably, hybridizes) to the nucleotide sequence of the moiety corresponding to the terminal moiety 2. The nucleotide sequence of the terminal moiety 3 can be selected as a sequence that binds (preferably, hybridizes) to the nucleotide sequence of the moiety corresponding to the terminal moiety 3. The nucleotide sequence of the terminal moiety 4 can be selected as a sequence that binds (preferably, hybridizes) to the nucleotide sequence of the moiety corresponding to the terminal moiety 4.

The nucleotide sequence of the terminal moiety 1 can be selected in such a way that it has 70% or higher, preferably 80% or higher, more preferably 90% or higher, particularly preferably 95% or higher complementarity to the nucleotide sequence of the moiety corresponding to the terminal moiety 1.

The nucleotide sequence of the terminal moiety 2 can be selected in such a way that it has 70% or higher, preferably 80% or higher, more preferably 90% or higher, particularly preferably 95% or higher complementarity to the nucleotide sequence of the moiety corresponding to the terminal moiety 2.

The nucleotide sequence of the terminal moiety 3 can be selected in such a way that it has 70% or higher, preferably 80% or higher, more preferably 90% or higher, particularly preferably 95% or higher complementarity to the nucleotide sequence of the moiety corresponding to the terminal moiety 3.

The nucleotide sequence of the terminal moiety 4 can be selected in such a way that it has 70% or higher, preferably 80% or higher, more preferably 90% or higher, particularly preferably 95% or higher complementarity to the nucleotide sequence of the moiety corresponding to the terminal moiety 4.

The sequence having 70% or higher complementarity to each nucleotide sequence is preferably a sequence complementary to the nucleotide sequence. As described later, a mismatch may be introduced therein for the purpose of improving detection sensitivity.

The nucleotide sequence of the terminal moiety 1 can be selected in such a way that it comprises at least 3, preferably at least 5, more preferably at least 7, further preferably at least 10 contiguous nucleotides of a sequence complementary to the nucleotide sequence of the moiety corresponding to the terminal moiety 1. Moreover, the nucleotide sequence of the moiety corresponding to the terminal moiety 1 can be selected in such a way that it comprises at least 3, preferably at least 5, more preferably at least 7, further preferably at least 10 contiguous nucleotides of a sequence complementary to the nucleotide sequence of the terminal moiety 1.

The nucleotide sequence of the terminal moiety 2 can be selected in such a way that it comprises at least 3, preferably at least 5, more preferably at least 7, further preferably at least 10 contiguous nucleotides of a sequence complementary to the nucleotide sequence of the moiety corresponding to the terminal moiety 2. Moreover, the nucleotide sequence of the moiety corresponding to the terminal moiety 2 can be selected in such a way that it comprises at least 3, preferably at least 5, more preferably at least 7, further preferably at least 10 contiguous nucleotides of a sequence complementary to the nucleotide sequence of the terminal moiety 2.

The nucleotide sequence of the terminal moiety 3 can be selected in such a way that it comprises at least 3, preferably at least 5, more preferably at least 7, further preferably at least 10 contiguous nucleotides of a sequence complementary to the nucleotide sequence of the moiety corresponding to the terminal moiety 3. Moreover, the nucleotide sequence of the moiety corresponding to the terminal moiety 3 can be selected in such a way that it comprises at least 3, preferably at least 5, more preferably at least 7, further preferably at least 10 contiguous nucleotides of a sequence complementary to the nucleotide sequence of the terminal moiety 3.

The nucleotide sequence of the terminal moiety 4 can be selected in such a way that it comprises at least 3, preferably at least 5, more preferably at least 7, further preferably at least 10 contiguous nucleotides of a sequence complementary to the nucleotide sequence of the moiety corresponding to the terminal moiety 4. Moreover, the nucleotide sequence of the moiety corresponding to the terminal moiety 4 can be selected in such a way that it comprises at least 3, preferably at least 5, more preferably at least 7, further preferably at least 10 contiguous nucleotides of a sequence complementary to the nucleotide sequence of the terminal moiety 4.

The nucleotide sequence of the terminal moiety 1 can be selected in such a way that the Tm value between the nucleotide sequence of the terminal moiety 1 and the nucleotide sequence of the moiety corresponding to the terminal moiety 1 is 5°C or more, preferably 10°C or more, more preferably 15°C or more, further preferably 20°C or more, higher than the reaction temperature.

The nucleotide sequence of the terminal moiety 2 can be selected in such a way that the Tm value between the nucleotide sequence of the terminal moiety 2 and the nucleotide sequence of the moiety corresponding to the terminal moiety 2 is 5°C or more, preferably 10°C or more, more preferably 15°C or more, further preferably 20°C or more, higher than the reaction temperature.

The nucleotide sequence of the terminal moiety 3 can be selected in such a way that the Tm value between the nucleotide sequence of the terminal moiety 3 and the nucleotide sequence of the moiety corresponding to the terminal moiety 3 is 5°C or more, preferably 10°C or more, more preferably 15°C or more, further preferably 20°C or more, higher than the reaction temperature.

The nucleotide sequence of the terminal moiety 4 can be selected in such a way that the Tm value between the nucleotide sequence of the terminal moiety 4 and the nucleotide sequence of the moiety corresponding to the terminal moiety 4 is 5°C or more, preferably 10°C or more, more preferably 15°C or more, further preferably 20°C or more, higher than the reaction temperature.

The base pair length of the nucleotide sequences of the terminal moieties (polynucleotides) 1 to 4 and the nucleotide sequences corresponding thereto in the integrated nucleic acid complex E or the integrated nucleic acid complex F is not particularly limited unless reversible structural change is inhibited. It can be set to, for example, 3 bases to 50 bases and is preferably 5 bases to 30 bases, more preferably 10 bases to 30 bases, even more preferably 10 bases to 20 bases.

The nucleic acid G1, the nucleic acid G2, the nucleic acid H1, and the nucleic acid H2 are also designed in such a way that only any one of the integrated nucleic acid complex E and the integrated nucleic acid complex F has a site to which the nucleic acid binding protein binds.

The present invention is based on the finding that structural change between nucleic acid duplexes is inhibited by the binding of a nucleic acid binding protein. In the integrated nucleic acid complex, the protein binding sequence is not varied between before and after strand exchange. For example, when the nucleic acid complex G (nucleic acid G1/nucleic acid G2) has a protein binding sequence, this protein binding sequence is also contained in the nucleic acid complex I (nucleic acid G1/nucleic acid H1) and the nucleic acid complex J (nucleic acid G2/nucleic acid H2). In this case, the nucleic acid binding protein binds to both the integrated nucleic acid complex E and the integrated nucleic acid complex F. For detecting protein binding using the present invention, the nucleic acids are designed in such a way that the nucleic acid binding protein binds to only any one of the integrated nucleic acid complexes.

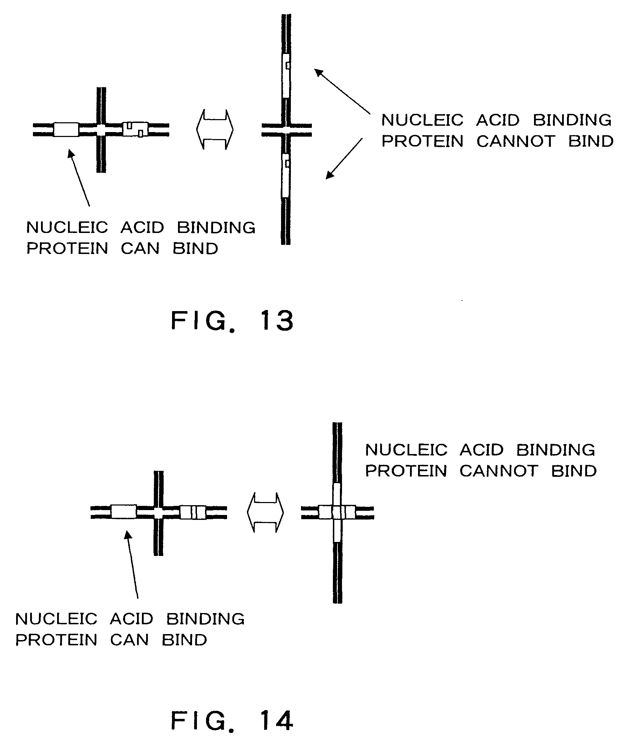

The binding of nucleic acid binding proteins that recognize and bind a nucleotide sequence is inhibited when the site recognized thereby has a variation. Thus, according to the present invention, a variation can be introduced, for example, in the protein binding sequence in such a way that only any one of the integrated nucleic acid complex E and the integrated nucleic acid complex F has a sequence to which the nucleic acid binding protein binds and the other integrated nucleic acid complex does not have a sequence to which the nucleic acid binding protein binds. Such a variation can be set to a variation that prevents the nucleic acid binding protein from binding to a binding sequence other than that of the nucleic acid complex of interest. One aspect of the specific structure thereof is shown in Figure 13.

For example, in the integrated nucleic acid complex E, the variation is introduced in the protein binding sites of the nucleic acid G1 nor the nucleic acid G2 in the nucleic acid complex G; and the variation is introduced in both the nucleic acid H1 and the nucleic acid H2 in the nucleic acid complex H. In this case, the nucleic acid binding protein can bind to the nucleic acid complex G and, according to the circumstances, to the nucleic acid complex H. However, when the integrated nucleic acid complex E is structurally changed to form the nucleic acid complex I and the nucleic acid complex J, which in turn have the variation in the nucleic acid H1 and in the nucleic acid H2, respectively. Therefore, the protein cannot bind to the integrated nucleic acid complex F.

Thus, at least one variation is introduced in each of any single-stranded nucleic acid and a strand complementary thereto (e.g., the fifth nucleotide sequence/the sequence corresponding to the fifth nucleotide sequence, the sixth nucleotide sequence/the sequence corresponding to the sixth nucleotide sequence, the fifth nucleotide sequence/the sixth nucleotide sequence, or the sequence corresponding to the fifth nucleotide sequence/the sequence corresponding to the sixth nucleotide sequence).

The variation is introduced preferably in the protein binding sequence, more preferably in a base deeply involved in protein binding in the protein binding sequence. The base deeply involved in protein binding in the protein binding sequence is known about each nucleic acid binding protein. The base deeply involved in protein binding can also be determined from a protein-nucleic acid complex structure determined by X-ray analysis or the like. Alternatively, it can also be presumed from highly conserved bases searchable from a database such as TFSEARCH (http://mbs.cbrc.jp/research/db/TFSEARCHJ.html).

The position of the variation can be determined in such a way that it does not inhibit strand exchange and inhibits the binding of the nucleic acid binding protein. For example, the variation may be introduced at locations that do not correspond to each other between the complementary strands (i.e., the variation is introduced, at each location, only in a base of one of the strands) or may be introduced at locations that correspond to each other therebetween. Moreover, when the variation is introduced at locations that correspond to each other therebetween, a variation resulting in a mismatch or a match can be introduced. However, for preventing reduction in strand exchange efficiency, the variation is preferably introduced at locations that do not correspond to each other between the complementary strands.

The type of the variation is not particularly limited as long as it can inhibit the binding of the nucleic acid binding protein. The type can be determined appropriately according to the nucleic acid binding protein of interest and the sequence recognized thereby. Examples thereof include nucleotide substitution, deletion, and insertion. The variation is preferably substitution.

The number of the variation is not particularly limited as long as it can inhibit the binding of the nucleic acid binding protein. The number can be determined appropriately according to the nucleic acid binding protein of interest and the sequence recognized thereby and is, for example, 1 to 10 variations, preferably 1 to 5 variations, more preferably 2 to 5 variations, in one strand.

The nucleic acid having the introduced variation can be synthesized according to a method known in the art.

Instead of the variation, an artificial base that does not bind to the protein may be introduced therein.

For example, a variation resulting in a structure that does not permit the protein binding can also be introduced in such a way that only any one of the integrated nucleic acid complex E and the integrated nucleic acid complex F has a sequence to which the nucleic acid binding protein binds and the other integrated nucleic acid complex does not have a sequence to which the nucleic acid binding protein binds. One aspect of the specific structure thereof is shown in Figure 14.

In this case, the full protein binding site is present only either before or after strand exchange by introducing a variation that inhibits strand exchange in progress.

The fifth and sixth nucleotide sequences and the sequences corresponding thereto are not particularly limited as long as reversible structural change is achieved between the integrated nucleic acid complex E and the integrated nucleic acid complex F. These sequences can be selected from optional sequences.

For example, the fifth nucleotide sequence in the nucleic acid G1 can be selected as a sequence that binds (preferably, hybridizes) to the sequence corresponding to the fifth nucleotide sequence in the nucleic acid G2. Moreover, the sixth nucleotide sequence in the nucleic acid H1 can be selected as a sequence that binds (preferably, hybridizes) to the sequence corresponding to the sixth nucleotide sequence in the nucleic acid H2.

The fifth nucleotide sequence can be selected in such a way that it has 70% or higher, preferably 80% or higher, more preferably 90% or higher, particularly preferably 95% or higher complementarity to the sequence corresponding to the fifth nucleotide sequence.

The sixth nucleotide sequence can be selected in such a way that it has 70% or higher, more preferably 80% or higher, particularly preferably 90% or higher, most preferably 95% or higher complementarity to the sequence corresponding to the sixth nucleotide sequence.

The sequence having 70% or higher complementarity to each nucleotide sequence is preferably a sequence complementary to the nucleotide sequence. As described later, a mismatch may be introduced therein for the purpose of improving detection sensitivity.

The fifth nucleotide sequence can be selected in such a way that it comprises at least 5, preferably at least 10, more preferably at least 15, further preferably at least 20 contiguous nucleotides of a sequence complementary to the sequence corresponding to the fifth nucleotide sequence. Moreover, the sequence corresponding to the fifth nucleotide sequence can be selected in such a way that it comprises at least 5, preferably at least 10, more preferably at least 15, further preferably at least 20 contiguous nucleotides of a sequence complementary to the fifth nucleotide sequence.

The sixth nucleotide sequence can be selected in such a way that it comprises at least 5, preferably at least 10, more preferably at least 15, further preferably at least 20 contiguous nucleotides of a sequence complementary to the sequence corresponding to the sixth nucleotide sequence. Moreover, the sequence corresponding to the sixth nucleotide sequence can be selected in such a way that it comprises at least 5, preferably at least 10, more preferably at least 15, further preferably at least 20 contiguous nucleotides of a sequence complementary to the sixth nucleotide sequence.

The fifth nucleotide sequence can be selected in such a way that the Tm value between the fifth nucleotide sequence and the sequence corresponding to the fifth nucleotide sequence is 5°C or more, preferably 10°C or more, more preferably 15°C or more, further preferably 20°C or more, higher than the reaction temperature.

The sixth nucleotide sequence can be selected in such a way that the Tm value between the sixth nucleotide sequence and the sequence corresponding to the sixth nucleotide sequence is 5°C or more, preferably 10°C or more, more preferably 15°C or more, further preferably 20°C or more, higher than the reaction temperature.

Moreover, the fifth nucleotide sequence in the nucleic acid G1 can be selected as a sequence that binds (preferably, hybridizes) to the sixth nucleotide sequence in the nucleic acid H1. Moreover, the sequence corresponding to the fifth nucleotide sequence in the nucleic acid G2 can be selected as a sequence that binds (preferably, hybridizes) to the sequence corresponding to the sixth nucleotide sequence in the nucleic acid G2.

The fifth nucleotide sequence can be selected in such a way that it has 70% or higher, preferably 80% or higher, more preferably 90% or higher, particularly preferably 95% or higher complementarity to the sixth nucleotide sequence.

The sequence corresponding to the fifth nucleotide sequence can be selected in such a way that it has 70% or higher, more preferably 80% or higher, particularly preferably 90% or higher, most preferably 95% or higher complementarity to the sequence corresponding to the sixth nucleotide sequence.

The sequence having 70% or higher complementarity to each nucleotide sequence is preferably a sequence complementary to the nucleotide sequence. As described later, a mismatch may be introduced therein for the purpose of improving detection sensitivity.

The fifth nucleotide sequence can be selected in such a way that it comprises at least 5, preferably at least 10, more preferably at least 15, further preferably at least 20 contiguous nucleotides of a sequence complementary to the sixth nucleotide sequence. Moreover, the sixth nucleotide sequence can be selected in such a way that it comprises at least 5, preferably at least 10, more preferably at least 15, further preferably at least 20 contiguous nucleotides of a sequence complementary to the fifth nucleotide sequence.

The sequence corresponding to the fifth nucleotide sequence can be selected in such a way that it comprises at least 5, preferably at least 10, more preferably at least 15, further preferably at least 20 contiguous nucleotides of a sequence complementary to the sequence corresponding to the sixth nucleotide sequence. Moreover, the sequence corresponding to the sixth nucleotide sequence can be selected in such a way that it comprises at least 5, preferably at least 10, more preferably at least 15, further preferably at least 20 contiguous nucleotides of a sequence complementary to the sequence corresponding to the fifth nucleotide sequence.

The fifth nucleotide sequence can be selected in such a way that the Tm value between the fifth nucleotide sequence and the sixth nucleotide sequence is 5°C or more, preferably 10°C or more, more preferably 15°C or more, further preferably 20°C or more, higher than the reaction temperature.

The sequence corresponding to the fifth nucleotide sequence can be selected in such a way that the Tm value between the sequence corresponding to the fifth nucleotide sequence and the sequence corresponding to the sixth nucleotide sequence is 5°C or more, preferably 10_{°}C or more, more preferably 15°C or more, further preferably 20°C or more, higher than the reaction temperature.

The base pair length of the fifth nucleotide sequence and the sequence corresponding to the fifth nucleotide sequence, and the sixth nucleotide sequence and the sequence corresponding to the sixth nucleotide sequence in the integrated nucleic acid complex E or the integrated nucleic acid complex F is not particularly limited unless reversible structural change is inhibited. The base pair length can be set to, for example, 5 base pairs to 100 base pairs and is preferably 8 base pairs to 50 base pairs, more preferably 10 base pairs to 30 base pairs.

The site to which the nucleic acid binding protein binds is at least one type of protein binding site existing in any of the duplex moieties of the integrated nucleic acid complex E or the integrated nucleic acid complex, or the branch moieties of the integrated nucleic acid complex and is located preferably in the duplex moiety, more preferably in the duplex moiety formed by the fifth nucleotide sequence and the sequence corresponding to the fifth nucleotide sequence and/or the duplex moiety formed by the sixth nucleotide sequence and the sequence corresponding to the sixth nucleotide sequence or in the duplex moiety formed by the fifth nucleotide sequence and the sixth nucleotide sequence and/or the duplex moiety formed by the sequence corresponding to the fifth nucleotide sequence and the sequence corresponding to the sixth nucleotide sequence. When proteins that recognize and bind a particular nucleotide sequence, such as transcription factors, are used, the protein binding site comprises the sequence recognized thereby and its surrounding sequence.

The binding pattern of the integrated nucleic acid complex is not particularly limited as long as the nucleic acid G1, the nucleic acid G2, the nucleic acid H1, and the nucleic acid H2 are bound to each other and reversible structural change attributed to strand exchange occurs. Preferably, the binding can be performed by hybridization.

The integrated nucleic acid complex may be an intramolecular higher-order structure of one nucleic acid strand or may be a multimer consisting of two or more nucleic acid strands. For example, the 5' terminus of the nucleic acid G1 and the 3' terminus of the nucleic acid G2 may bind to each other through a phosphodiester bond to form a single strand of the nucleic acid G1 and the nucleic acid G2. The 3' terminus of the nucleic acid G1 and the 5' terminus of the nucleic acid H1 may bind to each other through a phosphodiester bond to form a single strand of the nucleic acid G1 and the nucleic acid H1. The 3' terminus of the nucleic acid H1 and the 5' terminus of the nucleic acid H2 may bind to each other through a phosphodiester bond to form a single strand of the nucleic acid H1 and the nucleic acid H2. The 3' terminus of the nucleic acid H2 and the 5' terminus of the nucleic acid G2 may bind to each other through a phosphodiester bond to form a single strand of the nucleic acid G2 and the nucleic acid H2. For example, the integrated nucleic acid complex can assume the following structures:

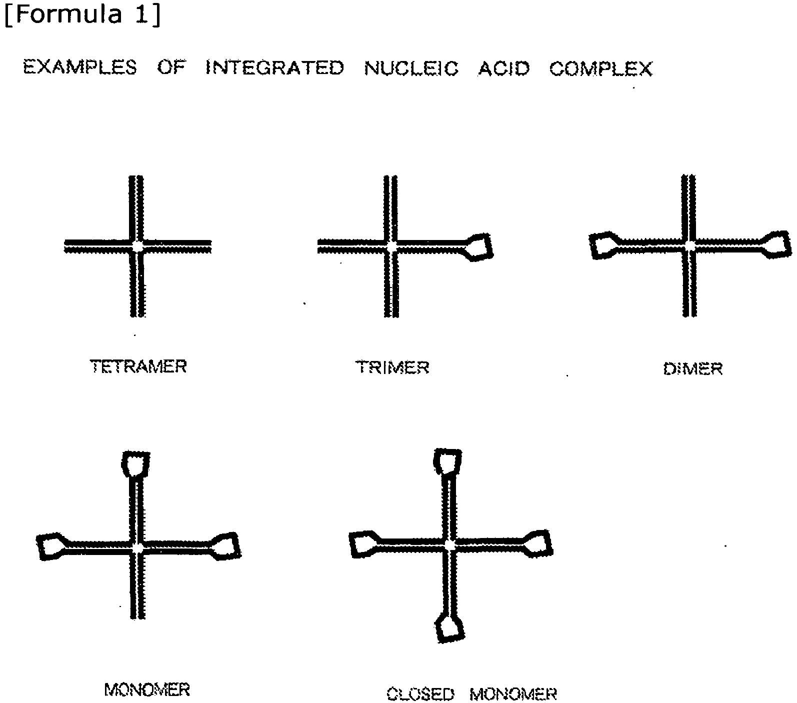

According to a preferable aspect of the present invention, there is provided the detection method according to the present invention, comprising determining the degree of structural change in the integrated nucleic acid complex, wherein
the nucleic acid G1 is a single-stranded nucleic acid consisting of a terminal moiety 1, a fifth nucleotide sequence, and a terminal moiety 3, the fifth nucleotide sequence being linked at the 3' terminus to the terminal moiety 1 and at the 5' terminus to the terminal moiety 3,
the nucleic acid G2 is a single-stranded nucleic acid consisting of a terminal moiety 2, a sequence corresponding to the fifth nucleotide sequence, and a moiety corresponding to the terminal moiety 3, the sequence corresponding to the fifth nucleotide sequence being linked at the 5' terminus to the terminal moiety 2 and at the 3' terminus to the moiety corresponding to the terminal moiety 3,
the nucleic acid H1 is a single-stranded nucleic acid consisting of a moiety corresponding to the terminal moiety 1, a sixth nucleotide sequence, and a terminal moiety 4, the sixth nucleotide sequence being linked at the 5' terminus to the moiety corresponding to the terminal moiety 1 and at the 3' terminus to the terminal moiety 4, and
the nucleic acid H2 is a single-stranded nucleic acid consisting of a moiety corresponding to the terminal moiety 4, a sequence corresponding to the sixth nucleotide sequence, and a moiety corresponding to the terminal moiety 2, the sequence corresponding to the sixth nucleotide sequence being linked at the 5' terminus to the moiety corresponding to the terminal moiety 4 and at the 3' terminus to the moiety corresponding to the terminal moiety 2; wherein
the terminal moieties 1 to 4 and the moieties corresponding thereto are polynucleotides, wherein
a low stable base pair group (e.g., 2 to 10 base pairs) is formed between the 5'-terminal nucleotide sequence of the terminal moiety 1 and the 3'-terminal nucleotide sequence of the terminal moiety 2 and between the 3'-terminal nucleotide sequence of the moiety corresponding to the terminal moiety 1 and the 5'-terminal nucleotide sequence of the moiety corresponding to the terminal moiety 2, and
a highly stable base pair group (e.g., 2 to 10 base pairs) is formed between the 5'-terminal nucleotide sequence of the terminal moiety 1 and the 3'-terminal nucleotide sequence of the moiety corresponding to the terminal moiety 1 and between the 3'-terminal nucleotide sequence of the terminal moiety 2 and the 5'-terminal nucleotide sequence of the moiety corresponding to the terminal moiety 2, and
a low stable base pair group (e.g., 2 to 10 base pairs) is formed between the 3'-terminal nucleotide sequence of the terminal moiety 3 and the 5'-terminal nucleotide sequence of the terminal moiety 4 and between the 5'-terminal nucleotide sequence of the moiety corresponding to the terminal moiety 3 and the 3'-terminal nucleotide sequence of the moiety corresponding to the terminal moiety 4, and
a highly stable base pair group (e.g., 2 to 10 base pairs) is formed between the 3'-terminal nucleotide sequence of the terminal moiety 3 and the 5'-terminal nucleotide sequence of the moiety corresponding to the terminal moiety 3 and between the 5'-terminal nucleotide sequence of the terminal moiety 4 and the 3'-terminal nucleotide sequence of the moiety corresponding to the terminal moiety 4; and wherein
a variation is introduced in such a way that only any one of the integrated nucleic acid complex E and the integrated nucleic acid complex F has a sequence to which the nucleic acid binding protein binds (in this context, the variation is introduced in the protein binding sequence and introduced at locations that do not correspond to each other between the complementary strands, and the number of the variation is 1 to 5). In a more preferable aspect, there is provided the detection method according to the present invention, wherein the degree of structural change is determined using fluorescence resonance energy transfer.

### Nucleic acid binding protein

In the present specification, the "nucleic acid binding protein" is not particularly limited as long as it is a protein binding to nucleic acids. Examples thereof include transcription factors, structural proteins, repair enzymes, branch binding proteins, recombinases, polymerases, RNA binding proteins, single-stranded DNA binding proteins, and DNA or RNA cleaving enzymes.

Examples of the transcription factors include TFIIID, NF_{κ}B, p53, and AP1.

Examples of the structural proteins include telomere DNA binding proteins (e.g., human TRF1, TRF2, and POT1).

Examples of the repair enzymes include mismatch binding proteins, for example, *E. coli* MutS and human MSH2 and MSH6.

Examples of the branch binding proteins include *E. coli* RuvA.

Examples of the recombinases include *E. coli* RecA and *Yeast* Rad51.

Examples of the polymerases include RNA polymerase and DNA polymerase.

Examples of the RNA binding proteins include U1 snRNP, U2 snRNP, or U6 snRNP contained in a spliceosome complex, poly A binding proteins, RNA repair enzymes, and RISC complex.

Examples of the single-stranded DNA binding proteins include *E. coli* SSB and human RPA. The single-stranded DNA binding proteins bind to, for example, the single-stranded moiety in at least one of the nucleic acid duplex A and the nucleic acid duplex B and inhibit the formation of a nucleic acid duplex complex.

Examples of the DNA or RNA cleaving enzymes include restriction enzymes (e.g., EcoRI and HindIII), nicking enzymes, and Dicer. The cleavage of the single or double strands inhibits the formation of a nucleic acid duplex complex and subsequent structural change.

The nucleic acid binding protein may be used in a purified form or may be used as a cell extract.

### Detection method

### Fi rst aspect

The nucleic acid duplex A and the nucleic acid duplex B having a terminal single-stranded moiety bind to each other at their terminal sequences to form a nucleic acid duplex complex, which is subsequently structurally changed to form new nucleic acid duplexes C and D. The newly formed nucleic acid duplex C and nucleic acid duplex D neither bind to each other at their terminal sequences nor form a complex. However, upon binding of a nucleic acid binding protein to the nucleic acid duplex A and/or the nucleic acid duplex B, such formation of a nucleic acid duplex complex and the subsequent formation of new nucleic acid duplexes are inhibited. Thus, binding between a nucleic acid and a nucleic acid binding protein can be detected using the degree of structural change in the nucleic acid duplex complex as an index, specifically, using an amount of the nucleic acid duplex and/or the nucleic acid duplex complex as an index.

The binding between a nucleic acid and a nucleic acid binding protein can be detected by performing the followings:
(i) contacting the nucleic acid binding protein with the nucleic acid duplex A and the nucleic acid duplex B; and
(ii) determining the degree of structural change in the nucleic acid duplex complex.

Specifically, in (ii), the degree of structural change in the nucleic acid duplex complex can be determined by measuring an amount of a nucleic acid duplex consisting of the nucleic acid A1 and the nucleic acid B1 (nucleic acid duplex C) and/or a nucleic acid duplex consisting of the nucleic acid A2 and the nucleic acid B2 (nucleic acid duplex D).

The detection method may further comprise comparing the measured amount with an amount of the nucleic acid duplex C and/or the nucleic acid duplex D measured in the absence of the nucleic acid binding protein. In this case, when the amount of the nucleic acid duplex C and/or the nucleic acid duplex D formed in the presence of the nucleic acid binding protein is lower than that in the absence of the nucleic acid binding protein, the nucleic acid binding protein can be determined to bind to the nucleic acid.

Moreover, in (ii), the degree of structural change in the nucleic acid duplex complex can also be determined by measuring an amount of the nucleic acid duplex A and/or the nucleic acid duplex B.

The detection method may further comprise comparing the measured amount with an amount of the nucleic acid duplex A and/or the nucleic acid duplex B measured in the absence of the nucleic acid binding protein. In this case, when the amount of the nucleic acid duplex A and/or the nucleic acid duplex B in the presence of the nucleic acid binding protein is higher than that in the absence of the nucleic acid binding protein, the nucleic acid binding protein can be determined to bind to the nucleic acid.

In this context, the "amount of the nucleic acid duplex A and/or the nucleic acid duplex B" also encompasses an amount of the nucleic acid duplex A and/or the nucleic acid duplex B in the nucleic acid duplex complex.

In the detection method of the first aspect according to the present invention, the "contacting" may be performed by simultaneously mixing all the nucleic acid duplex A, the nucleic acid binding protein, and the nucleic acid duplex B or by mixing the nucleic acid duplex A and the nucleic acid binding protein and then adding the nucleic acid duplex B to the mixture.

In the detection method of the first aspect according to the present invention, structural change efficiency can be enhanced by preparing any one of the nucleic acid duplex A and the nucleic acid duplex B in excess of the other nucleic acid duplex. Moreover, structural change with sufficient efficiency can be caused even in equal amounts of these nucleic acid duplexes by selecting appropriate sequences that do not inhibit the occurrence of structural change.

### Second aspect

The integrated nucleic acid complex E is a nucleic acid complex formed by the nucleic acid complex G and the nucleic acid complex H bound to each other. The integrated nucleic acid complex F is a nucleic acid complex formed by the nucleic acid complex I and the nucleic acid complex J bound to each other. The integrated nucleic acid complex E is converted through strand exchange reaction between the nucleic acid complex G and the nucleic acid complex H to a new integrated nucleic acid complex F consisting of the nucleic acid complex I and the nucleic acid complex J. Moreover, the integrated nucleic acid complex F is also converted through strand exchange reaction between the nucleic acid complex I and the nucleic acid complex J to a new integrated nucleic acid complex E consisting of the nucleic acid complex G and the nucleic acid complex H. Such structural change between the integrated nucleic acid complex E and the integrated nucleic acid complex F is reversibly repeated. However, upon binding of a nucleic acid binding protein to any of the nucleic acid complex G, the nucleic acid complex H, the nucleic acid complex I, and the nucleic acid complex J, such reversible structural change is inhibited to stabilize the structure of either the integrated nucleic acid complex E or the integrated nucleic acid complex F.

In this case, labels are used which allow the integrated nucleic acid complex E and the integrated nucleic acid complex F to show different values (e.g., the nucleic acid G1 and the nucleic acid G2, the nucleic acid G1 and the nucleic acid H1, the nucleic acid G1 and the nucleic acid H2, the nucleic acid G2 and the nucleic acid H1, the nucleic acid G2 and the nucleic acid H2, or the nucleic acid H1 and the nucleic acid H2 are fluorescently labeled or quenching-labeled, respectively). As a result, the values of the labels are also stabilized (i.e., the values of the labels are increased or decreased) with the stabilization of the structure.

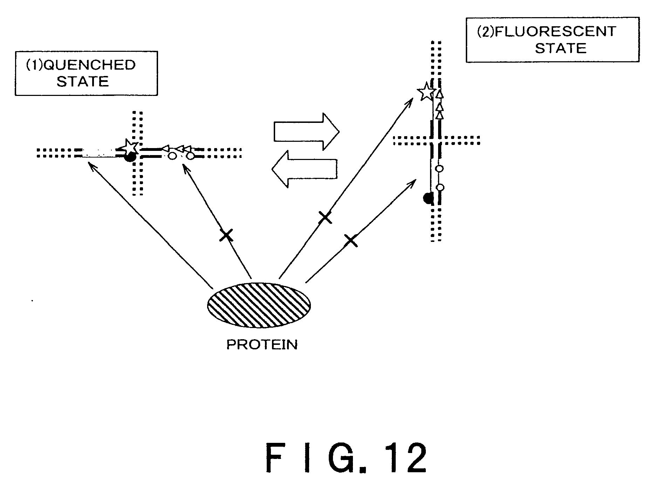

Thus, binding between a nucleic acid and a nucleic acid binding protein can be detected using the degree of structural change in the integrated nucleic acid complex as an index, specifically, using an amount of the integrated nucleic acid complex E or the integrated nucleic acid complex F as an index. One aspect of the detection of the second aspect is shown in Figure 12.

The binding between a nucleic acid and a nucleic acid binding protein can be detected by performing the followings:
(iii) contacting the nucleic acid binding protein with the integrated nucleic acid complex E or the integrated nucleic acid complex F; and
(iv) determining the degree of structural change in the integrated nucleic acid complex.

Specifically, in (iv), the degree of structural change in the nucleic acid complex can be determined by measuring an amount of the integrated nucleic acid complex of interest.

For example, when the nucleic acid complexes are designed in such a way that the nucleic acid binding protein binds to the nucleic acid complex G or the nucleic acid complex H, the amount of the nucleic acid binding protein bound to the nucleic acid can be determined from the amount of the integrated nucleic acid complex E. Alternatively, when the nucleic acid complexes are designed in such a way that the nucleic acid binding protein binds to nucleic acid complex I or the nucleic acid complex J, the amount of the nucleic acid binding protein bound to the nucleic acid can be determined from the amount of the integrated nucleic acid complex F.

In the detection method of the second aspect according to the present invention, the "contacting" can be performed by simultaneously mixing the integrated nucleic acid complex F or the integrated nucleic acid complex G with the nucleic acid binding protein.

The detection method of the second aspect according to the present invention needs only to mix the nucleic acid complexes prepared in advance with the protein and is therefore advantageous in that improvement in operability can be expected, such as a decreased number of operation steps and reduction in operation mistake attributed to complication.

In the detection method according to the present invention, the nucleic acid binding protein may be contacted together with its effector molecule (protein).

According to the detection method according to the present invention, the binding potential between the nucleic acid and the nucleic acid binding protein can also be calculated from the amount of the obtained nucleic acid formed.

According to the detection method according to the present invention, an unknown sequence to which a nucleic acid binding protein binds can be determined by determining whether or not the protein can bind to a particular sequence using the detection method of the present invention.

According to the detection method according to the present invention, an unknown nucleic acid binding protein that binds to a binding sequence of interest can be identified by determining whether or not a test protein can bind to the sequence of interest using the detection method of the present invention.

In the detection method according to the present invention, the structural change is not a reaction that requires a catalyst (e.g., structural change in the first aspect occurs upon contact of nucleic acid duplexes of which terminal single-stranded moieties are capable of binding to each other). However, the reaction can also be promoted using a catalyst such as *E. coli* RuvB proteins.

The detection method according to the present invention may be performed in the presence of a substance that promotes hybridization (e.g., polyethylene glycol or dextran sulfate). Moreover, the detection method according to the present invention may be performed in the presence of a substance that has the effect of preventing protein or DNA adsorption or protecting proteins or DNA (e.g., bovine serum albumin or DNA irrelevant thereto).

The detection method according to the present invention can be performed at a temperature suitable for the nucleic acid binding protein used. In general, DNA binding proteins can be detected at 20°C to 30°C. Thermophilic proteins can be detected at a higher temperature.

Any method of measuring an amount of the nucleic acid duplex, the nucleic acid duplex complex, or the nucleic acid complex can be used as long as it is the principle or a method known by those skilled in the art. Examples thereof include methods using radioactive labeling, fluorescent labeling, electrophoresis, or fluorescence resonance energy transfer. The amount of the nucleic acid duplex, the nucleic acid duplex complex, or the nucleic acid complex can be measured preferably by a method using fluorescent labeling or fluorescence resonance energy transfer, more preferably by a method using fluorescence resonance energy transfer that achieves assay without the need of washing procedures (for removing excessive labels) or electrophoresis.

According to the first aspect, for example, the nucleic acid A1 can be labeled at the 5' terminus with a fluorescent material, while the nucleic acid B1 can be labeled at the 3' terminus with a material quenching the fluorescence of the fluorescent material. In this case, fluorescence from the nucleic acid A1 is emitted in the nucleic acid duplex A formed thereby and is however quenched by the label of the nucleic acid B1 in the nucleic acid duplex C newly formed through the hybridization of the nucleic acid A1 to the nucleic acid B1. Therefore, the amount of the nucleic acid duplex A can be measured easily by measuring the fluorescence value.

Moreover, the nucleic acid A1 can be labeled at the 5' terminus with a fluorescent material, while the nucleic acid A2 can be labeled at the 3' terminus with a material capable of quenching the fluorescence of the fluorescent material. In this case, fluorescence from the nucleic acid A1 is quenched in the nucleic acid duplex A formed thereby and can however be emitted in the nucleic acid duplex C newly formed through the hybridization of the nucleic acid A1 to the nucleic acid B1. Therefore, the amount of the nucleic acid duplex C can be measured by measuring the fluorescence value.

Any of the nucleic acid A1, the nucleic acid A2, the nucleic acid B1, and the nucleic acid B2 may be labeled. The position of each label may be within the nucleic acid or at the terminus thereof and is preferably at the terminus thereof.

According to the second aspect, for example, the fifth nucleotide sequence in the nucleic acid G1 can be labeled at the 3' terminus with a fluorescent material, while the sequence corresponding to the fifth nucleotide sequence in the nucleic acid G2 can be labeled at the 5' terminus with a material quenching the fluorescence of the fluorescent material. In this case, fluorescence from the nucleic acid G1 is quenched in the nucleic acid complex G formed thereby and is however emitted by the label of the nucleic acid G1 in the nucleic acid complex I formed thereby. Therefore, the amount of the single-stranded nucleic acid complex E or the single-stranded nucleic acid complex F can be measured easily by measuring the fluorescence value. The quenching material and the fluorescent material are also interchangeable.

Moreover, the fifth nucleotide sequence in the nucleic acid G1 can be labeled at the 3' terminus with a fluorescent material, while the sequence corresponding to the sixth nucleotide sequence in the nucleic acid H2 can be labeled at the 3' terminus with a material capable of quenching the fluorescence of the fluorescent material. In this case, fluorescence from the nucleic acid G1 is quenched in the nucleic acid complex G formed thereby and can however be emitted in the nucleic acid complex I formed by the nucleic acid G1. Therefore, the amount of the single-stranded nucleic acid complex E or the single-stranded nucleic acid complex F can be measured easily by measuring the fluorescence value. The quenching material and the fluorescent material are also interchangeable.

Furthermore, the fifth nucleotide sequence in the nucleic acid G1 can be labeled at the 5' terminus with a fluorescent material, while the sequence corresponding to the sixth nucleotide sequence in the nucleic acid H2 can be labeled at the 5' terminus with a material capable of quenching the fluorescence of the fluorescent material. In this case, fluorescence from the nucleic acid G1 can be emitted in the nucleic acid complex G formed thereby and is however quenched in the nucleic acid complex I formed by the nucleic acid G1. Therefore, the amount of the single-stranded nucleic acid complex E or the single-stranded nucleic acid complex F can be measured easily by measuring the fluorescence value. The quenching material and the fluorescent material are also interchangeable.

Any of the nucleic acid G1 (preferably the fifth nucleotide sequence), the nucleic acid G2 (preferably the sequence corresponding to the fifth nucleotide sequence), the nucleic acid H1 (preferably the sixth nucleotide sequence), and the nucleic acid H2 (preferably the sequence corresponding to the sixth nucleotide sequence) may be labeled. The position of each label may be within the nucleic acid or at the terminus thereof and is preferably a position at which the difference in distance between the positions of the labels is increased between before and after structural change.

Examples of the fluorescent label include fluorescein, TAMRA, HEX, TET, JOE, Cy5, Cy3, Alexa series (Molecular Probes, Inc.), and green fluorescence protein (GFP). The fluorescent label is preferably fluorescein, TAMRA, HEX, TET, JOE, Cy5, Cy3, or Alexa series.

Examples of the quenching material include DABCYL, TAMRA, and BHQ (Molecular Probes, Inc.). The quenching material can be selected appropriately according to the fluorescent material used.

The fluorescence resonance energy transfer can be used not only as quenching but also as shift in the fluorescence wavelength. This fluorescence energy transfer can be measured using a general-purpose apparatus such as a spectrophotofluorometer, a real-time PCR apparatus, or a fluorescence plate reader.

### Introduction of mismatch

It is known that the introduction of a mismatch in nucleotide sequences reduces strand exchange efficiency in nucleotide strand exchange reaction (Panyutin IG et al. J. Mol. Biol. 1993, 230, 413-424). However, the present inventors have found that the introduction of a mismatch in nucleic acids used in a nucleic acid complex can potentiate the effect of suppressing strand exchange reaction by a nucleic acid binding protein, without largely reducing strand exchange efficiency (Example 4). Moreover, the present inventors have also found that the use of the mismatch introduction improves an S/N ratio in the detection of a nucleic acid binding protein (Example 4).

The fact that the mismatch introduction potentiates the effect of suppressing strand exchange reaction and improves an S/N ratio is advantageous in that cost can be reduced because assay is achieved using small amounts of proteins in the detection method or screening method according to the present invention. Particularly, in *in-vivo* detection of proteins, this fact is advantageous in that valuable samples can be saved and detailed analysis is achieved because of the widened range of measurement.

The mismatch is introduced in such a way that the mismatch exists between complementary nucleic acid strands after exchange in strand exchange reaction.

For example, in the nucleic acid duplex complex, mismatch base(s) can be introduced alone or in combination, selected from: one or more mismatch bases in the nucleotide sequence (preferably the first nucleotide sequence) of the nucleic acid A1 with respect to the nucleotide sequence of the nucleic acid B1; one or more mismatch bases in the nucleotide sequence (preferably the fourth nucleotide sequence) of the nucleic acid B1 with respect to the nucleotide sequence of the nucleic acid A1; one or more mismatch bases in the nucleotide sequence (preferably the sequence corresponding to the first nucleotide sequence) of the nucleic acid A2 with respect to the nucleotide sequence of the nucleic acid B2; and one or more mismatch bases in the nucleotide sequence (preferably the sequence corresponding to the fourth nucleotide sequence) of the nucleic acid B2 with respect to the nucleotide sequence of the nucleic acid A2.

In this case, the position of mismatch introduction is not particularly limited as well as it is in a sequence to undergo strand exchange and unless it largely deteriorates strand exchange efficiency. The position is preferably in a sequence other than the sequence to which a nucleic acid binding protein binds.

In this case, the number of the mismatch is not particularly limited unless it largely deteriorates strand exchange efficiency. For example, the number of the mismatch introduced can be the number in which the first nucleotide sequence to the fourth nucleotide sequence, the fourth nucleotide sequence to the first nucleotide sequence, the sequence corresponding to the first nucleotide sequence to the sequence corresponding to the fourth nucleotide sequence, and the sequence corresponding to the fourth nucleotide sequence to the sequence corresponding to the first nucleotide sequence respectively have 90% or higher, preferably 92% or higher, more preferably 95% or higher, even more preferably 98% or higher, particularly preferably 99% or higher complementarity.

In this case, the mismatch forming base(s) can be introduced alone or in combination in any of the nucleic acid A1, the nucleic acid A2, the nucleic acid B1, and the nucleic acid B2 in the nucleic acid duplex complex and are preferably introduced in such a way that the mismatch is formed between the first nucleotide sequence and the fourth nucleotide sequence and/or between the sequence corresponding to the first nucleotide sequence and the sequence corresponding to the fourth nucleotide sequence, more preferably between the first nucleotide sequence and the fourth nucleotide sequence and between the sequence corresponding to the first nucleotide sequence and the sequence corresponding to the fourth nucleotide sequence.

In this case, the mismatch forming base(s) may be introduced at locations that do not correspond to each other between the complementary strands before strand exchange or may be introduced at locations that correspond to each other therebetween. The mismatch forming base(s) are preferably introduced at locations that correspond to each other between the complementary strands before strand exchange.

In a preferable aspect for the nucleic acid duplex complex, the mismatch is introduced between the first nucleotide sequence and the fourth nucleotide sequence and between the sequence corresponding to the first nucleotide sequence and the sequence corresponding to the fourth nucleotide sequence; and the number of the mismatch is the number in which the complementarity between the first nucleotide sequence and the fourth nucleotide sequence and the complementarity between the sequence corresponding to the first nucleotide sequence and the sequence corresponding to the fourth nucleotide sequence are respectively 90% or higher, preferably 92% or higher, more preferably 95% or higher, even more preferably 98% or higher, particularly preferably 99% or higher complementarity.

In the integrated complex, mismatch base(s) can be introduced alone or in combination, selected from: one or more mismatch bases in the nucleotide sequence (preferably the fifth nucleotide sequence) of the nucleic acid G1 with respect to the nucleotide sequence of the nucleic acid H1 or the nucleotide sequence of the nucleic acid G2; one or more mismatch bases in the nucleotide sequence (preferably the sequence corresponding to the fifth nucleotide sequence) of the nucleic acid G2 with respect to the nucleotide sequence of the nucleic acid H2 or the nucleotide sequence of the nucleic acid G1; one or more mismatch bases in the nucleotide sequence (preferably the sixth nucleotide sequence) of the nucleic acid H1 with respect to the nucleotide sequence of the nucleic acid G1 or the nucleotide sequence of the nucleic acid H2; and one or more mismatch bases in the nucleotide sequence (preferably the sequence corresponding to the sixth nucleotide sequence) of the nucleic acid H2 with respect to the nucleotide sequence of the nucleic acid G2 or the nucleotide sequence of the nucleic acid H1.

In this case, the position of mismatch introduction is not particularly limited as well as it is in a sequence to undergo strand exchange and unless it largely deteriorates strand exchange efficiency. The position is preferably in a sequence other than the sequence to which a nucleic acid binding protein binds.

In this case, the number of the mismatch is not particularly limited unless it largely deteriorates strand exchange efficiency. For example, the number of the mismatch introduced can be the number in which the fifth nucleotide sequence to the sixth nucleotide sequence, the sixth nucleotide sequence to the fifth nucleotide sequence, the sequence corresponding to the fifth nucleotide sequence to the sequence corresponding to the sixth nucleotide sequence, the sequence corresponding to the sixth nucleotide sequence to the sequence corresponding to the fifth nucleotide sequence, the fifth nucleotide sequence to the sequence corresponding to the fifth nucleotide sequence, the sequence corresponding to the fifth nucleotide sequence to the fifth nucleotide sequence, the sixth nucleotide sequence to the sequence corresponding to the sixth nucleotide sequence, and the sequence corresponding to the sixth nucleotide sequence to the sixth nucleotide sequence respectively have 90% or higher, preferably 92% or higher, more preferably 95% or higher, even more preferably 98% or higher, particularly preferably 99% or higher complementarity.

In this case, the mismatch forming base(s) can be introduced alone or in combination in any of the nucleic acid G1, the nucleic acid G2, the nucleic acid H1, and the nucleic acid H2 in the nucleic acid complex and are preferably introduced in such a way that the mismatch is formed between the fifth nucleotide sequence and the sixth nucleotide sequence and/or between the sequence corresponding to the fifth nucleotide sequence and the sequence corresponding to the sixth nucleotide sequence, or between the fifth nucleotide sequence and the sequence corresponding to the fifth nucleotide sequence and/or between the sixth nucleotide sequence and the sequence corresponding to the sixth nucleotide sequence, more preferably between the fifth nucleotide sequence and the sixth nucleotide sequence and between the sequence corresponding to the fifth nucleotide sequence and the sequence corresponding to the sixth nucleotide sequence, or between the fifth nucleotide sequence and the sequence corresponding to the fifth nucleotide sequence and between the sixth nucleotide sequence and the sequence corresponding to the sixth nucleotide sequence. In this context, the mismatch forming base(s) are preferably introduced in such a way that the balance of reversible structural change is tilted toward a protein binding sequence-free integrated nucleic acid complex, of the integrated nucleic acid complex E and the integrated nucleic acid complex F. Thus, when the nucleic acids are designed in such a way that the integrated nucleic acid complex E has a protein binding sequence, the mismatch forming base(s) are preferably introduced in such a way that the mismatch is formed between the fifth nucleotide sequence and the sequence corresponding to the fifth nucleotide sequence and/or between the sixth nucleotide sequence and the sequence corresponding to the sixth nucleotide sequence.

In a preferable aspect for the integrated complex, the mismatch is introduced between the fifth nucleotide sequence and the sixth nucleotide sequence and between the sequence corresponding to the fifth nucleotide sequence and the sequence corresponding to the sixth nucleotide sequence, and the number of the mismatch is the number in which the complementarity between the fifth nucleotide sequence and the sixth nucleotide sequence and the complementarity between the sequence corresponding to the fifth nucleotide sequence and the sequence corresponding to the sixth nucleotide sequence are respectively 90% or higher, preferably 92% or higher, more preferably 95% or higher, even more preferably 98% or higher, particularly preferably 99% or higher complementarity; and the mismatch is introduced between the fifth nucleotide sequence and the sequence corresponding to the fifth nucleotide sequence and between the sixth nucleotide sequence and the sequence corresponding to the sixth nucleotide sequence, and the number of the mismatch is the number in which the complementarity between the fifth nucleotide sequence and the fifth nucleotide sequence and the complementarity between the sixth nucleotide sequence and the sequence corresponding to the sixth nucleotide sequence are respectively 90% or higher, preferably 92% or higher, more preferably 95% or higher, even more preferably 98% or higher, particularly preferably 99% or higher complementarity.

### Multiplexed detection

According to the detection method of the present invention, the bindings of a plurality of nucleic acid binding proteins to nucleic acids can be detected simultaneously using a plurality of nucleic acid complexes (multiplexed detection).

The multiplexed detection can enhance the efficiency of screening of an inhibitor or the like for a nucleic acid binding protein. Influence on proteins other than the target is responsible for adverse reaction and must therefore be confirmed to be small at as an early stage as possible. According to the multiplexed detection, the effect of a drug on the target protein is examined, while its influence on other proteins can also be examined.

The multiplexed detection can also assay binding activities to a plurality of intracellular proteins. Therefore, life phenomena that take place intracellularly can be grasped in more detail. According to the multiplexed detection, more detailed findings can be obtained on nucleic acid binding proteins of which nucleic acid binding activities are known to be changed through posttranslational modification or interaction with other proteins.

The sequences of nucleic acids used in the multiplexed detection method are not particularly limited as long as a plurality of nucleic acid complexes independently cause structural change. The sequence of each nucleic acid in a reaction system associated with one nucleic acid complex can be designed in such a way that it has influence on neither binding between nucleic acids in other reaction systems nor the structural change in the nucleic acid complex.

In multiplexed detection using the nucleic acid duplex complex, for example, the first to fourth nucleotide sequences and the sequences corresponding thereto in each reaction system as well as the nucleotide sequences of nucleic acids in other reaction systems can be designed as follows:

The first nucleotide sequence in each reaction system can be selected in such a way that it has 70% or higher, preferably 80% or higher, more preferably 90% or higher, further preferably 95% or higher, particularly preferably 98% or higher complementarity to the sequence corresponding to the first nucleotide sequence.

The fourth nucleotide sequence in each reaction system can be selected in such a way that it has 70% or higher, more preferably 80% or higher, particularly preferably 90% or higher, further preferably 95% or higher, particularly preferably 98% or higher complementarity to the sequence corresponding to the fourth nucleotide sequence.

The sequence having 70% or higher complementarity to each nucleotide sequence is preferably a sequence complementary to the nucleotide sequence.

The first nucleotide sequence in each reaction system can be selected in such a way that it comprises at least 5, preferably at least 10, more preferably at least 15, further preferably at least 20, particularly preferably at least 25, most preferably at least 30 contiguous nucleotides of a sequence complementary to the sequence corresponding to the first nucleotide sequence. Moreover, the sequence corresponding to the first nucleotide sequence in each reaction system can be selected in such a way that it comprises at least 5, preferably at least 10, more preferably at least 15, further preferably at least 20, particularly preferably at least 25, most preferably at least 30 contiguous nucleotides of a sequence complementary to the first nucleotide sequence.

The fourth nucleotide sequence in each reaction system can be selected in such a way that it comprises at least 5, preferably at least 10, more preferably at least 15, further preferably at least 20, particularly preferably at least 25, most preferably at least 30 contiguous nucleotides of a sequence complementary to the sequence corresponding to the fourth nucleotide sequence. Moreover, the sequence corresponding to the fourth nucleotide sequence in each reaction system can be selected in such a way that it comprises at least 5, preferably at least 10, more preferably at least 15, further preferably at least 20, particularly preferably at least 25, most preferably at least 30 contiguous nucleotides of a sequence complementary to the fourth nucleotide sequence.

The first nucleotide sequence in each reaction system can be selected in such a way that the Tm value between the first nucleotide sequence and the sequence corresponding to the first nucleotide sequence is 5°C or more, preferably 10°C or more, more preferably 15°C or more, further preferably 20°C or more, particularly preferably 25°C or more, higher than the reaction temperature.

The fourth nucleotide sequence in each reaction system can be selected in such a way that the Tm value between the fourth nucleotide sequence and the sequence corresponding to the fourth nucleotide sequence is 5°C or more, preferably 10°C or more, more preferably 15°C or more, further preferably 20°C or more, particularly preferably 25°C or more, higher than the reaction temperature.

The second nucleotide sequence in each reaction system can be selected in such a way that it has 70% or higher, preferably 80% or higher, more preferably 90% or higher, further preferably 95% or higher, particularly preferably 98% or higher complementarity to the sequence corresponding to the second nucleotide sequence. On the other hand, the second nucleotide sequence in each reaction system can be selected in such a way that it has complementarity lower than 50%, more preferably lower than 30%, particularly preferably lower than 20%, to each of the third nucleotide sequence and the sequence corresponding to the third nucleotide sequence in the same reaction system thereas and the nucleotide sequences of nucleic acids in other reaction systems. Moreover, the sequence corresponding to the second nucleotide sequence in each reaction system can be selected in such a way that it has complementarity lower than 50%, more preferably lower than 30%, particularly preferably lower than 20%, to each of the third nucleotide sequence and the sequence corresponding to the third nucleotide sequence and the nucleotide sequences of nucleic acids in other reaction systems.

The third nucleotide sequence in each reaction system can be selected in such a way that it has 70% or higher, preferably 80% or higher, more preferably 90% or higher, further preferably 95% or higher, particularly preferably 98% or higher complementarity to the sequence corresponding to the third nucleotide sequence. On the other hand, the third nucleotide sequence in each reaction system can be selected in such a way that it has complementarity lower than 50%, more preferably lower than 30%, particularly preferably lower than 20%, to each of the second nucleotide sequence and the sequence corresponding to the second nucleotide sequence in the same reaction system thereas and the nucleotide sequences of nucleic acids in other reaction systems. Moreover, the sequence corresponding to the third nucleotide sequence in each reaction system can be selected in such a way that it has complementarity lower than 50%, more preferably lower than 30%, particularly preferably lower than 20%, to each of the second nucleotide sequence and the sequence corresponding to the second nucleotide sequence in the same reaction system thereas and the nucleotide sequences of nucleic acids in other reaction systems.

The sequence having 70% or higher complementarity to each nucleotide sequence is preferably a sequence complementary to the nucleotide sequence.

The second nucleotide sequence in each reaction system can be selected in such a way that it comprises at least 5, preferably at least 10, more preferably at least 15, further preferably at least 20 contiguous nucleotides of a sequence complementary to the sequence corresponding to the second nucleotide sequence. Moreover, the sequence corresponding to the second nucleotide sequence in each reaction system can be selected in such a way that it comprises at least 5, preferably at least 10, more preferably at least 15, further preferably at least 20 contiguous nucleotides of a sequence complementary to the second nucleotide sequence.

On the other hand, the second nucleotide sequence in each reaction system may contain contiguous nucleotides of a sequence complementary to any of the third nucleotide sequence and the sequence corresponding to the third nucleotide sequence in the same reaction system thereas and the nucleotide sequences of nucleic acids in other reaction systems. However, the number of the nucleotides is 4 at the maximum, preferably 3 or less, more preferably 2 or less. Moreover, the sequence corresponding to the second nucleotide sequence in each reaction system may contain contiguous nucleotides of a sequence complementary to any of the third nucleotide sequence and the sequence corresponding to the third nucleotide sequence in the same reaction system thereas and the nucleotide sequences of nucleic acids in other reaction systems. However, the number of the nucleotides is 4 at the maximum, preferably 3 or less, more preferably 2 or less. In addition, the third nucleotide sequence in each reaction system may contain contiguous nucleotides of a sequence complementary to any of the second nucleotide sequence and the sequence corresponding to the second nucleotide sequence in the same reaction system thereas. However, the number of the nucleotides is 4 at the maximum, preferably 3 or less, more preferably 2 or less. Moreover, the sequence corresponding to the third nucleotide sequence in each reaction system may contain contiguous nucleotides of a sequence complementary to any of the second nucleotide sequence and the sequence corresponding to the second nucleotide sequence in each reaction system. However, the number of the nucleotides is 4 at the maximum, preferably 3 or less, more preferably 2 or less. Furthermore, the nucleotide sequences of nucleic acids in other reaction systems may contain contiguous nucleotides of a sequence complementary to any of the second nucleotide sequence and the sequence corresponding to the second nucleotide sequence in each reaction system. However, the number of the nucleotides is 4 at the maximum, preferably 3 or less, more preferably 2 or less.

The third nucleotide sequence in each reaction system can be selected in such a way that it comprises at least 5, preferably at least 10, more preferably at least 15, further preferably at least 20 contiguous nucleotides of a sequence complementary to the sequence corresponding to the third nucleotide sequence. Moreover, the sequence corresponding to the third nucleotide sequence in each reaction system can be selected in such a way that it comprises at least 5, preferably at least 10, more preferably at least 15, further preferably at least 20 contiguous nucleotides of a sequence complementary to the third nucleotide sequence.

On the other hand, the third nucleotide sequence in each reaction system may contain contiguous nucleotides of a sequence complementary to any of the second nucleotide sequence and the sequence corresponding to the second nucleotide sequence in the same reaction system thereas and the nucleotide sequences of nucleic acids in other reaction systems. However, the number of the nucleotides is 4 at the maximum, preferably 3 or less, more preferably 2 or less. Moreover, the sequence corresponding to the third nucleotide sequence in each reaction system may contain contiguous nucleotides of a sequence complementary to any of the second nucleotide sequence and the sequence corresponding to the second nucleotide sequence in the same reaction system thereas and the nucleotide sequences of nucleic acids in other reaction systems. However, the number of the nucleotides is 4 at the maximum, preferably 3 or less, more preferably 2 or less. In addition, the second nucleotide sequence in each reaction system may contain contiguous nucleotides of a sequence complementary to any of the third nucleotide sequence and the sequence corresponding to the third nucleotide sequence in the same reaction system thereas. However, the number of the nucleotides is 4 at the maximum, preferably 3 or less, more preferably 2 or less. Moreover, the sequence corresponding to the second nucleotide sequence in each reaction system may contain contiguous nucleotides of a sequence complementary to any of the third nucleotide sequence and the sequence corresponding to the third nucleotide sequence in the same reaction system thereas. However, the number of the nucleotides is 4 at the maximum, preferably 3 or less, more preferably 2 or less. Furthermore, the nucleotide sequences of nucleic acids in other reaction systems may contain contiguous nucleotides of a sequence complementary to any of the third nucleotide sequence and the sequence corresponding to the third nucleotide sequence in each reaction system. However, the number of the nucleotides is 4 at the maximum, preferably 3 or less, more preferably 2 or less.

The second nucleotide sequence in each reaction system can be selected in such a way that the Tm value between the second nucleotide sequence and the sequence corresponding to the second nucleotide sequence is 5°C or more, preferably 10°C or more, more preferably 15°C or more, further preferably 20°C or more, particularly preferably 25°C or more, higher than the reaction temperature. On the other hand, the second nucleotide sequence in each reaction system can be selected in such a way that the Tm value between the second nucleotide sequence and each of the third nucleotide sequence and the sequence corresponding to the third nucleotide sequence in the same reaction system thereas and the nucleotide sequences of nucleic acids in other reaction systems is 5°C or more, preferably 10°C or more, more preferably 15°C or more, further preferably 20°C or more, particularly preferably 25°C or more, lower than the reaction temperature. Moreover, the sequence corresponding to the second nucleotide sequence in each reaction system can be selected in such a way that the Tm value between the sequence corresponding to the second nucleotide sequence and each of the third nucleotide sequence and the sequence corresponding to the third nucleotide sequence in the same reaction system thereas and the nucleotide sequences of nucleic acids in other reaction systems is 5°C or more, preferably 10°C or more, more preferably 15°C or more, further preferably 20°C or more, particularly preferably 25°C or more, lower than the reaction temperature.

The third nucleotide sequence in each reaction system can be selected in such a way that the Tm value between the third nucleotide sequence and the sequence corresponding to the third nucleotide sequence is 5°C or more, preferably 10°C or more, more preferably 15°C or more, further preferably 20°C or more, particularly preferably 25°C or more, higher than the reaction temperature. On the other hand, the third nucleotide sequence in each reaction system can be selected in such a way that the Tm value between the third nucleotide sequence and each of the second nucleotide sequence and the sequence corresponding to the second nucleotide sequence in the same reaction system thereas and the nucleotide sequences of nucleic acids in other reaction systems is 5°C or more, preferably 10°C or more, more preferably 15°C or more, further preferably 20°C or more, particularly preferably 25°C or more, lower than the reaction temperature. Moreover, the sequence corresponding to the third nucleotide sequence in each reaction system can be selected in such a way that the Tm value between the sequence corresponding to the third nucleotide sequence and each of the second nucleotide sequence and the sequence corresponding to the second nucleotide sequence in the same reaction system thereas and the nucleotide sequences of nucleic acids in other reaction systems is 5°C or more, preferably 10°C or more, more preferably 15°C or more, further preferably 20°C or more, particularly preferably 25°C or more, lower than the reaction temperature.

The first nucleotide sequence in each reaction system can be selected in such a way that it has 70% or higher, preferably 80% or higher, more preferably 90% or higher, further preferably 95% or higher, particularly preferably 98% or higher complementarity to the fourth nucleotide sequence in the same reaction system thereas.

The sequence corresponding to the first nucleotide sequence in each reaction system can be selected in such a way that it has 70% or higher, more preferably 80% or higher, particularly preferably 90% or higher, further preferably 95% or higher, particularly preferably 98% or higher complementarity to the sequence corresponding to the fourth nucleotide sequence in the same reaction system thereas.

The sequence having 70% or higher complementarity to each nucleotide sequence in each reaction system is preferably a sequence complementary to the nucleotide sequence.

The first nucleotide sequence in each reaction system can be selected in such a way that it comprises at least 5, preferably at least 10, more preferably at least 15, further preferably at least 20, particularly preferably at least 25, most preferably at least 30 contiguous nucleotides of a sequence complementary to the fourth nucleotide sequence in the same reaction system thereas. Moreover, the fourth nucleotide sequence in each reaction system can be selected in such a way that it comprises at least 5, preferably at least 10, more preferably at least 15, further preferably at least 20, particularly preferably at least 25, most preferably at least 30 contiguous nucleotides of a sequence complementary to the first nucleotide sequence in the same reaction system thereas.

The sequence corresponding to the first nucleotide sequence in each reaction system can be selected in such a way that it comprises at least 5, preferably at least 10, more preferably at least 15, further preferably at least 20, particularly preferably at least 25, most preferably at least 30 contiguous nucleotides of a sequence complementary to the sequence corresponding to the fourth nucleotide sequence in the same reaction system thereas. Moreover, the sequence corresponding to the fourth nucleotide sequence in each reaction system can be selected in such a way that it comprises at least 5, preferably at least 10, more preferably at least 15, further preferably at least 20, particularly preferably at least 25, most preferably at least 30 contiguous nucleotides of a sequence complementary to the sequence corresponding to the first nucleotide sequence in the same reaction system thereas.

The first nucleotide sequence in each reaction system can be selected in such a way that the Tm value between the first nucleotide sequence and the fourth nucleotide sequence in the same reaction system thereas is 5°C or more, preferably 10°C or more, more preferably 15°C or more, further preferably 20°C or more, particularly preferably 25°C or more, higher than the reaction temperature.

The sequence corresponding to the first nucleotide sequence in each reaction system can be selected in such a way that the Tm value between the sequence corresponding to the first nucleotide sequence and the sequence corresponding to the fourth nucleotide sequence in the same reaction system thereas is 5°C or more, preferably 10°C or more, more preferably 15°C or more, further preferably 20°C or more, particularly preferably 25°C or more, higher than the reaction temperature.

Multiplexed detection using the integrated nucleic acid complex is less susceptible to other reaction systems. Therefore, the nucleotide sequences (e.g., the fifth nucleotide sequence, the sixth nucleotide sequence, the nucleotide sequences of terminal moieties 1 to 4, and the sequences corresponding thereto) used can be selected appropriately by those skilled in the art.

Labels used in the multiplexed detection method are not particularly limited as long as the structural change of interest can be differentiated sufficiently from that of other nucleic acid duplex complexes and sufficient fluorescence resonance energy transfer is obtained. The labels are preferably a combination of fluorescein (fluorescent material) with DABCYL (quenching material) or a combination of Cy3 (fluorescent material) and BHQ1 (quenching material).

Sites to which the nucleic acid binding protein binds, used in a plurality of reactions, may be binding sites for different proteins or may be different binding sites for the same proteins.

For example, in the screening for inhibitors for the different proteins, highly specific drugs are easily obtained by assaying simultaneously therewith proteins that are related to the target proteins but undesired to suffer inhibitory effect.

Moreover, the binding properties of the same proteins to nucleic acids can be determined in more detail by simultaneously assaying the bindings of the proteins to different binding sites.

### Screening method

### First aspect

Upon binding of a nucleic acid binding protein to the nucleic acid duplex A and/or the nucleic acid duplex B, the formation of a nucleic acid duplex complex and the subsequent formation of a new nucleic acid duplex C and/or a new nucleic acid duplex D are inhibited. Thus, whether or not a test substance inhibits or promotes the binding of a nucleic acid binding protein to a nucleic acid can be determined by performing the detection method of the present invention in the presence of and in the absence of the test substance and comparing the degree of structural change therebetween.

When the amount of the nucleic acid duplex C and/or the nucleic acid duplex D formed in the presence of the test substance is higher than that in the absence of the test substance, the test substance can be determined to be a binding inhibitor for the nucleic acid binding protein. Moreover, when the amount of the nucleic acid duplex A and/or the nucleic acid duplex B in the presence of the test substance is lower than that in the absence of the test substance, the test substance can be determined to be a binding inhibitor for the nucleic acid binding protein.

On the other hand, when the amount of the nucleic acid duplex C and/or the nucleic acid duplex D formed in the presence of the test substance is lower than that in the absence of the test substance, the test substance can be determined to be a binding promoter for the nucleic acid binding protein. Moreover, when the amount of the nucleic acid duplex A and/or the nucleic acid duplex B in the presence of the test substance is higher than that in the absence of the test substance, the test substance can be determined to be a binding promoter for the nucleic acid binding protein.

### Second aspect

Upon binding of a nucleic acid binding protein to any of the integrated nucleic acid complex E and the integrated nucleic acid complex F, structural change between the integrated nucleic acid complex E and the integrated nucleic acid complex F is inhibited to stabilize the state of the integrated nucleic acid complex bound with the nucleic acid binding protein. Thus, whether or not a test substance inhibits or promotes the binding of a nucleic acid binding protein to a nucleic acid can be determined by performing the detection method of the present invention in the presence of and in the absence of the test substance and comparing therebetween an amount of an integrated nucleic acid complex designed to bind to the nucleic acid binding protein.

When the amount of the integrated nucleic acid complex E or the integrated nucleic acid complex F in the presence of the test substance is lower than that in the absence of the test substance, the test substance can be determined to be a binding inhibitor for the nucleic acid binding protein. Moreover, when the amount of the integrated nucleic acid complex E or the integrated nucleic acid complex F in the presence of the test substance is higher than that in the absence of the test substance, the test substance can be determined to be a binding promoter for the nucleic acid binding protein.

In the screening method according to the present invention, the "test substance" means every candidate substance as an inhibitor or promoter and is preferably a low-molecular-weight compound.

It has been demonstrated that the screening method according to the present invention can adopt a nucleic acid set that does not bind to the target nucleic acid binding protein, as a negative control for confirming that the test substance acts on the binding of the target nucleic acid binding protein and does not have influence on strand exchange by the nonspecific action on the nucleic acid (Example 5).

Specifically, the screening method according to the present invention can further comprise determining the degree of structural change in a nucleic acid complex consisting of nucleic acids that do not bind to a nucleic acid binding protein targeted by the test substance.

A method of detecting the negative control may be the detection method of the first aspect or the second aspect. When the screening of interest uses the detection method of the first aspect, the detection method of the second aspect may be used for detecting the negative control. Alternatively, when the screening of interest uses the detection method of the second aspect, the detection method of the first aspect may be used for detecting the negative control. However, the same detection method as in the screening of interest is preferably used.

The screening method according to the present invention is advantageous in that the inhibitory effect of a test substance can be assayed conveniently and rapidly without the use of gel shift assay.

### Kit

According to the present invention, there is provided a detection kit for performing the detection method according to the present invention or the screening method according to the present invention.

### Fi rst aspect

An example of the detection kit according to the present invention includes a detection kit for performing the detection method according to the present invention, specifically, a kit for detecting binding between a nucleic acid and a nucleic acid binding protein, comprising at least a nucleic acid duplex A and a nucleic acid duplex B capable of binding to each other at their terminal sequences. These nucleic acid duplexes may be labeled. This detection kit may further contain the nucleic acid binding protein of interest. Moreover, this detection kit detects binding between a nucleic acid and a nucleic acid binding protein by determining the degree of inhibition of structural change in the nucleic acid duplex complex. Thus, if desired, the detection kit can further contain various reagents for determining the degree of inhibition of structural change in the nucleic acid duplex complex, for example, reaction buffers, positive standards, instructions, and/or equipment.

Another example of the detection kit according to the present invention includes a detection kit for performing the screening method according to the present invention, specifically, a kit for screening for an inhibitor or promoter for a nucleic acid binding protein, comprising at least a nucleic acid duplex A and a nucleic acid duplex B capable of binding to each other at their terminal sequences. These nucleic acid duplexes may be labeled. This detection kit may further contain the nucleic acid binding protein of interest. Moreover, this detection kit screens for an inhibitor or promoter for a nucleic acid binding protein by determining the degree of inhibition of structural change in the nucleic acid duplex complex. Thus, if desired, the detection kit can further contain various reagents for determining the degree of inhibition of structural change in the nucleic acid duplex complex, for example, reaction buffers, positive standards, instructions, and/or equipment. Moreover, the detection kit can further contain a nucleic acid duplex or an integrated nucleic acid complex used as a negative control.

### Second aspect

An example of the detection kit according to the present invention includes a detection kit for performing the detection method according to the present invention, specifically, a kit for detecting binding between a nucleic acid and a nucleic acid binding protein, comprising at least an integrated nucleic acid complex E or an integrated nucleic acid complex F. This integrated nucleic acid complex may be labeled. This detection kit may further contain the nucleic acid binding protein of interest. Moreover, this detection kit detects binding between a nucleic acid and a nucleic acid binding protein by determining the degree of inhibition of structural change in the integrated nucleic acid complex. Thus, if desired, the detection kit can further contain various reagents for determining the degree of inhibition of structural change in the integrated nucleic acid complex, for example, reaction buffers, positive standards, instructions, and/or equipment.

Another example of the detection kit according to the present invention includes a detection kit for performing the screening method according to the present invention, specifically, a kit for screening for an inhibitor or promoter for a nucleic acid binding protein, comprising at least an integrated nucleic acid complex E or an integrated nucleic acid complex F. This integrated nucleic acid complex may be labeled. This detection kit may further contain the nucleic acid binding protein of interest. Moreover, this detection kit screens for an inhibitor or promoter for a nucleic acid binding protein by determining the degree of inhibition of structural change in the integrated nucleic acid complex. Thus, if desired, the detection kit can further contain various reagents for determining the degree of inhibition of structural change in the integrated nucleic acid complex, for example, reaction buffers, positive standards, instructions, and/or equipment. Moreover, the detection kit can further contain a nucleic acid duplex or an integrated nucleic acid complex used as a negative control.

The kit of the second aspect is advantageous in that in addition to improved operability, cost reduction brought about by a decreased number of reagent components can be expected.

### Examples

Hereinafter, the present invention will be described more specifically with reference to Examples. However, the present invention is not limited to them.

### Example 1: Study on amount of NFκB p50 bound

A transcription factor NFκB p50 belongs to the Rel family having a loop structure and has a DNA binding sequence. This transcription factor is known to bind to an NFκB binding sequence (e.g., 5'-GGGACTTTCC-3').

### (1) Preparation of duplex

A synthetic oligonucleotide NFkB-01-5F labeled at the 5' terminus with 6-FAM and a synthetic oligonucleotide NFkB-14, or a synthetic oligonucleotide NFkB-02-3D labeled at the 3' terminus with DABCYL and a synthetic oligonucleotide NFkB-13 were mixed (20 µL each) into a duplex forming solution (10 mM HEPES-NaOH (pH 7.9), 50 mM KCI, 30 mM NaCl, 0.1 mM EDTA, 2.5 mM DTT, 10% Glycerol, 0.05% IGEPAL CA-630) and subjected to heat denaturation and annealing to prepare duplexes 01F/14 and 02D/13 having a single-stranded terminus. All the synthetic oligonucleotides were used at a concentration of 20 pmol. In the description below, all labels used were synthesized and prepared by JBioS Japan Bio Service Co., Ltd under the request.

The heat denaturation and annealing were performed under the following temperature conditions:
95°C 120 seconds-90°C 30 seconds-85°C 90 seconds-80°C 90
seconds-77°C 90 seconds-75°C 90 seconds-72°C 90
seconds-70°C 90 seconds-67°C 90 seconds-65°C 90
seconds-62°C 90 seconds-60°C 90 seconds-57°C 90
seconds-55°C 90 seconds-52°C 90 seconds-50°C 90
seconds-47°C 90 seconds-45°C 90 seconds-42°C 90
seconds-40°C 90 seconds-37°C 90 seconds-35°C 90
seconds-32°C 90 seconds-30°C 90 seconds.

Each sequence used is as follows:

**[Table 1]**

| |
|---|
| NFkB-01-5F: |
| 5' AGTTGAGGGGACTTTCOCAGGCGACTCACTATAGG*CGGTGTCTCGCTCGC* 3' (SEQ ID NO: 1) |
| NFkB-02-3D: |
| 5' *GCGAGOGAGACACCOCC*TATAGTGAGTCGCCTGGGAAAGTCCCCTCAACT 3' (SEQ ID NO: 2) |
| NFkB-13: |
| 5' AGTTGAGGGGACTTTCCCAGCCGACTCACTATAGG*CACCACACCATTOCC* 3' (SEQ ID NO: 3) |
| NFkB-14: |
| 5' *GGGAATGGTGTGGTOC*CTATAGTGAGTCGCCTGGGAAAGTCCCCTCAACT 3' (SEQ ID NO: 4) |

The underlines represent an NFκB binding sequence. The italic types represent a single-stranded sequence in the duplex.

### (2) Detection of NFκB p50 binding

0.28 pmol of the duplex 01F/14 and each concentration of recombinant human NFκB p50 (Promega Corp.) were mixed into 20 µL of a reaction solution (12 mM HEPES-NaOH pH 7.9, 50 mM KCI, 0.1 mM EDTA, 30.5 mM DTT, 11% glycerol, 0.06% IGEPAL CA-630, 5 mM NaCl, 0.05 mM PMSF, 1 µM ZnSO₄) and reacted at 25°C for 30 minutes. Then, the reaction mixture was left standing on ice for 5 minutes. 2.8 µL (0.28 pmol) of the duplex 02D/13 diluted with an NFκB binding solution (10 mM HEPES-NaOH pH 7.9, 50 mM KCl, 0.1 mM EDTA, 25 mM DTT, 10% glycerol, 0.05% IGEPAL CA-630) was added thereto and then reacted at 30°C for 11 minutes. The fluorescence value was measured using a real-time PCR apparatus Rotor-Gene 2000 (Corbett Life Science).

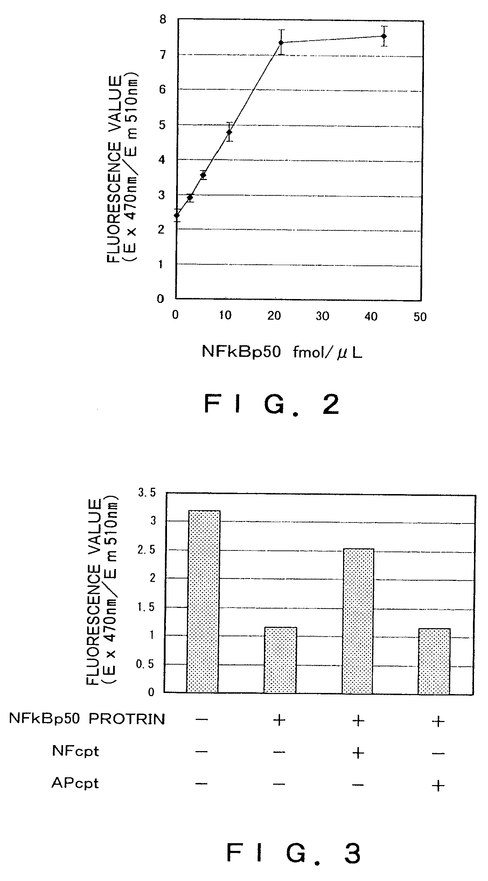

As a result, the fluorescence value was increased depending on the NFκB p50 concentration (Figure 2).

01F/14 emits fluorescence owing to 6-FAM introduced in NFkB-01-5F. However, 01F/14 and 02D/13 are mixed to form, through structural change, new perfect duplexes 01F/02D (NFkB-01-5F and NFkB-02-3D) and 13/14 (NFkB-13 and NFkB-14) in which the fluorescence of 6-FAM is quenched by DABCYL in NFkB-02-3D. On the other hand, upon binding of NFκB p50, the structural change is inhibited. This is probably the reason why the fluorescence value is increased depending on the NFκB p50 concentration.

These results demonstrated that according to the method described above, structural change caused by NFκB p50 binding can be detected by measuring a fluorescence value.

### Example 2: Influence of decoy oligonucleotide on amount of NFκB p50 bound

### (1) Preparation of duplex

A synthetic oligonucleotide NFkB-01-5F labeled at the 5' terminus with 6-FAM and a synthetic oligonucleotide NFkB-14-03D labeled at the 3' terminus with DABCYL, or synthetic oligonucleotides NFkB-02 and NFkB-13 were mixed to prepare duplexes 01F/14D and 02/13 having a single-stranded terminus under the same conditions as in Example 1.

Moreover, synthetic oligonucleotides NFcpt01 and NFcpt02 having an NFκB binding sequence, or synthetic oligonucleotides APcpt01 and APcpt02 free from an NFκB binding sequence were mixed to prepare decoy oligonucleotide duplexes NFcpt and APcpt under the same conditions as in Example 1. The duplex NFcpt contains an NFκB binding sequence and therefore inhibits the binding of NFκB proteins to DNAs having other NFκB binding sequences. However, the duplex APcpt is free from an NFκB binding sequence and therefore, does not inhibit the binding of NFκB proteins to other NFκB binding sequences.

Each sequence used is as follows:

**[Table 2]**

| |
|---|
| NFkB-01-5F: |
| 5' AGTTGAGGGGACTTTCOCAGGCGACTCACTATAGG*OGGTGTCTOGCTOGC* 3' (SEQ ID NO: 5) |
| NFkB-02-3D: |
| 5' *GCGAGCGAGACAOOO*OCTATAGTGAGTCGCCTGGGAAAGTOCOOCTCAACT 3' (SEQ ID NO: 6) |
| NFkB-13: |
| 5' AGTTGAGGGGACTTTCOCAGGOGACTCACTATAGG*CACCACACCATTOCC* 3' (SEQ ID NO: 7) |
| NFkB-14: |
| 5' *GGGAATGGTGTGGTO*CCTATAGTGAGTCGCCTGGAAAGTOOOCTCAACT (SEQ ID NO: 8) |
| NFcpt01: |
| GCACTAGGACTTTOCGOCAOG (SEQ ID NO: 9) |
| NFcpT02: |
| CGTGGCGGAAAGTCCCTAGTGC (SEQ ID NO: 10) |
| APcpt01: |
| GGTGTCATGAGTCAGCTCGGAG (SEQ ID NO: 11) |
| APcpt02: |
| CTOOGAGCTGACTCATGACACC (SEQ ID NO: 12) |

The underlines represent an NFκB binding sequence. The italic types represent a single-stranded sequence in the duplexes 01F/14D and 02/13.

### (2) Detection of NFκB p50 binding

1.68 pmol each of the decoy oligonucleotide duplexes, 0.42 pmol of the duplex 01F/14D, 1.58 pmol of recombinant human NFκB p50 (Promega Corp.), 0.42 pmol of the duplex 02/13 were mixed into 34.2 µL of an NFκB reaction solution with the same composition as in Example 1 and then reacted at 30°C for 21 minutes. The fluorescence value was measured using a real-time PCR apparatus Rotor-Gene 2000 (Corbett Life Science).

As a result, the addition of the decoy oligonucleotide duplex (NFcpt) as an NFκB binding inhibitor increased the fluorescence value decreased by the addition of NFκB p50, whereas the addition of the decoy oligonucleotide duplex (APcpt) free from an NFκB binding sequence was not confirmed to cause such increase in fluorescence value (Figure 3).

In 01F/14D, the fluorescence of 6-FAM introduced in NFkB-01-5F is quenched by DABCYL introduced in NFkB-14-3D. However, 01F/14D and 02/13 are mixed to form, through structural change, new perfect duplexes 01F/02 (NFkB-01-5F and NFkB-02) and 13/14D (NFkB-13 and NFkB-14-3D) in which 6-FAM is sufficiently distant from DABCYL and therefore emits fluorescence (Figure 3: the absence of NFκB p50). Upon addition of NFκB p50, the structural change is inhibited. Therefore, the fluorescence value is decreased (Figure 3: the addition of NFκB p50). However, the further addition of NFcpt inhibits the binding of NFκB p50 to the synthetic oligonucleotide and also inhibits structural change. Therefore, the fluorescence value is increased (Figure 3: the addition of NFκB p50 + NFcpt). On the other hand, the addition of APcpt inhibits neither NFκB p50 binding nor structural change. Therefore, the fluorescence value is decreased (Figure 3: the addition of NFκB p50 + APcpt).

These results demonstrated that NFκB p50 binding inhibition specific to a decoy oligonucleotide duplex (NFcpt) having an NFκB binding sequence can be detected by measuring a fluorescence value. Thus, it was shown that the method described above can be used in the screening for an inhibitor for a nucleic acid binding protein.

### Example 3-1: Binding test on human AP1 proteins

A transcription factor AP1 (c-jun) has a DNA binding motif having a leucine zipper structure and is known to bind to an AP1 binding sequence (e.g., 5'-TGAGTCA-3').

### (1) Preparation of duplex

Synthetic oligonucleotides AP-01-5C5 and AP-04-3BH, synthetic oligonucleotides AP-02 and AP-03, synthetic oligonucleotides SP1-01-5A5 and SP1-04-3BH3, or synthetic oligonucleotides SP1-02 and SP1-03 were allowed to hybridize to each other to prepare duplexes AP01C/04B, AP02/03, SP01A/04B, and SP02/03 having a single-stranded terminus under the same conditions as in Example 1.

The duplexes AP01C/04B and AP02/03 have an AP1 binding sequence, while the duplexes SP01A/04B and SP02/03 are free from an AP1 binding sequence.

Each sequence used is as follows:

**[Table 3]**

| |
|---|
| AP-01-5C5: |
| 5' cy5 - CGCTTGATGAGTCAGCOGGAA*GTGGTTGGGTAAGGG* 3' (SEQ ID NO: 13) |
| AP-02 : |
| *CCCTTAOCCAACCAC*TTOCGGCTGACTCATCAAGCG 3' (SEQ ID NO: 14) |
| AP-03 : |
| OGCTTGATGAGTCAGCCGGAA*CGGACAGGACGATAT* 3' (SEQ ID NO: 15) |
| AP-04-3BH : |
| *ATATOGTCCTGTCCGT*TCCGGCTGACTCATCAAGCG 3' BHQ 3' (SEQ ID NO: 16) |

The underlines represent an AP1 binding sequence. The italic types represent a single-stranded sequence in the duplex.

**[Table 4]**

| |
|---|
| SP1-01-5A647 : |
| 5' Alexa647 - GGGATTCGATCGGGGCGGGGCGACGAGC*GTGGTTGGGTAAGGG* 3' (SEQ ID NO: 17) |
| SP1-02: |
| 5' *CCCTTACCCAACCAC*GCTCGTCGCOOOGOCCCOGATCGAATCCC 3' (SEQ ID NO: 18) |
| SP1-03: |
| 5' GGGATTCGATCGGGGCGGGGCGACGAGC*CACCACACCATTOCC* 3' (SEQ ID NO: 19) |
| SPI-04-3BH3 : |
| 5' *GGGAATGGTGTGGTG*GCTCGTCGCCCCGCCCCGATCGAATOCC - BHQ 3' (SEQ ID NO: 20) |

The italic types represent a single-stranded sequence in the duplex. The underlines represent an SP1 binding sequence.

### (2) Detection of AP1 (c-jun binding

60 fmol of the duplex AP01C/04B or SP01A/04B and 1.5 pmol of recombinant human AP1 (c-jun) (Promega Corp.) were mixed into 30 µL of a reaction solution (10 mM HEPES-NaOH pH 7.9, 53 mM KCl, 0.17 mM EDTA, 2.6 mM DTT, 12% glycerol, 0.05% IGEPAL CA-630, 1.3 mM Tris-HCl pH 7.5, 0.67 mM MgCl₂, 33 mM guanidine hydrochloride) and reacted at 25°C for 30 minutes. Then, the reaction mixture was left standing on ice for 5 minutes. 3 µL (60 fmol) of the duplex AP02/03 or SP02/03 diluted with an NFκB binding solution was added thereto and then reacted at 30°C for 2 hours. The fluorescence value was measured using a real-time PCR apparatus Rotor-Gene 2000 (Corbett Life Science).

In Example 3, the inhibitory effect of protein binding on structural change was detected based on decrease in fluorescence value in the same way as in Example 2 except that Cy5 or Alexa 647 was used as a fluorescent material instead of 6-FAM of Example 2 and BHQ3 was used as a quenching material instead of DABCYL.

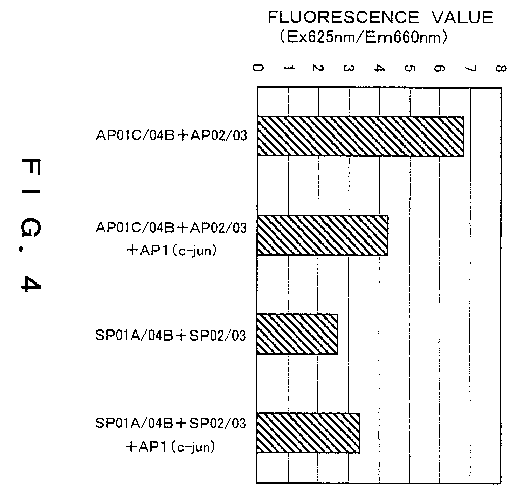

As a result, when the duplexes (AP01C/04B and AP02/03) having an AP1 binding sequence were used, decrease in fluorescence value caused by the addition of AP1 (c-jun), i.e., the inhibition of structural change by AP1 (c-jun), was observed (Figure 4). However, when the duplexes (SP01A/04B and SP02/03) having an SP1 binding sequence were used, decrease in fluorescence value, i.e., the inhibition of structural change by AP1 (c-jun), was not observed even after the addition of AP1 (c-jun) (Figure 4).

### Example 3-2: Test to detect binding of human SP1 proteins

A transcription factor SP1 has a DNA binding motif having a Zn finger structure and is known to bind to an SP1 binding sequence (e.g., 5'-GGGGCGGGGC-3').

### (1) Preparation of duplex

Duplexes were prepared in the same way as in Example 3.

### (2) Detection of SP1 binding

60 fmol of the duplex AP01C/04B or SP01A/04B and 1.65 pmol of recombinant human SP1 (Promega Corp.) were mixed into 30 µL of a reaction solution (10 mM HEPES-NaOH pH 7.9, 53.0 mM KCl, 0.1 mM EDTA, 2.6 mM DTT, 13.0% glycerol, 0.056% IGEPAL CA-630, 0.36 mM MgCl₂, 303 nM ZnSO₄, 0.72 mM HEPES-KOH pH 7.5) and reacted at 25°C for 30 minutes. Then, the reaction mixture was left standing on ice for 5 minutes. 3 µL (60 fmol) of the duplex AP02/03 or SP02/03 diluted with an NFκB binding solution was added thereto and then reacted at 30°C for 4 hours. The fluorescence value was measured using a real-time PCR apparatus Rotor-Gene 2000 (Corbett Life Science).

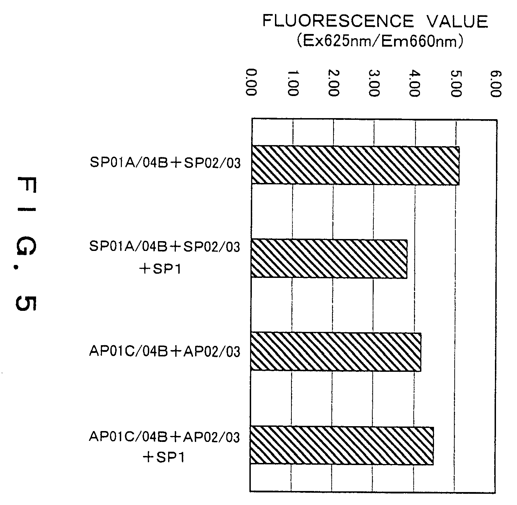

As a result, when the duplexes (SP01A/04B and SP02/03) having an SP1 binding sequence were used, decrease in fluorescence value caused by the addition of SP1, i.e., the inhibition of structural change by SP1, was observed (Figure 5). However, when the duplexes (AP01C/04B and AP02/03) having an AP1 binding sequence were used, decrease in fluorescence value, i.e., the inhibition of structural change by SP1, was not observed even after the addition of SP1 (Figure 5).

### Example 3-3: Binding experiment on TRF2 proteins

A telomere DNA binding protein TRF2 has a Myb-type DNA binding domain consisting of a C-terminal helix-turn-helix motif and is known to form a telomere structure through the binding to telomere DNA (5'-TTAGGG-3' repeats).

### (1) Reparation of recombinant human TRF2ΔB

TRF2ΔB is a recombinant human TRF2 protein deficient in the N-terminal basic domain of TRF2 (N-terminal deletion variant).

cDNA encoding full-length TRF2 was obtained from cDNA prepared based on mRNA extracted from a human cultured cell line (IHW09084 (obtained from International HLA Workshop)). This cDNA was used as a template in PCR to amplify a DNA region corresponding to residues 45 to 500 of the amino acid sequence. After addition of a histidine tag sequence, the obtained DNA fragment was introduced into pFastBac1 (Invitrogen Corp.) to obtain a bacmid. Insect cells (Sf9 (obtained from Invitrogen Corp.)) were transfected with the bacmid to prepare a virus. Sf9 was infected with the obtained virus for expression of recombinant human TRF2ΔB. TRF2ΔB was purified on an Ni-NTA spin column (QIAGEN). The protein concentration was calculated with bovine serum albumin as reference using a protein assay kit ((Bio-Rad Laboratories, Inc.).

### (2) Preparation of duplex

A synthetic oligonucleotide TLM-06 and a synthetic oligonucleotide TLM-01-5C3 labeled at the 5' terminus with Cy3 were mixed to prepare a duplex T01C/06 having a single-stranded terminus under the same conditions as in Example 1. The duplex T01C/06 has a TRF2 binding sequence.

A synthetic oligonucleotide TLM-16 and a synthetic oligonucleotide TLM-13-5C3 labeled at the 5' terminus with Cy3 were mixed to prepare a duplex T13C/16 having a single-stranded terminus under the same conditions as in Example 1. The duplex T13C/16 is free from a TRF2 binding sequence.

A synthetic oligonucleotide TLM-05 and a synthetic oligonucleotide TLM-02-3B1 labeled at the 3' terminus with BHQ1 were mixed to prepare a duplex T02B/05 having a single-stranded terminus under the same conditions as in Example 1. The duplex T02B/05 has a TRF2 binding sequence.

A synthetic oligonucleotide TLM-15 and a synthetic oligonucleotide TLM-14-3B1 labeled at the 3' terminus with BHQ1 were mixed to prepare a duplex T14B/15 having a single-stranded terminus under the same conditions as in Example 1. The duplex T14B/15 is free from a TRF2 binding sequence.

Each sequence used is as follows:

**[Table 5]**

| |
|---|
| TLH-01-5C3 |
| 5' Cy3-AGTTGAGTTAGGGTTAGGGTTAGGGTTAGGGCAGG *CGGTGTCTCGCTCGC* 3' (SEQ ID NO: 21) |
| TLB-02-3B1 : |
| 5' *GGGAGCGAGACACCG* CCTGCCCTAACCCTAACCCTAACCCTAACTCAACT-BHQ1 3' (SEQ ID NO: 22) |
| TLM-05 : |
| 5' AGTTGAGTTAGGGTTAGGGTTAGGTTAGGGCAGG *CACCACACCATTCCC* 3' (SEQ ID NO: 23) |
| TLH-06 : |
| 5' *GGGAATGGTGTGGTG* CCCTGCCCTAAGCCTAACCCTAACCCTAACTCAACT 3' (SEQ ID NO: 24) |

The underlines represent a TRF2 binding sequence. The italic types represent a single-stranded sequence in the duplex.

**[Table 6]**

| |
|---|
| TLH-13-503 : |
| 5' Cy3-AGTTGAGTTAGGGTTAGCGTTAGCGTTAGCGCAGG *CGGTGTCTCGCTCGC* 3' (SEQ ID NO: 25) |
| TLH-14-3B1 : |
| 5' *GCGAGCGAGACACCG* CCTGCGCTAACGCTAACGCTAACGC TAACTCAACT-BHQ1 3' (SEQ ID NO: 26) |
| TLM-15 : |
| 5' AGTTGAGTTAGGGTTAGCGTTAGCGTTAGGGCAGG *CACCACACCATTCCC* 3' (SEQ ID NO: 27) |
| TLM-16 : |
| 5' *GGGAATGGTGTGGTG* CGTGCGCTAACGCTAACGCTAACGCTAACTCAACT 3' (SEQ ID NO: 28) |

The underlines represent a TRF2 binding sequence, which however cannot bind to TRF2 due to variations indicated in the shaded areas. The italic types represent a single-stranded sequence in the duplex.

### (3) Detection of TRF2 binding

100 fmol of T01C/06 or T13C/16 and 1 µg of TRF2ΔB were mixed into a reaction solution (10 mM HEPES-NaOH pH 7.9, 150 mM KCI, 0.1 mM EDTA, 5 mM DTT, 10% glycerol, 0.05% IGEPAL CA-630, 20 µL) and reacted at 25°C for 30 minutes. Then, 100 fmol each of T02B/05 and T14B/15 was added to T01C/06 and T13C/16, respectively, and 50 µL each of the resulting mixtures was then reacted at 25°C for 60 minutes. The fluorescence value of Cy3 was measured using a fluorescence plate reader Ultra (Tecan Group Ltd.).

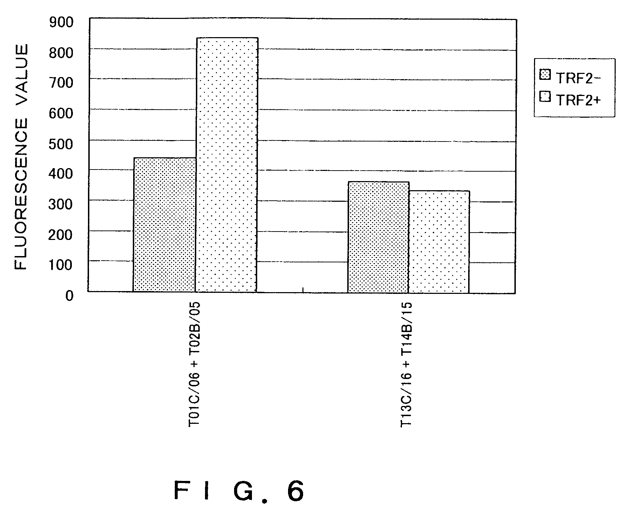

As a result, in the DNA set (T01C/06 and T02B/05) having a TRF2 binding sequence, T01C/06 and T02B/05 formed a tetramer, which was in turn converted to T01C/02B by complete strand exchange through branch migration. The addition of TRF2ΔB suppressed decrease in fluorescence value caused by this conversion (Figure 6). On the other hand, in the DNA set (T13C/16 and T14B/15) having a TRF2 binding sequence variant, T13C/16 and T14B/15 formed a tetramer, which was in turn converted to T13C/14B by complete strand exchange through branch migration. The addition of TRF2ΔB did not suppress decrease in fluorescence value caused by this conversion (Figure 6).

Thus, the method of the present invention successfully detected the inhibition of the binding of DNA binding proteins having a typical DNA binding motif loop, leucine zipper, or Zn finger. Moreover, the method of the present invention successfully detected the inhibition of the binding of telomere DNA binding proteins. Thus, it was shown that the present invention is widely applicable to general nucleic acid binding proteins.

Moreover, it was also shown that the method according to the present invention is not limited by fluorescent materials used. According to the present invention, various types of fluorescent materials can be used. Therefore, for example, when a compound serving as a drug emits fluorescence, a fluorescent label that presents no obstacle to the fluorescence of the compound can be selected.

### Example 4: Test to confirm detection sensitivity depending on mismatch introduction

### 4-1. Test using SP1

### (1) Preparation of duplex

A synthetic oligonucleotide SP1-05-5C3 labeled at the 5' terminus with Cy3 and a synthetic oligonucleotide SP1-08-3B1 labeled at the 3' terminus with BHQ1 were mixed to prepare a duplex SP05C/08B having a single-stranded terminus under the same conditions as in Example 1. Moreover, synthetic oligonucleotides SP1-06 and SP1-07, synthetic oligonucleotides SP1-21 and SP1-22, or synthetic oligonucleotides SP1-23 and SP1-24 were mixed to prepare duplexes SP06/07, SP22/21, and SP24/23 having a single-stranded terminus under the same conditions as in Example 1. These duplexes have an SP1 binding sequence.

Each sequence used is as follows:

**[Table 7]**

| SP1 oligo DNA |
|---|
| SP1-05-5C3 : |
| 5' Cy3- ATTCGATCGGGGCGGGGCGACGAGC *GGGAATGGACAGGTT* 3' (SEQ ID NO: 29) |
| SP1-08-3B1 : |
| 5' *CGGTCAGAGTAGGCT* GCTCGTCGCCCCGCCCCGATCGAAT -BHG1 3' (SEQ ID NO: 30) |
| SP1-07 |
| 5' ATTCGATCGGGGCGGGGCGACGAGC *AGGGTACTCTGACCG* 3' (SEQ ID NO: 31) |
| SP1-21 : |
| 5' ATTGGATCGGGGCGGGGCGCGAGC *AGGCTACTCTCTGACCG* 3' (SEQ ID NO: 32) |
| SP1-23 : |
| 5' ATTCGATCGGGGCGGGGCGACGAGA *AGGCTACTCTCTGACCG* 3' (SEQ ID NO: 33) |
| SP1-06 : |
| 5' *AACCTGTCCATTCCG* GCTCGTCGCCCCGCCCCGATCGAAT 3' (SEQ ID NO: 34) |
| SP1-22 : |
| 5' *AACCTGTCCATTCCC* GCTCGGCGCCCCGCCCCGATCGAAT 3' (SEQ ID NO: 35) |
| SP1-24 : |
| 5' *AACCTGTCCATTCCC* TCTCGTCGCCCGCCCCGATCGAAT 3' (SEQ ID NO: 36) |

The underlines represent an SP1 binding sequence. The italic types represent a single-stranded sequence in the duplex. The shaded areas represent mismatch bases in SP1-21 and SP1-23 with respect to SP1-08-3B1 or mismatch bases in SP1-22 and SP1-24 with respect to SP1-05-5C3.

### (2) Confirmation of effect of suppressing strand exchange

40 fmol of SP05C/08B and 2.5 pmol of recombinant human SP1 (Promega Corp.) were mixed into a reaction solution (10 mM HEPES-NaOH pH 7.9, 50 mM KCI, 0.1 mM EDTA, 5 mM DTT, 10% glycerol, 0.05% IGEPAL CA-630, 15 µL) and reacted at 25°C for 30 minutes. 80 fmol of SP06/07, SP22/21, SP24/23, SP1-07, SP1-21, or SP1-23 was added thereto, and 50 µL of the resulting mixture was then reacted at 25°C for 120 minutes. The fluorescence value of Cy3 was measured using a fluorescence plate reader Ultra (Tecan Group Ltd.).

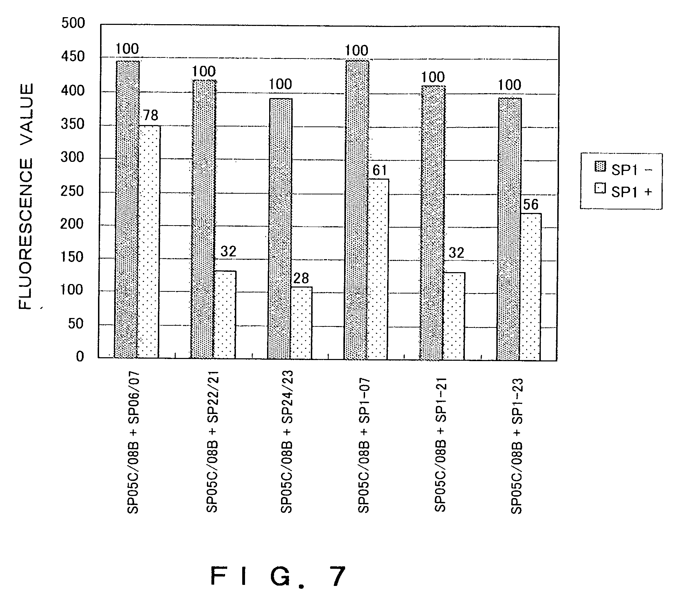

As a result, SP05C/08B and SP06/07 formed a tetramer, which was in turn converted to SP05C/06 by complete strand exchange through branch migration. The addition of SP1 suppressed increase in fluorescence value caused by this conversion (Figure 7). The same results were also obtained using SP22/21 or SP24/23 instead of SP06/07 (Figure 7).

Moreover, SP05C/08B and SP1-07 formed a trimer, which was in turn converted to a dimer 08B/07 and a monomer SP1-05-5C3 by complete strand exchange through branch migration. The addition of SP1 suppressed increase in fluorescence value caused by this conversion (Figure 7). The same results were also obtained using SP1-21 or SP1-23 instead of SP1-07 (Figure 7).

In this context, the suppression of the fluorescence value by the addition of the SP1 proteins was confirmed to be much larger in the addition of SP22/21, SP24/23, SP-21, or SP-23 having a mismatch than in the addition of SP06/07 or SP1-07 having no mismatch with respect to SP05C/08B.

These results demonstrated that mismatch introduction potentiates the effect of suppressing strand exchange by the addition of SP1 proteins and increases the ratio of a fluorescence value between the non-addition and addition of the proteins.

### 4-2. Test Example as to NF-κB

### (1) Preparation of duplex

A synthetic oligonucleotide NFkB-01-5F labeled at the 5' terminus with fluorescein and a synthetic oligonucleotide NFkB-14-3D labeled at the 3' terminus with DABCYL were mixed to prepare a duplex NF01F/14D having a single-stranded terminus under the same conditions as in Example 1. Moreover, synthetic oligonucleotides NFkB-02 and NFkB-13, synthetic oligonucleotides NFkB-21 and NFkB-26, or synthetic oligonucleotides NFkB-49 and NFkB-50 were mixed to prepare duplexes NF02/13, NF26/21, and NF50/49 having a single-stranded terminus under the same conditions as in Example 1. These duplexes have an NF-κB binding sequence.

Each sequence used is as follows:

**[Table 8]**

| NF-κB oligo DNA |
|---|
| NFk6-01-5F : |
| 5' Fluerescein - AGTTGAGGGGACTTTCCCAGGCGACTCACTATAGG *CGGTGTGTCGCTCGC* 3' (SEQ ID NO: 37) |
| HFkB-14-3D : |
| 5' *GGGAATGGTGTGGTG* CCTATAGTGAGTCGCCTGGGAAAGTCCCCTCAACT- Dabcyl 3' (SEQ ID NO: 38) |
| NFkB-13 : |
| 5' AGTTGAGGGGACTTTCCCAGGCGACTCACTATAGG *CACCAGACGATTCCC* 3' (SEQ ID NO: 39) |
| NFkB-21 : |
| 5' AGTTGAGGGGACTTGCCCAGGCGACTCACTATAGG *CACCACACCATTCCC* 3' (SEQ ID NO: 40) |
| NFkB-40 : |
| 5' AGTTGAGGGGACTTTCCCAGGCGACTGACTATAGA *CACCACACCATTCCC* 3' (SEQ ID NO: 41) |
| NFkE-02 : |
| 5' GCGAGCGAGACACCG CCTATAGTGAGTCGCCTGGGAAAGTCCCCTCAACT 3' (SEQ ID NO: 42) |
| NFkB-26 : |
| 5' *GCGAGCGAGACACCG* CCTATAGTGAGTCGCCTGGGCAAGTCCCCTCAAGT 3' (SEQ ID NO: 43) |
| NFkB-50 : |
| 5' *GCGAGCGAGACACCG* TCTATAGTGAGTCGCCTGGGAAAGTCCCCTCAAGT 3' (SEQ ID NO: 44) |

The underlines represent an NF-κB binding sequence. The italic types represent a single-stranded sequence in the duplex. The shaded areas represent mismatch bases in NFkB-21 and NFkB-49 with respect to NFkB-14-3D or mismatch bases in NFkB-26 and NFkB-50 with respect to NFkB-01-5F.

### (2) Confirmation of effect of suppressing strand exchange

100 fmol of NF01F/14D and 500 pmol of recombinant human NF-κB (p50) (Promega Corp.) were mixed into a reaction solution (10 mM HEPES-NaOH pH 7.9, 50 mM KCl, 0.1 mM EDTA, 5 mM DTT, 10% glycerol, 0.05% IGEPAL CA-630, 15 µL) and reacted at 25°C for 30 minutes. 200 fmol of NF02/13, NF26/21, or NF50/49 was added thereto, and 50 µL of the resulting mixture was then reacted at 37°C for 120 minutes. The fluorescence value of fluorescein was measured using a fluorescence plate reader Ultra (Tecan Group Ltd.).

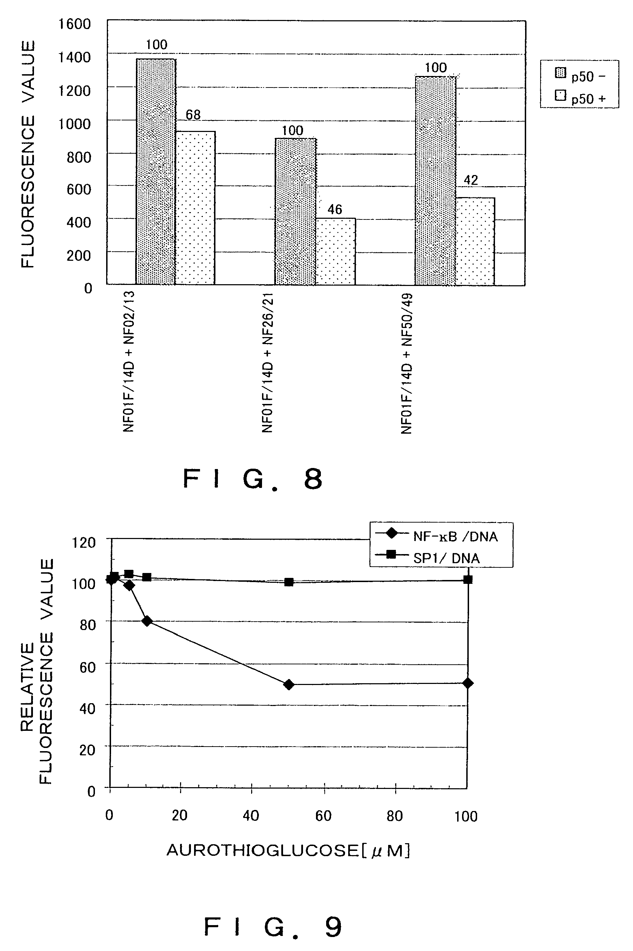

As a result, NF01F/14D and NF02/13 formed a tetramer, which was in turn converted to NF01F/02 by complete strand exchange through branch migration. The addition of p50 suppressed increase in fluorescence value caused by this conversion (Figure 8). The same results were also obtained using NF26/21 or NF50/49 instead of NF02/13 (Figure 8).

In this context, the addition of the NF-κB (p50) proteins decreased the fluorescence value by approximately 30% in the combination of NF01F/14D with NF02/13 having no mismatch, whereas it decreased this fluorescence value by 50% or more in the combination of NF01F/14D with NF26/21 or NF50/49 having a mismatch (Figure 8).

These results demonstrated that mismatch introduction potentiates the effect of suppressing strand exchange by NF-κB (p50) proteins and increases the ratio of a fluorescence value between the non-addition and addition of the proteins.

### Example 5: Study on screening system using negative control

The screening of an inhibitor or the like for a nucleic acid binding protein requires confirming that a test substance acts on the binding of the target nucleic acid binding protein and does not have influence on strand exchange by the nonspecific action on the nucleic acid. Thus, study has been conducted on a screening system using a nucleic acid set that does not bind to the target nucleic acid binding protein, as a negative control.

Aurothioglucose (Yang et al. FEBS letters 1995, 361, 89-96) known to inhibit NF-κB was used as a test substance. Moreover, a nucleic acid set that does not bind to NF-κB was used as a negative control.

### (1) Preparation of duplex

A synthetic oligonucleotide NFkB-42 and a synthetic oligonucleotide NFkB-31-5F labeled at the 5' terminus with fluorescein, or a synthetic oligonucleotide NFkB-41 and a synthetic oligonucleotide NFkB-32-3D labeled at the 3' terminus with DABCYL were mixed to prepare duplexes 31F/42 and 32D/41 having a single-stranded terminus under the same conditions as in Example 1. The duplexes 31F/42 and 32D/41 have an NF-κB binding sequence.

A synthetic oligonucleotide SP1-08 and a synthetic oligonucleotide SP1-05-5C3 labeled at the 5' terminus with Cy3, or a synthetic oligonucleotide SP1-07 and a synthetic oligonucleotide SP1-06-3B1 labeled at the 3' terminus with BHQ1 were mixed to prepare duplexes 05C/08 and 06B/07 having a single-stranded terminus under the same conditions as in Example 1. The duplexes 05C/08 and 06B/07 have an SP1 binding sequence.

Each sequence used is as follows:

**[Table 9]**

| NF-*κ*B oligo DMA |
|---|
| NFkB-31-5F : |
| 5' Fluorescein - AGTTGAGGGGACTTTCCCAGGC *CTCACGCACACCAGC* 3' (SEQ ID NO: 45) |
| NFk6-32-3D : |
| 5' *GCTGGTGTGCGTGAG* GCCTGGGAAAGTCCCCTCAAGT - Dabeyl 3' (SEQ ID NO: 46) |
| NFkB-41 : |
| 5' AGTTGAGGGGACTTTGCCAGGC *AAGACGAGCAAGGAGGG* 3' (SEQ ID NO: 47) |
| NFkB-42 : |
| 5' *CCCTGGTTGCTCGTCTT* GCCTGGGAAAGTCCCCTCAACT 3' (SEQ ID NO: 48) |

The underlines represent an NF-κB binding sequence. The italic types represent a single-stranded sequence in the duplex.

**[Table 10]**

| SP1 oligo DNA |
|---|
| SP1-05-5C3: |
| 5' Cy3 - ATTCGATCGGGGCGGGGCGACGAGC *GGGAATGGACAGGTT* 3' (SEQ ID NO: 49) |
| SP1-06-3B1 : |
| 5' *AACCTGTCCATTCCC* GCTCGTCGCCCCGCCCCGATCGAAT 3' (SEQ ID NO: 50) |
| SP1-07 : |
| 5' ATTCGATCGGGGCGGGGCGACGAGC *ACGCTACTCTCTGACCG* 3' (SEQ ID NO: 51) |
| SP1-08 : |
| 5' *CGGTCAGAGAGTAGCCT* GCTCGTCGCCCCGCCCCGATCGAAT 3' (SEQ ID NO: 52) |

The underlines represent an SP1 binding sequence. The italic types represent a single-stranded sequence in the duplex.

### (2) Detection of NF-κB (p50) inhibitory effect of aurothioglucose

To a mixed solution of 31F/42 and 05C/08 (50 fmol each), 0 to 5 nmol of aurothioglucose and 0.5 pmol of recombinant human NFκB p50 (Promega Corp.) were added, and further a mixed solution of 32D/41 and 06B/07 (200 fmol each) was added. The resulting solution was mixed and reacted at 25°C for 30 minutes. A reaction solution (10 mM HEPES-NaOH pH 7.9, 50 mM KCl, 0.1 mM EDTA, 5 mM DTT, 10% glycerol, 0.05% IGEPAL CA-630) had a final amount of 50 µL. The fluorescence values of fluorescein and Cy3 were measured using a fluorescence plate reader Ultra (Tecan Group Ltd.). The fluorescein was excited at 485 nm, and its fluorescence was measured at 535 nm. The Cy3 was excited at 535 nm and its fluorescence was measured at 595 nm.

As a result, the relative fluorescence value of the NF-κB/oligonucleotide complex was decreased depending on the concentration of aurothioglucose inhibiting NF-κB. On the other hand, the addition of aurothioglucose hardly influenced the relative fluorescence value of the SP1/oligonucleotide (Figure 9). This demonstrated that aurothioglucose has inhibitory effect on the binding of NF-κB (p50) to nucleic acids but does not influence strand exchange itself.

These results demonstrated that according to the screening method of the present invention, the inhibitory effect of a test substance can be assayed conveniently and rapidly without the use of gel shift assay.

### Example 6: Study on amount of NF-κB p50 bound using integrated nucleic acid complex

### (1) Preparation of tetramer

Synthetic oligonucleotides N-Cd-U2m4 (C4) and N-Bc-L2m5 (B5), a synthetic oligonucleotide N-Ab-U2-30F (Af) labeled at thymine 30 (counted from the 5' terminus) with fluorescein, and a synthetic oligonucleotide N-Da-L2-20D (Dd) labeled at thymine 20 (counted from the 5' terminus) with DABCYL were mixed to prepare an integrated tetramer Af/B5/C4/Dd. N-Ab-U2-30F and N-Da-L2-20D were mixed to form a dimer Af/Da as a quenching reference. The tetramer was prepared under the same conditions as in Example 1 except that these 4 types of DNAs were simultaneously mixed.

For decoy DNA, synthetic oligonucleotides NFcpt01 and NFcpt02 or synthetic oligonucleotides NFcpt03 and NFcpt04 were mixed to prepare duplexes NF01/02 and NF03/04 having a single-stranded terminus under the same conditions as in Example 1. The duplex NF01/02 has an NF-κB binding sequence. However, the duplex NF03/04 has an NF-κB binding sequence variant.

Each sequence used is as follows:

**[Table 11]**

| |
|---|
| Fluorescence-labeled DNA (fluorescein-labeled thymine at the 30th base counted from the 5' terminus) |
| N-Ab-112-30F : |
| 5' CTCACGCACACCAGCGGGGACTTTCGGAGTATATAAAGACGAGCAAGCGG 3' (SEQ ID NO: 53) |
| Quenching-labeled DNA (DABCYL-labeled thymine at the 20th base counted from the 5' terminus) |
| N-Da-L2-20D : |
| 5' GGTCAGAGAGTAGCCTATAtACTGGGAAAGTCGCCGCTGGTGTGCGTGAG 3' (SEQ ID NO: 54) |
| Unlabeled DNA |
| N-Bc-L2m5 |
| 5' CGGCTTGCTCGTCTTTATATACTGTGAACGTCACCAACCTGTCCATTCCC 3' (SEQ ID NO: 55) |
| N-Cd-U2m4 |
| 5' GGGAATGGACAGGTTGAGGACTTACCCAGTATATAGGCTACTCTCTGACC 3' (SEQ ID NO: 56) |

**[Table 12]**

| decoy DNA |
|---|
| NFcpt01 : |
| 5' GCACTAGGGACTTTCCGCCACG (SEQ ID NO: 57) |
| NFcpt02 : |
| 5' CGTGGCGGAAAGTCGGTAGTGC 3' (SEQ ID NO: 58) |
| NFcpt03 : |
| 5' GCACTAAGGACTTGCGCCACG 3' (SEQ ID NO: 59) |
| NFcpt04 : |
| 5' CGTGGCGCAAAGTCCTTAGTGC 3' (SEQ ID NO: 60) |

The underlines represent a moiety to undergo strand exchange. The shaded areas represent an NF-κB binding sequence.

### (2) Detection of NF-κB (p50) binding

50 fmol of Af/B5/C4/Dd and 484 fmol of recombinant human NF-κB (p50) (Promega Corp.) were mixed into a reaction solution (10 mM HEPES-NaOH pH 7.9, 50 mM KCl, 0.1 mM EDTA, 5 mM DTT, 10% glycerol, 0.05% IGEPAL CA-630, 50 µL) and reacted at 25°C for 30 minutes. Then, the fluorescence value of fluorescein was measured using a fluorescence plate reader Ultra (Tecan Group Ltd.) (excitation: 485 nm, detection: 535 nm). The decoy DNA was added at a concentration of 1 pmol. Change in fluorescence value caused by the proteins was evaluated based on a background-corrected fluorescence value obtained by subtracting the fluorescence value of Af/Dd therefrom.

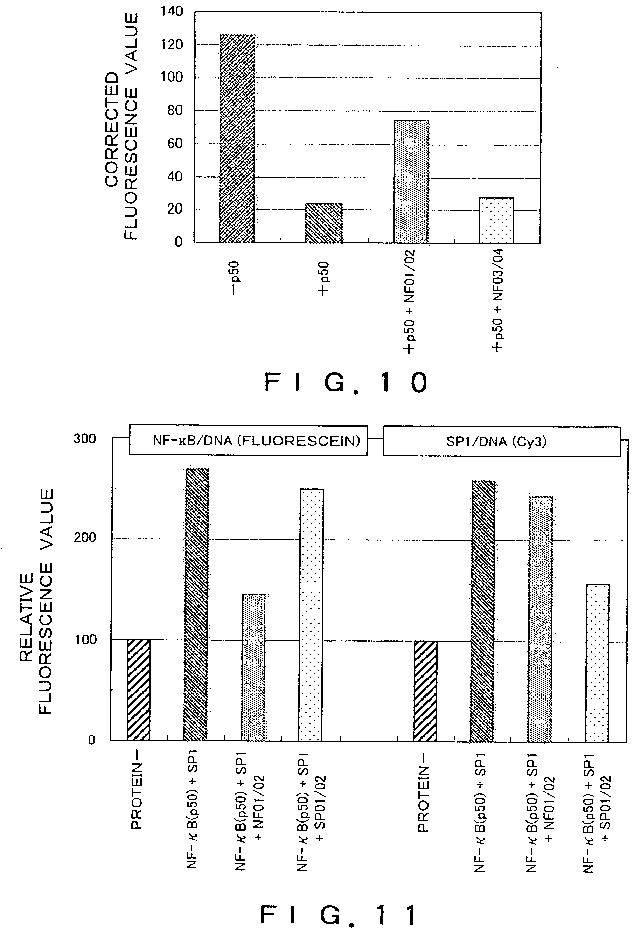

As a result, the fluorescence value of Af/B5/C4/Dd was suppressed by the addition of NF-κB (p50). Upon addition of the decoy DNA (NF01/02) having an NF-κB binding sequence, the fluorescence value was restored. On the other hand, the fluorescence value was not restored even after the addition of the decoy DNA (NF03/04) having an NF-κB binding sequence variant (Figure 10).

These results demonstrated that according to the method described above, the sequence-specific binding of nucleic acid binding proteins can be detected by measuring a fluorescence value.

### Example 7: Study on multiplexed detection system

To study a multiplexed detection system, the bindings of NF-κB (p50) and SP1 to DNAs were simultaneously detected. Furthermore, the specific inhibitory effect of an NF-κB (p50) inhibitor (decoy oligonucleotide) was detected.

### (1) Preparation of duplex

A synthetic oligonucleotide NFkB-42 and a synthetic oligonucleotide NFkB-31-5F labeled at the 5' terminus with fluorescein, or a synthetic oligonucleotide NFkB-41 and a synthetic oligonucleotide NFkB-32-3D labeled at the 3' terminus with DABCYL were mixed to prepare duplexes 31F/42 and 32D/41 having a single-stranded terminus under the same conditions as in Example 1. The duplexes 31F/42 and 32D/41 have an NF-κB binding sequence.

A synthetic oligonucleotide SP1-08 and a synthetic oligonucleotide SP1-05-5C3 labeled at the 5' terminus with Cy3, or a synthetic oligonucleotide SP1-07 and a synthetic oligonucleotide SP1-06-3B1 labeled at the 3' terminus with BHQ1 were mixed to prepare duplexes 05C/08 and 06B/07 having a single-stranded terminus under the same conditions as in Example 1. The duplexes 05C/08 and 06B/07 have an SP1 binding sequence.

Synthetic oligonucleotides NFcpt01 and NFcpt02 were mixed to prepare a duplex NF01/02 having a single-stranded terminus under the same conditions as in Example 1. The duplex NF01/02 has an NF-κB binding sequence.

Synthetic oligonucleotides SPcpt01 and SPcpt02 were mixed to prepare a duplex SP01/02 having a single-stranded terminus under the same conditions as in Example 1. The duplex SP01/02 has an SP1 binding sequence.

Each sequence used is as follows:

**[Table 13]**

| |
|---|
| NF-*κ*B DNA |
| NFkB-31-5F : |
| 5' Fluorescein- AGTTGAGGGGACTTTCCCAGGC *CTCACGCACACCAGC* 3' (SEQ ID NO: 61) |
| NFkB-32-30 : |
| 5' *GCTGGTGTGCGTGAG* GCCTGGGAAAGTCCCCTCAACT -Dabcyl 3' (SEQ ID NO: 62) |
| NFkB-41 : |
| 5' AGTTGAGGGGGACTTTCCCAGGC *AAGACGAGCAAGGAGGG* 3' (SEQ ID NO: 63) |
| NFkB-42 : |
| 5' *CCCTCCTTGCTCGTCTT* GCCTGGGAAAGTCCCCTCAACT 3' (SEQ ID NO: 64) |

The underlines represent an NF-κB binding sequence. The italic types represent a single-stranded sequence in the duplex.

**[Table 14]**

| SP1 DNA |
|---|
| SP1-05-5C3 : |
| 5' Cy3- ATTGGATCGGGGGGGGGGACGAGC *GGGAATGGACAGGTT* 3' (SEQ ID NO: 65) |
| SP1-06-3B1 : |
| 5' *AACCTGTCCATTCCC* GCTCGTCGCCCCGCCCCGATCGAAT -BHQ1 3' (SEQ ID NO: 66) |
| SP1-07 |
| 5' ATTCGATCGGGGCGGGGCGACGAGC *AGGCTACTCTCTGACCG* 3' (SEQ ID NO: 67) |
| SP1-08 : |
| 5' *CGGTCAGAGAGTAGCCT* GCTCGTCGCCCCGCCCCGATCGAAT 3' (SEQ ID NO: 68) |

The underlines represent an SP1 binding sequence. The italic types represent a single-stranded sequence in the duplex.

**[Table 15]**

| NF-*κ*B decoy |
|---|
| NFcpt01 : |
| 5' GCAGTAGGGACTTTCCGCCACG 3' (SEQ ID NO: 69) |
| NFcpt02 : |
| 5' CGTGGCGGAAAGTCCCTAGTGC 3' (SEQ ID NO: 70) |

The underlines represent an NF-κB binding sequence.

**[Table 16]**

| SP1 decoy |
|---|
| SPcpt01 |
| 5' TCGAGCGGGGCGGGGCGACGAA 3' (SEQ ID NO: 71) |
| SPcpt02 |
| 5' TTCGTCGCCCCGCGCCGCTCGA 3' (SEQ ID NO: 72) |

The underlines represent SP1.

### (2) Detection of protein binding

A mixed solution of 31F/42 and 05C/08 (40 fmol each), 800 fmol of the decoy (NF01/02 or SP01/02), a solution of a protein mixture (2.5 pmol of recombinant human SP1 and 0.125 pmol of recombinant human NFκB p50) were mixed into a reaction solution (10 mM HEPES-NaOH pH 7.9, 50 mM KCl, 0.1 mM EDTA, 5 mM DTT, 10% glycerol, 0.05% IGEPAL CA-630, 15 µL) and reacted at 25°C for 30 minutes. A mixed solution of 32D/41 and 06B/07 (80 fmol each) was added thereto, and 50 µL of the resulting mixture was then reacted at 25°C for 120 minutes. The fluorescence values of fluorescein and Cy3 were measured using a fluorescence plate reader Ultra (Tecan Group Ltd.). The fluorescein was excited at 485 nm, and its fluorescence was measured at 535 nm. The Cy3 was excited at 535 nm and its fluorescence was measured at 595 nm.

As a result, 31F/42 and 32D/41 formed a tetramer, which was in turn converted to 31F/32D by complete strand exchange through branch migration. The addition of the protein mixture (NF-κB (p50) + SP1) increased the fluorescence value decreased by this conversion (Figure 11; NF-κB).

05C/08 and 06B/07 formed a tetramer, which was in turn converted to 05C/06B by complete strand exchange through branch migration. The addition of the protein mixture (NF-κB (p50) + SP1) increased the fluorescence value decreased by this conversion (Figure 11; SP1).

In this context, NF01/02 was confirmed to decrease only the relative fluorescence value of the NF-κB/DNA. Moreover, SP01/02 was confirmed to decrease only the relative fluorescence value of the SP1/DNA (Figure 11; SP1).

These results demonstrated that according to this detection system, not only can mixed proteins be detected independently but also the screening of a drug inhibiting only a particular protein is achieved.

## Claims

1. A method of detecting binding between a nucleic acid and a nucleic acid binding protein, comprising determining the degree of structural change in a nucleic acid complex having at least two nucleic acid duplex moieties, wherein the nucleic acid complex having at least two nucleic acid duplex moieties is a complex comprising the two nucleic acid duplexes bound to each other at their terminal sequences (nucleic acid duplex complex), wherein the structural change is nucleotide strand exchange between the two nucleic acid duplexes, wherein the nucleotide strand exchange between the nucleic acid duplexes is a replacement of a strand complementary to each nucleotide strand forming one nucleic acid duplex with a nucleotide strand forming the other nucleic acid duplex, and wherein the method comprises
(i) contacting the nucleic acid binding protein with a nucleic acid duplex A and a nucleic acid duplex B, wherein the nucleic acid duplex A consists of two single-stranded nucleic acids (hereinafter, referred to as a "nucleic acid A1" and a "nucleic acid A2", respectively) and the nucleic acid duplex B consists of two single-stranded nucleic acids (hereinafter, referred to as a "nucleic acid B1" and a "nucleic acid B2", respectively); and
(ii) determining the degree of structural change in the nucleic acid duplex complex
wherein the nucleic acid A1 consists of a single-stranded nucleic acid having a first nucleotide sequence and a second nucleotide sequence; the nucleic acid A2 consists of a single-stranded nucleic acid having a sequence corresponding to the first nucleotide sequence and a third nucleotide sequence; the nucleic acid B1 consists of a single-stranded nucleic acid having a sequence corresponding to the second nucleotide sequence and a fourth nucleotide sequence; and the nucleic acid B2 consists of a single-stranded nucleic acid having a sequence corresponding to the third nucleotide sequence and a sequence corresponding to the fourth nucleotide sequence and
wherein the contacting in (i) may be performed by simultaneously mixing all the nucleic acid duplex A, the nucleic acid binding protein and the nucleic acid duplex B or by mixing the nucleic acid duplex A and the nucleic acid binding protein and then adding the nucleic acid duplex B to the mixture.

2. The method according to claim 1,
wherein in (ii), the degree of structural change in the nucleic acid duplex complex is determined by measuring an amount of a nucleic acid duplex consisting of the nucleic acid A1 and the nucleic acid B1 (nucleic acid duplex C) and/or a nucleic acid duplex consisting of the nucleic acid A2 and the nucleic acid B2 (nucleic acid duplex D); or
wherein in (ii), the degree of structural change in the nucleic acid duplex complex is determined by measuring an amount of the nucleic acid duplex A and/or the nucleic acid duplex B.

3. The method according to any one of claims 1 or 2, wherein mismatch base(s) exist alone or in combination, selected from:
one or more mismatch bases in the nucleotide sequence of the nucleic acid A1 with respect to the nucleotide sequence of the nucleic acid B1;
one or more mismatch bases in the nucleotide sequence of the nucleic acid B1 with respect to the nucleotide sequence of the nucleic acid A1;
one or more mismatch bases in the nucleotide sequence of the nucleic acid A2 with respect to the nucleotide sequence of the nucleic acid B2; and
one or more mismatch bases in the nucleotide sequence of the nucleic acid B2 with respect to the nucleotide sequence of the nucleic acid A2.

4. A method of detecting binding between a nucleic acid and a nucleic acid binding protein, comprising determining the degree of structural change in a nucleic acid complex having at least two nucleic acid duplex moieties, wherein the nucleic acid complex having at least two nucleic acid duplex moieties is a complex comprising the two nucleic acid duplex moieties to cause strand exchange reversibly (integrated nucleic acid complex), wherein the integrated nucleic acid complex is a nucleic acid complex formed by the nucleic acid complexes bound to each other, wherein the structural change is nucleotide strand exchange between the two nucleic acid duplexes, wherein the nucleotide strand exchange between the nucleic acid duplexes is a replacement of a strand complementary to each nucleotide strand forming one nucleic acid duplex with a nucleotide strand forming the other nucleic acid duplex, and wherein the method comprises
(iii) contacting the nucleic acid binding protein with an integrated nucleic acid complex E, wherein the integrated nucleic acid complex E comprises a nucleic acid complex G having a nucleic acid duplex moiety and a nucleic acid complex H having a nucleic acid duplex moiety, the nucleic acid complex G comprising two single-stranded nucleic acid-containing nucleic acids (hereinafter, referred to as a "nucleic acid G1" and a "nucleic acid G2", respectively) and the nucleic acid complex H comprising two single-stranded nucleic acid-containing nucleic acids (hereinafter, referred to as a "nucleic acid H1" and a "nucleic acid H2", respectively); and
(iv) determining the degree of structural change in the integrated nucleic acid complex, wherein
the nucleic acid G1 is a single-stranded nucleic acid-containing nucleic acid consisting of a terminal moiety 1, a fifth nucleotide sequence, and a terminal moiety 3, the fifth nucleotide sequence being linked at the 3' terminus to the terminal moiety 1 and at the 5' terminus to the terminal moiety 3;
the nucleic acid G2 is a single-stranded nucleic acid-containing nucleic acid consisting of a terminal moiety 2, a sequence corresponding to the fifth nucleotide sequence, and a moiety corresponding to the terminal moiety 3, the sequence corresponding to the fifth nucleotide sequence being linked at the 5' terminus to the terminal moiety 2 and at the 3' terminus to the moiety corresponding to the terminal moiety 3;
the nucleic acid H1 is a single-stranded nucleic acid-containing nucleic acid consisting of a moiety corresponding to the terminal moiety 1, a sixth nucleotide sequence, and a terminal moiety 4, the sixth nucleotide sequence being linked at the 5' terminus to the moiety corresponding to the terminal moiety 1 and at the 3' terminus to the terminal moiety 4; and
the nucleic acid H2 is a single-stranded nucleic acid-containing nucleic acid consisting of a moiety corresponding to the terminal moiety 4, a sequence corresponding to the sixth nucleotide sequence, and a moiety corresponding to the terminal moiety 2, the sequence corresponding to the sixth nucleotide sequence being linked at the 5' terminus to the moiety corresponding to the terminal moiety 4 and at the 3' terminus to the moiety corresponding to the terminal moiety 2
wherein the terminal moieties are capable of stopping the progress of strand exchange between two duplex moieties and reversing its direction,
wherein in (iv), the degree of structural change in the integrated nucleic acid complex is determined by measuring an amount of the integrated nucleic acid complex E; or
wherein in (iv), the degree of structural change in the integrated nucleic acid complex is determined by measuring an amount of an integrated nucleic acid complex F comprising a nucleic acid complex I and a nucleic acid complex J, the nucleic acid complex I comprising the nucleic acid G1 and the nucleic acid H1 and having a nucleic acid duplex moiety and the nucleic acid complex J comprising the nucleic acid G2 and the nucleic acid H2 and having a nucleic acid duplex moiety
wherein only any one of the integrated nucleic acid complex E or the integrated nucleic acid F has a site to which a nucleic acid binding protein binds,

5. The method according to claim 4, wherein the terminal moieties are polynucleotides, wherein
a highly stable base pair group is formed neither between the 5'-terminal nucleotide sequence of the terminal moiety 1 and the 3'-terminal nucleotide sequence of the terminal moiety 2 nor between the 3'-terminal nucleotide sequence of the moiety corresponding to the terminal moiety 1 and the 5'-terminal nucleotide sequence of the moiety corresponding to the terminal moiety 2; and/or
a highly stable base pair group is formed between the 5'-terminal nucleotide sequence of the terminal moiety 1 and the 3'-terminal nucleotide sequence of the moiety corresponding to the terminal moiety 1 and between the 3'-terminal nucleotide sequence of the terminal moiety 2 and the 5'-terminal nucleotide sequence of the moiety corresponding to the terminal moiety 2;
and
a highly stable base pair group is formed neither between the 3'-terminal nucleotide sequence of the terminal moiety 3 and the 5'-terminal nucleotide sequence of the terminal moiety 4 nor between the 5'-terminal nucleotide sequence of the moiety corresponding to the terminal moiety 3 and the 3'-terminal nucleotide sequence of the moiety corresponding to the terminal moiety 4; and/or
a highly stable base pair group is formed between the 3'-terminal nucleotide sequence of the terminal moiety 3 and the 5'-terminal nucleotide sequence of the moiety corresponding to the terminal moiety 3 and between the 5'-terminal nucleotide sequence of the terminal moiety 4 and the 3'-terminal nucleotide sequence of the moiety corresponding to the terminal moiety 4.

6. The method according to any one of claims 4 or 5, wherein mismatch base(s) exist alone or in combination, selected from:
one or more mismatch bases in the nucleotide sequence of the nucleic acid G1 with respect to the nucleotide sequence of the nucleic acid H1;
one or more mismatch bases in the nucleotide sequence of the nucleic acid H1 with respect to the nucleotide sequence of the nucleic acid G1;
one or more mismatch bases in the nucleotide sequence of the nucleic acid G2 with respect to the nucleotide sequence of the nucleic acid H2; and
one or more mismatch bases in the nucleotide sequence of the nucleic acid H2 with respect to the nucleotide sequence of the nucleic acid G2; or
wherein mismatch base(s) exist alone or in combination, selected from:
one or more mismatch bases in the nucleotide sequence of the nucleic acid G1 with respect to the nucleotide sequence of the nucleic acid G2;
one or more mismatch bases in the nucleotide sequence of the nucleic acid G2 with respect to the nucleotide sequence of the nucleic acid G1;
one or more mismatch bases in the nucleotide sequence of the nucleic acid H1 with respect to the nucleotide sequence of the nucleic acid H2; and
one or more mismatch bases in the nucleotide sequence of the nucleic acid H2 with respect to the nucleotide sequence of the nucleic acid H1.

7. The method according to any one of claims 1 to 6, wherein the method is performed in a multiplexed method.

8. A method of screening for a binding inhibitor or promoter for a nucleic acid binding protein, comprising performing a method according to any one of claims 1 to 7 in the presence of and in the absence of a test substance.

9. The screening method according to claim 8, further comprising determining the degree of structural change in a nucleic acid complex consisting of nucleic acids that do not bind to a nucleic acid binding protein targeted by the test substance.

## Patentansprüche

1. Ein Verfahren zur Bestimmung einer Bindung zwischen einer Nukleinsäure und einen Nukleinsäure- Bindungsprotein, umfassend Bestimmen des Grades der Strukturänderung in einem Nukleinsäure- Komplex, der zumindest mindestens zwei Nukleinsäure- Duplex- Teile aufweist, wobei der Nukleinsäure- Komplex, der mindestens zwei Nukleinsäure- Duplex- Teile aufweist, ein Komplex ist, der zwei Nukleinsäure- Duplexe umfasst, die miteinander an ihren terminalen Sequenzen (Nukleinsäure- Duplex- Komplex) gebunden sind, wobei die strukturelle Veränderung ein Nukleotid- Strang- Austausch zwischen den beiden Nukleinsäure- Duplexen ist, wobei der Nukleotid- Strang-Austausch zwischen den Nukleinsäure- Duplexen ein Ersatz eines Stranges ist, der komplementär zu jedem Nukleotid- Strang ist, der ein Nukleinsäure- Duplex mit einem Nukleotid- Strang bildet, der den anderen Nukleinsäure- Duplex bildet, und wobei das Verfahren umfasst:
(i) Inkontaktbringen des Nukleinsäure- Bindungs- Protein mit einem Nukleinsäure-Duplex A und einem Nukleinsäure- Duplex B, wobei der Nukleinsäure- Duplex A aus zwei einsträngigen Nukleinsäuren (im folgenden als "Nukleinsäure A1" und "Nukleinsäure A2" bezeichnet) besteht und der Nukleinsäure- Duplex B aus zwei einsträngigen Nukleinsäuren (im Folgenden bezeichnet als "Nukleinsäure B1" und "B2 Nukleinsäure" bezeichnet) besteht; und
(ii) Bestimmung des Grades der Strukturänderung in dem Nukleinsäure- Duplex-Komplex
wobei die Nukleinsäure A1 aus einer einsträngigen Nukleinsäure besteht, die eine erste Nukleotid- Sequenz und eine zweite Nukleotid- Sequenz aufweist; die Nukleinsäure A2 aus einer einsträngigen Nukleinsäure besteht, die eine Sequenz hat,
die der ersten Nukleotid- Sequenz und einer dritten Nukleotid- Sequenz entspricht; die Nukleinsäure B1 besteht aus eine einsträngige Nukleinsäure, die eine Sequenz hat, die der zweite Nukleotid- Sequenz und einer vierten Nukleotid- Sequenz entspricht; und die Nukleinsäure B2 besteht aus einer einsträngigen Nukleinsäure, die eine Sequenz hat, die der dritten Nukleotid- Sequenz und einer vierten Nukleotid- Sequenz entspricht, und
wobei das Inkontaktbringen in (i) durch gleichzeitiges Mischen aller Nukleinsäure-Duplex A, des Nukleinsäure- Bindungsprotein und der Nukleinsäure- Duplex B erfolgen kann oder durch Mischen des Nukleinsäure- Duplex A und des Nukleinsäure-Bindungsprotein und dann durch Hinzufügen des Nukleinsäure- Duplex B zu dem Gemisch.

2. Das Verfahren nach Anspruch 1,
wobei in (ii) der Grad der strukturellen Änderung in dem Nukleinsäure- Duplex-Komplex durch Messen einer Menge eines Nukleinsäure- Duplexes bestimmt wird, bestehend aus der Nukleinsäure A1 und der Nukleinsäure B1 (Nukleinsäure- Duplex C) und / oder eines Nukleinsäure- Duplex, der aus der Nukleinsäure A2 und der Nukleinsäure B2 (Nukleinsäure- Duplex D) beseht; oder
wobei in (ii) der Grad der strukturellen Änderung in dem Nukleinsäure- Duplex-Komplex durch Messen einer Menge des Nukleinsäure- Duplex A und / oder des Nukleinsäure- Duplex B bestimmt wird.

3. Das Verfahren nach einem der Ansprüche 1 oder 2, wobei Mismatch- Base(n) allein oder in Kombination existieren, ausgewählt aus:
einer oder mehrere Mismatch- Basen in der Nukleotid- Sequenz der Nukleinsäure A1 in Bezug auf die Nukleotid- Sequenz der Nukleinsäure B1;
eine oder mehrere Mismatch- Basen in der Nukleotid- Sequenz der Nukleinsäure B1 in Bezug auf die Nukleotid- Sequenz der Nukleinsäure A1;
eine oder mehrere Mismatch- Basen in der Nukleotid- Sequenz der Nukleinsäure A2 in Bezug auf die Nukleotid- Sequenz der Nukleinsäure B2; und
eine oder mehrere Mismatch- Basen in der Nukleotid- Sequenz der Nukleinsäure B2 in Bezug auf die Nukleotid- Sequenz der Nukleinsäure A2.

4. Ein Verfahren zum Nachweis einer Bindung zwischen einer Nukleinsäure und einem Nukleinsäure bindenden Proteins, umfassend Bestimmen des Grads der Strukturänderung in einem Nukleinsäure- Komplex, der mindestens zwei Nukleinsäure-Duplex- Teile aufweist,
wobei der Nukleinsäure- Komplex, der mindestens zwei Nukleinsäure- Duplex- Teile aufweist, ein Komplex ist, der die beiden Nukleinsäure- Duplex- Teile umfasst, um reversibel Strangaustausch zu verursachen (integrierte Nukleinsäure- Komplex), wobei der integrierte Nukleinsäure- Komplex ein Nukleinsäure- Komplex ist, der durch die Nukleinsäure- Komplexe, die miteinander gebunden sind, ausgebildet ist, wobei die Strukturänderung ein Nukleotid- Strangaustausch zwischen den zwei Nukleinsäure-Duplexen ein Ersatz eines komplementären Stranges zu jedem Nukleotid- Strang ist, der einen Nukleinsäure- Duplex mit einem Nukleotid- Strang bildet, der den anderen Nukleinsäure- Duplex bildet, und wobei das Verfahren umfasst,
(iii) Inkontaktbringen des Nukleinsäure- Bindungs- Protein mit einem integrierten Nukleinsäure- Komplexes E, wobei die integrierte Nukleinsäure- Komplex E eine Nukleinsäure- Komplex G umfasst, der ein Nukleinsäure- Duplex- Teil und einen Nukleinsäure- Komplex H umfasst, der ein Nukleinsäure- Duplex- Teil aufweist, der Nukleinsäure- Komplex G zwei einsträngige Nukleinsäuren- enthaltende Nukleinsäuren (nachfolgend jeweils als "Nukleinsäure G1" und "Nukleinsäure G2" bezeichnet) umfasst, und der Nukleinsäure- Komplex H aus zwei einsträngigen Nukleinsäure enthaltenden Nukleinsäuren (nachfolgend jeweils als "Nukleinsäure H1" und " Nukleinsäure H2" bezeichnet); und
(iv) Bestimmung des Grades der Strukturänderung in dem integrierten Nukleinsäure-Komplex, wobei die Nukleinsäure G1 eine einsträngige Nukleinsäure ist, die eine Nukleinsäure enthält, die aus einem terminalen Teil 1, einer fünften Nukleotid-Sequenz und einem terminalen Teil besteht, die fünfte Nukleotid- Sequenz ist am 3ten Terminus mit dem terminalen Teil 1 und am 5ten Terminus mit der terminalen Teil 3 verbunden;
die Nukleinsäure G2 ist eine einsträngige Nukleinsäure, die eine Nukleinsäure enthält, die aus einem terminalen Teil 2, aus einer Sequenz korrespondierend zu der fünften Nukleotid- Sequenz, und aus einem Teil, die zu dem terminalen Teil 3 korrespondiert, besteht, wobei die Sequenz, die mit der fünften Nukleotid- Sequenz korrespondiert,
am 5ten Terminus mit dem terminalen Teil 2 und am 3ten Terminus mit dem Teil, das zu dem terminalen Teil korrespondiert, verbunden ist;
die Nukleinsäure H1 ist eine einsträngige Nukleinsäure, die eine Nukleinsäure enthält, die aus einem Teil, das zu dem terminalen Teil 1 korrespondiert, aus einer sechsten Nukleotid- Sequenz und aus einem terminalen Teil 4 besteht, die sechste Nukleotid-Sequenz ist am 5ten Terminus mit dem Teil, die zu dem terminalen Teil 1 korrespondiert, und am 3ten Terminus mit der terminalen Teil 4 und
die Nukleinsäure H2 ist eine einsträngige Nukleinsäure, die eine Nukleinsäure enthält, die aus einem terminalen Teil, das zu dem terminal Teil 4 korrespondiert, aus einer Sequenz, die zu der sechsten Nukleotid- Sequenz korrespondiert, und aus einem Teil,
das zu dem terminalen Teil 2 korrespondiert, besteht, wobei die Sequenz, die zu der sechste Nukleotid- Sequenz korrespondiert, am 5ten Terminus mit dem Teil, die zu dem terminalen Teil 4 korrespondiert, und am 3ten Terminus mit dem Teil, das zu dem terminalen Teil 2 korrespondiert, verbunden ist,
wobei die terminalen Teile in der Lage sind, den Fortschritt des Strangaustausch zwischen zwei Duplex-Teilen zu stoppen und seine Richtung umzukehren,
wobei in (iv) der Grad der strukturellen Änderung in dem integrierten Nukleinsäure-Komplexes bestimmt wird, indem eine Menge des integrierten Nukleinsäure-Komplexes E gemessen wird; oder wobei in (iv) der Grad der Strukturänderung in dem integrierten Nukleinsäure- Komplex durch Messen einer Menge eines integrierten Nukleinsäure- Komplex F bestimmt wird, der einen Nukleinsäure- Komplex I und einen Nukleinsäure- Komplex J enthält, der Nukleinsäure- Komplex I umfasst die Nukleinsäure G1 und die Nukleinsäure H1 und hat einen Nukleinsäure- Duplex- Teil und der Nukleinsäure- Komplex J umfasst die Nukleinsäure G2 und die Nukleinsäure H2 und hat einen Nukleinsäure- Duplex- Teil
wobei nur jeder der integrierten Nukleinsäure- Komplexe E oder der integrierten Nukleinsäure F eine Seite hat, an die sich ein Nukleinsäure bindendes Protein bindet.

5. Das Verfahren nach Anspruch 4, wobei die terminalen Teilen Polynukleotide sind, wobei
eine hochstabile Basen- Paargruppe weder zwischen der 5ten terminalen Nukleotid-Sequenz des terminalen Teils 1 und der 3ten terminalen Nukleotid- Sequenz des terminalen Teils 2, noch zwischen der 3ten- terminalen Nukleotid- Sequenz des Teils,
das zu dem terminalen Teil 1 korrespondiert, und dem 5ten terminalen Nukleotid-Sequenz des Teils, das zu dem terminalen Teil 2 korrespondiert, ausgebildet wird;
und / oder
ein sehr stabiles Basenpaar- Gruppe ist zwischen der 5ten terminalen Nukleotid-Sequenz des terminalen Teils 1 und der 3ten terminalen Nukleotid- Sequenz des Teils,
das zu dem terminalen Teil 1 korrespondiert, und zwischen der 3ten terminalen Nukleotid- Sequenz des Teils 2 und der 5ten terminale Nukleotid- Sequenz des Teils, das zu dem terminalen Teil 2 korrespondiert, gebildet;
und
eine hoch stabile Basenpaar- Gruppe ist weder zwischen der 3ten terminalen Nukleotid- Sequenz des terminalen Teils 3 und der 5ten terminalen Nukleotid-Sequenz des terminalen Teil 4, noch zwischen der 5ten terminalen Nukleotid- Sequenz des Teils, das zu dem entsprechenden terminalen Teil 3 korrespondiert, und der 3ten terminalen Nukleotid- Sequenz des Teil, das zu dem terminalen Teil 4 korrespondiert; und / oder
ein sehr stabiles Basenpaar- Gruppe ist zwischen der 3ten terminalen Nukleotid-Sequenz des terminalen Teils 3 und der 5ten terminalen Nukleotid- Sequenz des Teils,
das zu dem terminalen Teil 3 korrespondiert, und zwischen dem 5ten terminalen Nukleotid- Sequenz des terminalen Teils 4 und der 3ten terminale Nukleotid- Sequenz des Teil, dazu zum dem terminalen Teil 4 korrespondiert, ausgebildet.

6. Das Verfahren nach einem der Ansprüche 4 oder 5, wobei Mismatch- Basen (en) bestehen alleine oder in Kombination, ausgewählt aus:
eine oder mehrere Mismatch- Basen in der Nukleotid- Sequenz der Nukleinsäure G1 in Bezug auf die die Nukleotid- Sequenz der Nukleinsäure H1;
eine oder mehrere Mismatch- Basen in der Nukleotid- Sequenz der Nukleinsäure H1 in Bezug auf die Nukleotid- Sequenz der Nukleinsäure G1;
eine oder mehrere Mismatch- Basen in der Nukleotid- Sequenz der Nukleinsäure G2 in Bezug auf die Nukleotid- Sequenz der Nukleinsäure H2; und
eine oder mehrere Mismatch- Basen in der Nukleotid- Sequenz der Nukleinsäure H2 in Bezug auf die Nukleotid- Sequenz der Nukleinsäure G2;
oder
wobei Mismatch- Base (n) allein oder in Kombination vorhanden sind, ausgewählt aus:
eine oder mehrere Mismatch- Basen in der Nukleotid- Sequenz der Nukleinsäure G1 in Bezug auf die Nukleotid- Sequenz der Nukleinsäure G2;
eine oder mehrere Mismatch- Basen in der Nukleotid- Sequenz der Nukleinsäure G2 in Bezug auf die Nukleotid- Sequenz der Nukleinsäure G1;
eine oder mehrere Mismatch- Basen in der Nukleotid- Sequenz der Nukleinsäure H1 in Bezug auf die Nukleotid- Sequenz der Nukleinsäure H2; und
eine oder mehrere Mismatch- Basen in der Nukleotid- Sequenz der Nukleinsäure H2 in Bezug auf die Nukleotid- Sequenz der Nukleinsäure H1.

7. Das Verfahren nach einem der Ansprüche 1 bis 6, wobei das Verfahren in einem gemultiplexten Verfahren durchgeführt wird.

8. Verfahren zum Screenen für einen Bindungsinhibitor oder Promotor für ein Nukleinsäure- Bindungsprotein, umfassend Durchführen eines Verfahrens nach einem der Ansprüche 1 bis 7 in Gegenwart und in Abwesenheit einer Testsubstanz.

9. Das Screening- Verfahren nach Anspruch 8, das ferner umfasst, Bestimmen des Grades der strukturellen Änderung in einem Nukleinsäure- Komplex, bestehend aus Nukleinsäuren, die sich nicht an ein Nukleinsäure- Bindungsprotein binden auf das durch die Testsubstanz abgezielt wird.

## Revendications

1. Méthode de détection d'une liaison entre un acide nucléique et une protéine de liaison aux acides nucléiques, comprenant la détermination du degré de changement structural dans un complexe d'acides nucléiques ayant au moins deux fragments duplex d'acides nucléiques, le complexe d'acides nucléiques ayant au moins deux fragments duplex d'acides nucléiques étant un complexe comprenant les deux duplex d'acides nucléiques liés l'un à l'autre au niveau de leurs séquences terminales (complexe duplex d'acides nucléiques), le changement structural étant l'échange de brins nucléotidiques entre les deux duplex d'acides nucléiques, l'échange de brins nucléotidiques entre les duplex d'acides nucléiques étant le remplacement d'un brin complémentaire à chaque brin nucléotidique formant un duplex d'acides nucléiques avec le brin nucléotidique formant l'autre duplex d'acides nucléiques, et la méthode comprenant :
(i) le contact de la protéine de liaison aux acides nucléiques avec un duplex d'acides nucléiques A et un duplex d'acides nucléiques B, le duplex d'acides nucléiques A étant constitué de deux acides nucléiques monocaténaires (appelés respectivement par la suite « acide nucléique A1 » et « acide nucléique A2 ») et le duplex d'acides nucléiques B étant constitué de deux acides nucléiques monocaténaires (appelés respectivement par la suite « acide nucléique B1 » et « acide nucléique B2 ») ; et
(ii) la détermination du degré de changement structural dans le complexe duplex d'acides nucléiques,
l'acide nucléique A1 étant constitué d'un acide nucléique monocaténaire ayant une première séquence nucléotidique et une deuxième séquence nucléotidique ; l'acide nucléique A2 étant constitué d'un acide nucléique monocaténaire ayant une séquence correspondant à la première séquence nucléotidique et une troisième séquence nucléotidique ; l'acide nucléique B1 étant constitué d'un acide nucléique monocaténaire ayant une séquence correspondant à la deuxième séquence nucléotidique et une quatrième séquence nucléotidique ; et l'acide nucléique B2 étant constituée d'un acide nucléique monocaténaire ayant une séquence correspondant à la troisième séquence nucléotidique et une séquence correspondant à la quatrième séquence nucléotidique et
le contact en (i) pouvant être réalisé en mélangeant simultanément l'ensemble des duplex d'acides nucléiques A, protéine de liaison aux acides nucléiques et duplex d'acides nucléiques B ou en mélangeant le duplex d'acides nucléiques A et la protéine de liaison aux acides nucléiques puis en ajoutant au mélange le duplex d'acides nucléiques B.

2. Méthode selon la revendication 1,
dans laquelle, en (ii), le degré de changement structural dans le complexe duplex d'acides nucléiques est déterminé en mesurant la quantité de duplex d'acides nucléiques constitué de l'acide nucléique A1 et de l'acide nucléique B1 (duplex d'acides nucléiques C) et/ou de duplex d'acides nucléiques constitué de l'acide nucléique A2 et de l'acide nucléique B2 (duplex d'acides nucléiques D) ; ou
dans laquelle, en (ii), le degré de changement structural dans le complexe duplex d'acides nucléiques est déterminé en mesurant la quantité de duplex d'acides nucléiques A et/ou de duplex d'acides nucléiques B.

3. Méthode selon l'une quelconque des revendications 1 ou 2, dans laquelle il existe des bases mésappariées individuelles ou associées, choisies parmi :
une ou plusieurs bases mésappariées dans la séquence nucléotidique de l'acide nucléique A1 par rapport à la séquence nucléotidique de l'acide nucléique B1 ;
une ou plusieurs bases mésappariées dans la séquence nucléotidique de l'acide nucléique B1 par rapport à la séquence nucléotidique de l'acide nucléique A1 ;
une ou plusieurs bases mésappariées dans la séquence nucléotidique de l'acide nucléique A2 par rapport à la séquence nucléotidique de l'acide nucléique B2 ; et
une ou plusieurs bases mésappariées dans la séquence nucléotidique de l'acide nucléique B2 par rapport à la séquence nucléotidique de l'acide nucléique A2.

4. Méthode de détection d'une liaison entre un acide nucléique et une protéine de liaison aux acides nucléiques, comprenant la détermination du degré de changement structural dans un complexe d'acides nucléiques ayant au moins deux fragments duplex d'acides nucléiques, le complexe d'acides nucléiques ayant au moins deux fragments duplex d'acides nucléiques étant un complexe comprenant les deux fragments duplex d'acides nucléiques pour provoquer l'échange réversible de brins (complexe d'acides nucléiques intégré), le complexe d'acides nucléiques intégré étant un complexe d'acides nucléiques formé par les complexes d'acides nucléiques liés les uns aux autres, le changement structural étant l'échange de brins nucléotidiques entre les deux duplex d'acides nucléiques, l'échange de brins nucléotidiques entre les duplex d'acides nucléiques étant le remplacement d'un brin complémentaire à chaque brin nucléotidique formant un duplex d'acides nucléiques par un brin nucléotidique formant l'autre duplex d'acides nucléiques, et la méthode comprenant :
(iii) le contact de la protéine de liaison aux acides nucléiques avec un complexe d'acides nucléiques intégré E, le complexe d'acides nucléiques intégré E comprenant un complexe d'acides nucléiques G ayant un fragment duplex d'acides nucléiques et un complexe d'acides nucléiques H ayant un fragment duplex d'acides nucléiques, le complexe d'acides nucléiques G comprenant deux acides nucléiques contenant un acide nucléique monocaténaire (appelés respectivement dans ce qui suit « acide nucléique G1 » et « acide nucléique G2 ») et le complexe d'acides nucléiques H comprenant deux acides nucléiques contenant un acide nucléique monocaténaire (appelés respectivement dans ce qui suit « acide nucléique H1 » et « acide nucléique H2 ») ; et
(iv) la détermination du degré de changement structural dans le complexe d'acides nucléiques intégré,
l'acide nucléique G1 étant un acide nucléique contenant un acide nucléique monocaténaire constitué d'un fragment terminal 1, d'une cinquième séquence nucléotidique et d'un fragment terminal 3, la cinquième séquence nucléotidique étant liée au fragment terminal 1 au niveau de l'extrémité 3' et au fragment terminal 3 au niveau de l'extrémité 5' ;
l'acide nucléique G2 étant un acide nucléique contenant un acide nucléique monocaténaire constitué d'un fragment terminal 2, d'une séquence correspondant à la cinquième séquence nucléotidique et d'un fragment correspondant au fragment terminal 3, la séquence correspondant à la cinquième séquence nucléotidique étant liée au fragment terminal 2 au niveau de l'extrémité 5' et au fragment correspondant au fragment terminal 3 au niveau de l'extrémité 3' ;
l'acide nucléique H1 étant un acide nucléique contenant un acide nucléique monocaténaire constitué d'un fragment correspondant au fragment terminal 1, d'une sixième séquence nucléotidique et d'un fragment terminal 4, la sixième séquence nucléotidique étant liée au fragment correspondant au fragment terminal 1 au niveau de l'extrémité 5' et au fragment terminal 4 au niveau de l'extrémité 3' ; et
l'acide nucléique H2 étant un acide nucléique contenant un acide nucléique monocaténaire constitué d'un fragment correspondant au fragment terminal 4, d'une séquence correspondant à la sixième séquence nucléotidique et d'un fragment correspondant au fragment terminal 2, la séquence correspondant à la sixième séquence nucléotidique étant liée au fragment correspondant au fragment terminal 4 au niveau de l'extrémité 5' et au fragment correspondant au fragment terminal 2 au niveau de l'extrémité 3' ;
les fragments terminaux étant capables d'arrêter la progression de l'échange de brins entre deux fragments duplex et d'inverser son sens,
en (iv), le degré de changement structural dans le complexe d'acides nucléiques intégré étant déterminé en mesurant la quantité de complexe d'acides nucléiques intégré E ; ou
en (iv), le degré de changement structural dans le complexe d'acides nucléiques intégré étant déterminé en mesurant la quantité de complexe d'acides nucléiques intégré F comprenant un complexe d'acides nucléiques I et un complexe d'acides nucléiques J, le complexe d'acides nucléiques I comprenant l'acide nucléique G1 et l'acide nucléique H1 et ayant le fragment duplex d'acides nucléiques et le complexe d'acides nucléiques J comprenant l'acide nucléique G2 et l'acide nucléique H2 et ayant un fragment duplex d'acides nucléiques,
seul l'un ou l'autre du complexe d'acides nucléiques intégré E ou du complexe d'acides nucléiques intégré F ayant un site auquel se fixe la protéine de liaison aux acides nucléiques.

5. Méthode selon la revendication 4, dans laquelle les fragments terminaux sont des polynucléotides, dans laquelle
un groupe de paires de bases très stables n'est formé ni entre la séquence nucléotidique 5'-terminale du fragment terminal 1 et la séquence nucléotidique 3'-terminale du fragment terminal 2, ni entre la séquence nucléotidique 3'-terminale du fragment correspondant au fragment terminal 1 et la séquence nucléotidique 5'-terminale du fragment correspondant au fragment terminal 2 ; et/ou
un groupe de paires de bases très stables est formé entre la séquence nucléotidique 5'-terminale du fragment terminal 1 et la séquence nucléotidique 3'-terminale du fragment correspondant au fragment terminal 1 et entre la séquence nucléotidique 3'-terminale du fragment terminal 2 et la séquence nucléotidique 5'-terminale du fragment correspondant au fragment terminal 2 ; et
un groupe de paires de bases très stables n'est formé ni entre la séquence nucléotidique 3'-terminale du fragment terminal 3 et la séquence nucléotidique 5'-terminale du fragment terminal 4, ni entre la séquence nucléotidique 5'-terminale du fragment correspondant au fragment terminal 3 et la séquence nucléotidique 3'-terminale du fragment correspondant au fragment terminal 4 ; et/ou
un groupe de paires de bases très stables est formé entre la séquence nucléotidique 3'-terminale du fragment terminal 3 et la séquence nucléotidique 5'-terminale du fragment correspondant au fragment terminal 3 et entre la séquence nucléotidique 5'-terminale du fragment terminal 4 et la séquence nucléotidique 3'-terminale du fragment correspondant au fragment terminal 4.

6. Méthode selon l'une quelconque des revendications 4 ou 5, dans laquelle une ou plusieurs bases mésappariées existent individuellement ou associées, choisies parmi :
une ou plusieurs bases mésappariées dans la séquence nucléotidique de l'acide nucléique G1 par rapport à la séquence nucléotidique de l'acide nucléique H1 ;
une ou plusieurs bases mésappariées dans la séquence nucléotidique de l'acide nucléique H1 par rapport à la séquence nucléotidique de l'acide nucléique G1 ;
une ou plusieurs bases mésappariées dans la séquence nucléotidique de l'acide nucléique G2 par rapport à la séquence nucléotidique de l'acide nucléique H2 ; et
une ou plusieurs bases mésappariées dans la séquence nucléotidique de l'acide nucléique H2 par rapport à la séquence nucléotidique de l'acide nucléique G2 ; ou
dans laquelle une ou plusieurs bases mésappariées existent individuellement ou associées, choisies parmi :
une ou plusieurs bases mésappariées dans la séquence nucléotidique de l'acide nucléique G1 par rapport à la séquence nucléotidique de l'acide nucléique G2 ;
une ou plusieurs bases mésappariées dans la séquence nucléotidique de l'acide nucléique G2 par rapport à la séquence nucléotidique de l'acide nucléique G1 ;
une ou plusieurs bases mésappariées dans la séquence nucléotidique de l'acide nucléique H1 par rapport à la séquence nucléotidique de l'acide nucléique H2 ; et
une ou plusieurs bases mésappariées dans la séquence nucléotidique de l'acide nucléique H2 par rapport à la séquence nucléotidique de l'acide nucléique H1.

7. Méthode selon l'une quelconque des revendications 1 à 6, laquelle est réalisée en tant que méthode multiplexée.

8. Méthode de criblage d'un inhibiteur ou d'un promoteur de liaison pour protéine de liaison aux acides nucléiques, comprenant la réalisation de la méthode selon l'une quelconque des revendications 1 à 7 en présence et en l'absence d'une substance test.

9. Méthode de criblage selon la revendication 8, comprenant en outre la détermination du degré de changement structural dans un complexe d'acides nucléiques constitué d'acides nucléiques qui ne se lient pas à la protéine de liaison aux acides nucléiques ciblée par la substance test.


### SEQUENCE LISTING

<110> Hiroshima University
   Wakunaga Pharmaceutical Co., Ltd.
<120> Method and kit for detecting the nucleic acid binding protein
<130> 177218PX
<150> JP 2008-11750
   <151> 2008-01-22
<160> 72
<170> PatentIn version 3.4
<210> 1
   <211> 50
   <212> DNA
   <213> Artificial
<220>
   <223> synthetic oligonucleotide
<400> 1
   agttgagggg actttcccag gcgactcact ataggcggtg tctcgctcgc 50
<210> 2
   <211> 50
   <212> DNA
   <213> Artificial
<220>
   <223> synthetic oligonucleotide
<400> 2
   gcgagcgaga caccgcctat agtgagtcgc ctgggaaagt cccctcaact 50
<210> 3
   <211> 50
   <212> DNA
   <213> Artificial
<220>
   <223> synthetic oligonucleotide
<400> 3
   agttgagggg actttcccag gcgactcact ataggcacca caccattccc 50
<210> 4
   <211> 50
   <212> DNA
   <213> Artificial
<220>
   <223> synthetic oligonucleotide
<400> 4
   gggaatggtg tggtgcctat agtgagtcgc ctgggaaagt cccctcaact 50
<210> 5
   <211> 50
   <212> DNA
   <213> Artificial
<220>
   <223> synthetic oligonucleotide
<400> 5
   agttgagggg actttcccag gcgactcact ataggcggtg tctcgctcgc 50
<210> 6
   <211> 50
   <212> DNA
   <213> Artificial
<220>
   <223> synthetic oligonucleotide
<400> 6
   gcgagcgaga caccgcctat agtgagtcgc ctgggaaagt cccctcaact 50
<210> 7
   <211> 50
   <212> DNA
   <213> Artificial
<220>
   <223> synthetic oligonucleotide
<400> 7
   agttgagggg actttcccag gcgactcact ataggcacca caccattccc 50
<210> 8
   <211> 50
   <212> DNA
   <213> Artificial
<220>
   <223> synthetic oligonucleotide
<400> 8
   gggaatggtg tggtgcctat agtgagtcgc ctgggaaagt cccctcaact 50
<210> 9
   <211> 22
   <212> DNA
   <213> Artificial
<220>
   <223> synthetic oligonucleotide
<400> 9
   gcactaggga ctttccgcca cg 22
<210> 10
   <211> 22
   <212> DNA
   <213> Artificial
<220>
   <223> synthetic oligonucleotide
<400> 10
   cgtggcggaa agtccctagt gc 22
<210> 11
   <211> 22
   <212> DNA
   <213> Artificial
<220>
   <223> synthetic oligonucleotide
<400> 11
   ggtgtcatga gtcagctcgg ag 22
<210> 12
   <211> 22
   <212> DNA
   <213> Artificial
<220>
   <223> synthetic oligonucleotide
<400> 12
   ctccgagctg actcatgaca cc 22
<210> 13
   <211> 36
   <212> DNA
   <213> Artificial
<220>
   <223> synthetic oligonucleotide
<400> 13
   cgcttgatga gtcagccgga agtggttggg taaggg 36
<210> 14
   <211> 36
   <212> DNA
   <213> Artificial
<220>
   <223> synthetic oligonucleotide
<400> 14
   cccttaccca accacttccg gctgactcat caagcg 36
<210> 15
   <211> 36
   <212> DNA
   <213> Artificial
<220>
   <223> synthetic oligonucleotide
<400> 15
   cgcttgatga gtcagccgga acggacagga cgatat 36
<210> 16
   <211> 36
   <212> DNA
   <213> Artificial
<220>
   <223> synthetic oligonucleotide
<400> 16
   atatcgtcct gtccgttccg gctgactcat caagcg 36
<210> 17
   <211> 43
   <212> DNA
   <213> Artificial
<220>
   <223> synthetic oligonucleotide
<400> 17
   gggattcgat cggggcgggg cgacgagcgt ggttgggtaa ggg 43
<210> 18
   <211> 43
   <212> DNA
   <213> Artificial
<220>
   <223> synthetic oligonucleotide
<400> 18
   cccttaccca accacgctcg tcgccccgcc ccgatcgaat ccc 43
<210> 19
   <211> 43
   <212> DNA
   <213> Artificial
<220>
   <223> synthetic oligonucleotide
<400> 19
   gggattcgat cggggcgggg cgacgagcca ccacaccatt ccc 43
<210> 20
   <211> 43
   <212> DNA
   <213> Artificial
<220>
   <223> synthetic oligonucleotide
<400> 20
   gggaatggtg tggtggctcg tcgccccgcc ccgatcgaat ccc 43
<210> 21
   <211> 50
   <212> DNA
   <213> Artificial
<220>
   <223> synthetic oligonucleotide
<400> 21
   agttgagtta gggttagggt tagggttagg gcaggcggtg tctcgctcgc 50
<210> 22
   <211> 50
   <212> DNA
   <213> Artificial
<220>
   <223> synthetic oligonucleotide
<400> 22
   gcgagcgaga caccgcctgc cctaacccta accctaaccc taactcaact 50
<210> 23
   <211> 50
   <212> DNA
   <213> Artificial
<220>
   <223> synthetic oligonucleotide
<400> 23
   agttgagtta gggttagggt tagggttagg gcaggcacca caccattccc 50
<210> 24
   <211> 50
   <212> DNA
   <213> Artificial
<220>
   <223> synthetic oligonucleotide
<400> 24
   gggaatggtg tggtgcctgc cctaacccta accctaaccc taactcaact 50
<210> 25
   <211> 50
   <212> DNA
   <213> Artificial
<220>
   <223> synthetic oligonucleotide
<400> 25
   agttgagtta gcgttagcgt tagcgttagc gcaggcggtg tctcgctcgc 50
<210> 26
   <211> 50
   <212> DNA
   <213> Artificial
<220>
   <223> synthetic oligonucleotide
<400> 26
   gcgagcgaga caccgcctgc gctaacgcta acgctaacgc taactcaact 50
<210> 27
   <211> 50
   <212> DNA
   <213> Artificial
<220>
   <223> synthetic oligonucleotide
<400> 27
   agttgagtta gcgttagcgt tagcgttagc gcaggcacca caccattccc 50
<210> 28
   <211> 50
   <212> DNA
   <213> Artificial
<220>
   <223> synthetic oligonucleotide
<400> 28
   gggaatggtg tggtgcctgc gctaacgcta acgctaacgc taactcaact 50
<210> 29
   <211> 40
   <212> DNA
   <213> Artificial
<220>
   <223> synthetic oligonucleotide
<400> 29
   attcgatcgg ggcggggcga cgagcgggaa tggacaggtt 40
<210> 30
   <211> 42
   <212> DNA
   <213> Artificial
<220>
   <223> synthetic oligonucleotide
<400> 30
   cggtcagaga gtagcctgct cgtcgccccg ccccgatcga at 42
<210> 31
   <211> 42
   <212> DNA
   <213> Artificial
<220>
   <223> synthetic oligonucleotide
<400> 31
   attcgatcgg ggcggggcga cgagcaggct actctctgac cg 42
<210> 32
   <211> 42
   <212> DNA
   <213> Artificial
<220>
   <223> synthetic oligonucleotide
<400> 32
   attcgatcgg ggcggggcgc cgagcaggct actctctgac cg 42
<210> 33
   <211> 42
   <212> DNA
   <213> Artificial
<220>
   <223> synthetic oligonucleotide
<400> 33
   attcgatcgg ggcggggcga cgagaaggct actctctgac cg 42
<210> 34
   <211> 40
   <212> DNA
   <213> Artificial
<220>
   <223> synthetic oligonucleotide
<400> 34
   aacctgtcca ttcccgctcg tcgccccgcc ccgatcgaat 40
<210> 35
   <211> 40
   <212> DNA
   <213> Artificial
<220>
   <223> synthetic oligonucleotide
<400> 35
   aacctgtcca ttcccgctcg gcgccccgcc ccgatcgaat 40
<210> 36
   <211> 40
   <212> DNA
   <213> Artificial
<220>
   <223> synthetic oligonucleotide
<400> 36
   aacctgtcca ttccctctcg tcgccccgcc ccgatcgaat 40
<210> 37
   <211> 50
   <212> DNA
   <213> Artificial
<220>
   <223> synthetic oligonucleotide
<400> 37
   agttgagggg actttcccag gcgactcact ataggcggtg tctcgctcgc 50
<210> 38
   <211> 50
   <212> DNA
   <213> Artificial
<220>
   <223> synthetic oligonucleotide
<400> 38
   gggaatggtg tggtgcctat agtgagtcgc ctgggaaagt cccctcaact 50
<210> 39
   <211> 50
   <212> DNA
   <213> Artificial
<220>
   <223> synthetic oligonucleotide
<400> 39
   agttgagggg actttcccag gcgactcact ataggcacca caccattccc 50
<210> 40
   <211> 50
   <212> DNA
   <213> Artificial
<220>
   <223> synthetic oligonucleotide
<400> 40
   agttgagggg acttgcccag gcgactcact ataggcacca caccattccc 50
<210> 41
   <211> 50
   <212> DNA
   <213> Artificial
<220>
   <223> synthetic oligonucleotide
<400> 41
   agttgagggg actttcccag gcgactcact atagacacca caccattccc 50
<210> 42
   <211> 50
   <212> DNA
   <213> Artificial
<220>
   <223> synthetic oligonucleotide
<400> 42
   gcgagcgaga caccgcctat agtgagtcgc ctgggaaagt cccctcaact 50
<210> 43
   <211> 50
   <212> DNA
   <213> Artificial
<220>
   <223> synthetic oligonucleotide
<400> 43
   gcgagcgaga caccgcctat agtgagtcgc ctgggcaagt cccctcaact 50
<210> 44
   <211> 50
   <212> DNA
   <213> Artificial
<220>
   <223> synthetic oligonucleotide
<400> 44
   gcgagcgaga caccgtctat agtgagtcgc ctgggaaagt cccctcaact 50
<210> 45
   <211> 37
   <212> DNA
   <213> Artificial
<220>
   <223> synthetic oligonucleotide
<400> 45
   agttgagggg actttcccag gcctcacgca caccagc 37
<210> 46
   <211> 37
   <212> DNA
   <213> Artificial
<220>
   <223> synthetic oligonucleotide
<400> 46
   gctggtgtgc gtgaggcctg ggaaagtccc ctcaact 37
<210> 47
   <211> 39
   <212> DNA
   <213> Artificial
<220>
   <223> synthetic oligonucleotide
<400> 47
   agttgagggg actttcccag gcaagacgag caaggaggg 39
<210> 48
   <211> 39
   <212> DNA
   <213> Artificial
<220>
   <223> synthetic oligonucleotide
<400> 48
   ccctccttgc tcgtcttgcc tgggaaagtc ccctcaact 39
<210> 49
   <211> 40
   <212> DNA
   <213> Artificial
<220>
   <223> synthetic oligonucleotide
<400> 49
   attcgatcgg ggcggggcga cgagcgggaa tggacaggtt 40
<210> 50
   <211> 40
   <212> DNA
   <213> Artificial
<220>
   <223> synthetic oligonucleotide
<400> 50
   aacctgtcca ttcccgctcg tcgccccgcc ccgatcgaat 40
<210> 51
   <211> 42
   <212> DNA
   <213> Artificial
<220>
   <223> synthetic oligonucleotide
<400> 51
   attcgatcgg ggcggggcga cgagcaggct actctctgac cg 42
<210> 52
   <211> 42
   <212> DNA
   <213> Artificial
<220>
   <223> synthetic oligonucleotide
<400> 52
   cggtcagaga gtagcctgct cgtcgccccg ccccgatcga at 42
<210> 53
   <211> 50
   <212> DNA
   <213> Artificial
<220>
   <223> synthetic oligonucleotide
<400> 53
   ctcacgcaca ccagcgggga ctttcccagt atataaagac gagcaagcgg 50
<210> 54
   <211> 50
   <212> DNA
   <213> Artificial
<220>
   <223> synthetic oligonucleotide
<400> 54
   ggtcagagag tagcctatat actgggaaag tccccgctgg tgtgcgtgag 50
<210> 55
   <211> 50
   <212> DNA
   <213> Artificial
<220>
   <223> synthetic oligonucleotide
<400> 55
   ccgcttgctc gtctttatat actgtgaacg tcaccaacct gtccattccc 50
<210> 56
   <211> 50
   <212> DNA
   <213> Artificial
<220>
   <223> synthetic oligonucleotide
<400> 56
   gggaatggac aggttgagga cttacccagt atataggcta ctctctgacc 50
<210> 57
   <211> 22
   <212> DNA
   <213> Artificial
<220>
   <223> synthetic oligonucleotide
<400> 57
   gcactaggga ctttccgcca cg 22
<210> 58
   <211> 22
   <212> DNA
   <213> Artificial
<220>
   <223> synthetic oligonucleotide
<400> 58
   cgtggcggaa agtccctagt gc 22
<210> 59
   <211> 22
   <212> DNA
   <213> Artificial
<220>
   <223> synthetic oligonucleotide
<400> 59
   gcactaagga ctttgcgcca cg 22
<210> 60
   <211> 22
   <212> DNA
   <213> Artificial
<220>
   <223> synthetic oligonucleotide
<400> 60
   cgtggcgcaa agtccttagt gc 22
<210> 61
   <211> 37
   <212> DNA
   <213> Artificial
<220>
   <223> synthetic oligonucleotide
<400> 61
   agttgagggg actttcccag gcctcacgca caccagc 37
<210> 62
   <211> 37
   <212> DNA
   <213> Artificial
<220>
   <223> synthetic oligonucleotide
<400> 62
   gctggtgtgc gtgaggcctg ggaaagtccc ctcaact 37
<210> 63
   <211> 39
   <212> DNA
   <213> Artificial
<220>
   <223> synthetic oligonucleotide
<400> 63
   agttgagggg actttcccag gcaagacgag caaggaggg 39
<210> 64
   <211> 39
   <212> DNA
   <213> Artificial
<220>
   <223> synthetic oligonucleotide
<400> 64
   ccctccttgc tcgtcttgcc tgggaaagtc ccctcaact 39
<210> 65
   <211> 40
   <212> DNA
   <213> Artificial
<220>
   <223> synthetic oligonucleotide
<400> 65
   attcgatcgg ggcggggcga cgagcgggaa tggacaggtt 40
<210> 66
   <211> 40
   <212> DNA
   <213> Artificial
<220>
   <223> synthetic oligonucleotide
<400> 66
   aacctgtcca ttcccgctcg tcgccccgcc ccgatcgaat 40
<210> 67
   <211> 42
   <212> DNA
   <213> Artificial
<220>
   <223> synthetic oligonucleotide
<400> 67
   attcgatcgg ggcggggcga cgagcaggct actctctgac cg 42
<210> 68
   <211> 42
   <212> DNA
   <213> Artificial
<220>
   <223> synthetic oligonucleotide
<400> 68
   cggtcagaga gtagcctgct cgtcgccccg ccccgatcga at 42
<210> 69
   <211> 22
   <212> DNA
   <213> Artificial
<220>
   <223> synthetic oligonucleotide
<400> 69
   gcactaggga ctttccgcca cg 22
<210> 70
   <211> 22
   <212> DNA
   <213> Artificial
<220>
   <223> synthetic oligonucleotide
<400> 70
   cgtggcggaa agtccctagt gc 22
<210> 71
   <211> 22
   <212> DNA
   <213> Artificial
<220>
   <223> synthetic oligonucleotide
<400> 71
   tcgagcgggg cggggcgacg aa 22
<210> 72
   <211> 22
   <212> DNA
   <213> Artificial
<220>
   <223> synthetic oligonucleotide
<400> 72
   ttcgtcgccc cgccccgctc ga 22